# EUROPÄISCHE PATENTSCHRIFT

(11) **EP 0 350 846 B1**
(45) Veröffentlichungstag und Bekanntmachung des Hinweises auf die Patenterteilung: **11.10.1995**
(21) Anmeldenummer: 89112607.0
(22) Anmeldetag: 10.07.1989
(51) Int. Cl.: C07D 311/58, C07D 335/06, C07D 339/00, C07D 319/18, C07D 317/46, C07D 321/10, C07D 313/08, C07D 337/08, A61K 31/35, A61K 31/38

(54) **Neue kondensierte heterocyclische Verbindungen und ihre Verwendung für Heilmittel**
Condensed heterocyclic compounds and their use in therapy
Composés hétérocycliques condensés et leur usage en thérapie

(30) Priorität: 14.07.1988 CH 2694/88; 26.05.1989 CH 1994/89
(43) Veröffentlichungstag der Anmeldung: 17.01.1990
(73) Patentinhaber: F. HOFFMANN-LA ROCHE AG, 4002 Basel (CH)
(72) Erfinder: Klaus, Michael, Dr., D-7858 Weil/Rhein (DE); Mohr, Peter, Dr., CH-4051 Basel (CH); Weiss, Ekkehard, Dr., D-7854 Inzlingen (DE)
(74) Vertreter: Lederer, Franz, Dr.

(56) Entgegenhaltungen:
- EP-A- 0 130 795
- WO-A-85/00806
- DE-A- 2 015 351
- GB-A- 2 119 801
- LU-A- 86 259
- US-A- 4 826 984
- CHEMICAL ABSTRACTS, Band 70, 1969, Seite 299, Zusammenfassung Nr. 77526d,Columbus, Ohio, US; Y.P. TALWAR et al.: "Chemical utilization of dillapiole, awaste product of the [Amethum] sowa oil industry",& INDIAN PERFUM. 1966, 10(Pt. 2), 43-5
- CHEMICAL ABSTRACTS, Band 77, 1972, Seite 472, Zusammenfassung Nr. 114615z,Columbus, Ohio, US; & G. SAVONA et al.: "Synthesis ofpermethylnarciprimine and permethyl-isonarciprimine", & J.HETEROCYCL. CHEM. 1971, 8(4), 681-4
- TETRAHEDRON LETTERS, Band 29, Nr. 22, 1988, Seiten 2741-2744, Pergamon PressPlc, GB; M. BOTTA et al.: "6-alkyl-2-methoxy-4-(3H)-pyrimidinones in thetransformation of pyrimidines: Regiospecific preparation, antitumor andantimicrobial activity of 4-Q-acylated pyrimidine derivatives. New agents forselective acylation of amines"
- CHEMICAL ABSTRACTS, Band 83, 1975, Seite 434, Zusammenfassung Nr. 27289b,Columbus, Ohio, US; R.G. KINSMAN et al.: "1,2-Dihydroisoquinolines. X.X.Rearrangements. VI", & TETRAHEDRON 1975,31(5), 449-54
- CHEMICAL ABSTRACTS, Band 83, 1975, Seite 574, Zusammenfassung Nr. 97160b,Columbus, Ohio, US; V.S. FEDOROV et al.: "Unsaturated derivatives of benzo-1,4-dioxane", & KHIM. GETEROTSIKL. SOEDIN.1975, (4), 469-72
- CHEMICAL ABSTRACTS, Band 84, 1976, Seite 406, Zusammenfassung Nr. 73777c,Columbus, Ohio, US; R.S. TEWARI et al.: "Generation and reactions of somedimethyl benzylphosphonate carbanions: synthesis of trans-diaryl-substitutedethylenes", & J. CHEM. ENG. DATA 1976, 21(1), 125-31
- CHEMICAL ABSTRACTS, Band 84, 1976, Seite 541, Zusammenfassung Nr. 150546f,Columbus, Ohio, US; R.K. SAHDEV et al.: "Synthesis of possible pyrethrumsynergists: some N-substituted amides of dillapiolic acid",& J. INST. CHEM., CALCUTTA 1975, 47, Pt. 6, 234-6
- JOURNAL OF ORGANIC CHEMISTRY, Band 39, Nr. 9, 1974, Seiten 1242-1247,
- Washington, US; D.T. WITIAK et al.: "Vilsmeier-Haack cyclizations. Synthesis of2-substituted 3-dimethylamino-5,6-methylenedioxyindenes and the correspondingindanones"

## Beschreibung

Die Erfindung betrifft kondensierte heterocyclische Verbindungen der allgemeinen Formel 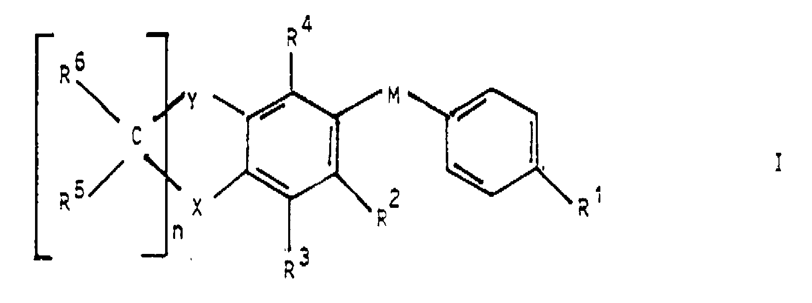
worin R¹ Wasserstoff, Acyl, nieder-Alkyl oder eine Gruppe -CHO, -CH₂OR¹⁰, -COR⁷, oder OR¹³; R², R³ und R⁴ Wasserstoff, nieder-Alkyl, nieder-Alkoxy oder Halogen: R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(R¹¹)=C(R¹²)-, -CONH-, oder -NH-CO-; R¹¹, R¹², R¹⁴ und R¹⁵ Wasserstoff oder nieder-Alkyl, R¹³ Wasserstoff, nieder-Alkyl, das durch Amino, mono- oder di-Alkylamino, Morpholino, Thiamorpholino oder Piperazino substituiert sein kann, oder nieder-Alkoxycarbonyl; und n 1, 2, 3 oder 4 bedeuten; und wobei "nieder" Gruppen mit 1-6 C-Atomen bezeichnet und Salze von Verbindungen der Formel I in denen R¹ eine Carboxygruppe darstellt zur Verwendung als Heilmittel.

Die Erfindung betrifft weiterhin neue Verbindungen der allgemeinen Formel I worin R¹ Acyl, oder eine Gruppe -CHO, -CH₂OR¹⁰, -COR⁷, oder OR¹³; R², R³ und R⁴ Wasserstoff, nieder-Alkyl, nieder-Alkoxy oder Halogen; R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(CH₃)=CH-, -CONH-, oder -NH-CO-; R¹⁴ und R¹⁵ Wasserstoff oder nieder-Alkyl, R¹³, nieder-Alkyl, das durch Amino, mono- oder di-Alkylamino, Morpholino, Thiamorpholino oder Piperazino substituiert ist, oder nieder-Alkoxycarbonyl; und n 1, 2, 3 oder 4 bedeuten; und wobei "nieder" Gruppen mit 1-6 C-Atomen bezeichnet und Salze von Verbindungen der Formel I in denen R¹ eine Carboxygruppe darstellt sowie ein Verfahren zur Herstellung der neuen Verbindungen der Formel I, pharmazeutische Präparate auf Basis der Verbindungen der Formel I, sowie die Verwendung der Verbindungen der Formel I bei der Herstellung von pharmazeutischen Präparaten zur Behandlung und Prophylaxe solcher Erkrankungen.

Die Bezeichnung "nieder" bezieht sich auf Gruppen mit 1-6 C-Atomen. Alkyl- und Alkoxygruppen können geradkettig oder verzweigt sein, wie Methyl, Aethyl, Propyl, Isopropyl, Butyl, sek.-Butyl oder tert.-Butyl bzw. Methoxy, Aethoxy, Propoxy, Isopropoxy, Butoxy, sek.-Butoxy und tert.-Butoxy. Beispiele von Acyloxygruppen sind Alkanoyloxygruppen, vorzugsweise nieder-Alkanoyloxygruppen, wie Acetoxy, Propionyloxy, Butyryloxy, Pivaloyloxy und Caproyloxy; oder Aroyloxygruppen wie Benzoyloxy, p-Nitrobenzoyloxy und Toluoyloxy; oder Aralkanoyloxygruppen wie Phenylacetoxy.

Eine bevorzugte Gruppe von Verbindungen der Formel I sind solche, in denen X und Y -O-, -S-, >SO, >SO₂ oder >NR¹⁸ sind, insbesondere solche, in denen X und Y -S- sind. Vorzugsweise ist n 2 oder 3, insbesondere 3. Bevorzugte Gruppen M sind -C(CH₃)=CH-; und -CONH-.

Die Verbindungen der Formel I können erfindungsgemäss dadurch hergestellt werden, dass man
a) eine Verbindung der allgemeinen Formel 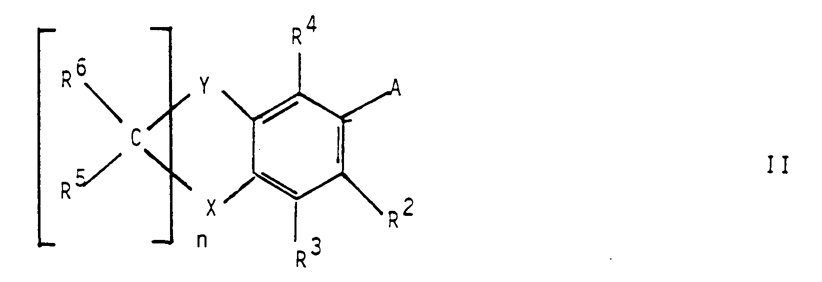 mit einer Verbindung der allgemeinen Formel 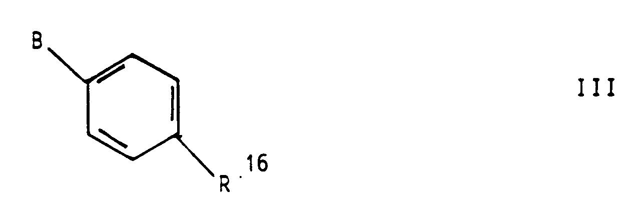 umsetzt, wobei entweder
   A einen der Reste -CH(CH₃)P⁺(Q)₃Z⁻ oder -CH(CH₃)-P(O)(OAlk)₂ und B einen Formylrest darstellt; oder
   A einen Rest CH₃CO und B einen der Reste -CH₂ P⁺(Q)₃Z⁻, oder -CH₂ -P-(O)(OAlk)₂ darstellt; Q Aryl; Z⁻ das Anion einer organischen oder anorganischen Säure; Alk eine niedere Alkylgruppe; und R¹⁶ einen Rest R¹ mit Ausnahme der Formyl-, Carboxyl-, und Hydroxymethylgruppe darstellt; oder
b) eine Verbindung der allgemeinen Formel 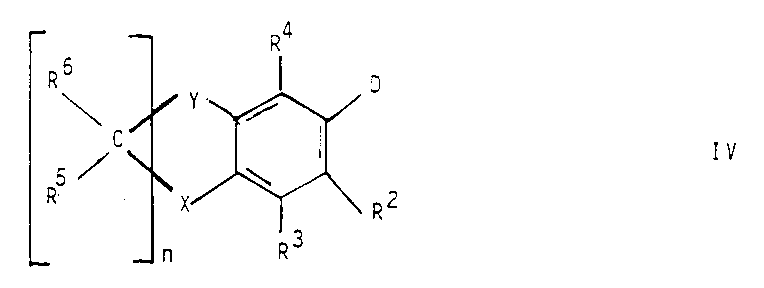 mit einer Verbindung der allgemeinen Formel 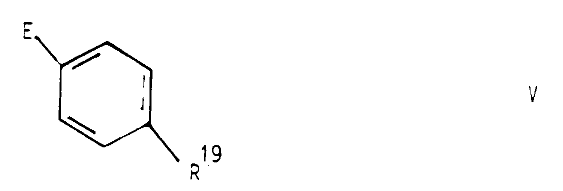 umsetzt,
   wobei entweder D eine Carboxylgruppe oder ein reaktionsfähiges Derivat davon und E eine Aminogruppe bedeutet, oder D eine Aminogruppe und E eine Carboxylgruppe oder ein reaktionsfähiges Derivat davon und R¹⁹ einen Rest R¹ mit Ausnahme der Carboxyl-, und Hydroxymethylgruppe bedeutet
c) eine Verbindung der allgemeinen Formel 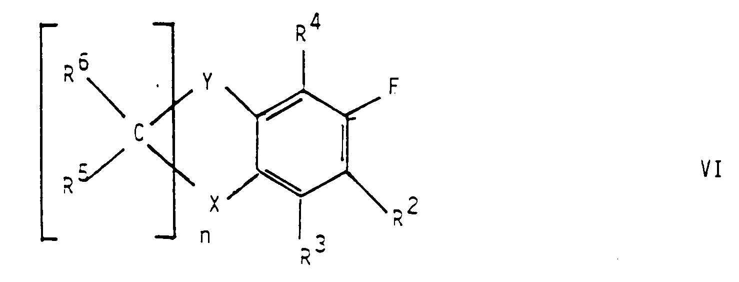 mit einer Verbindung der allgemeinen Formel 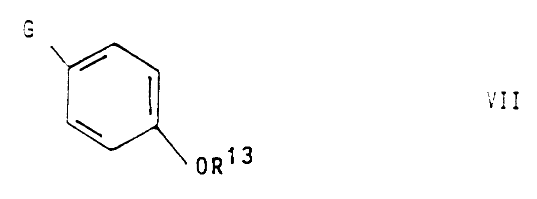 umsetzt und das Reaktionsprodukt dehydratisiert;
   wobei entweder F einen Rest -CH(CH₃)MgHal und G einen Formylrest ; oder F einen Formylrest und G einen Rest -CH₂ -MgHal; Hal Halogen darstellt;und
   wobei "nieder" Gruppen mit 1-6 C-Atomen bezeichnet und die übrigen Symbole in den Formeln II-VII die früher angegebene Bedeutung haben;
worauf man in dem erhaltenen Reaktionsprodukt der Formel I erwünschtenfalls den Rest R¹ funktionell abwandelt und/oder das Schwefelatom in einer Verbindung I, in der X und/oder Y -S- ist, zu einer Sulfoxyl- oder Sulfonylgruppe oxidiert.

Die Umsetzung der Verbindungen II und III gemäss Verfahrensvariante a) kann nach den bekannten Methoden der Wittig- bzw. Horner-Reaktion durchgeführt werden.

Bei der Wittig-Reaktion, d.h., bei Verwendung einer Verbindung der Formel II mit A = -CH(CH₃)P⁺(Q)₃Z⁻ oder der Formel III mit B = -CH₂P⁺(Q)₃Z⁻ werden die Komponenten in Gegenwart eines säurebindenden Mittels, z.B. in Gegenwart einer starken Base, wie z.B. Butyllithium, Natriumhydrid oder dem Natriumsalz von Dimethylsulfoxyd, oder K-tert.-Butylat, vornehmlich aber in Gegenwart eines gegebenenfalls durch niederes Alkyl substituierten Aethylenoxyds wie 1,2-Butylenoxyd, gegebenenfalls in einem Lösungsmittel, z.B. in einem Aether, wie Diäthyläther oder Tetrahydrofuran, oder in einem aromatischen Kohlenwasserstoff, wie Benzol in einem zwischen Raumtemperatur und dem Siedepunkt des Reaktionsgemisches liegenden Temperaturbereich miteinander umgesetzt.

Von den anorganischen Säureanionen Z⁻ ist das Chlor- und Brom-ion oder das Hydrosulfat-ion, von den organischen Säureanionen ist das Tosyloxy-ion bevorzugt. Der Arylrest Q ist vorzugsweise Phenyl.

Bei der Horner-Reaktion, d.h. bei Verwendung einer Verbindung der Formel II mit A = -CH(CH₃)P(O)(OAlk)₂ oder der Formel III mit B = -CH₂P(O)(OAlk)₂, werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid, Tetrahydrofuran, Dioxan oder 1,2-Dimethoxyalkan, oder auch mit Hilfe eines Natriumalkoholates in einem Alkanol, z.B. Natriummethylat in Methanol, in einem zwischen 0° und dem Siedepunkt des Reaktionsgemisches liegenden Temperaturbereich kondensiert. Die Alkoxyreste in A und B sind vornehmlich niedere Alkoxyreste mit 1-6 Kohlenstoffatomen, wie Methoxy oder Aethoxy.

Gemäss Verfahrensvariante a) werden Verbindungen der Formel I erhalten, in denen M eine Gruppe -C(CH₃)=CH-darstellt.

Die Umsetzung einer Verbindung IV mit einer Verbindung V gemäss Verfahrensvariante b) kann nach an sich bekannten Methoden für die Acylierung von Aminen durchgeführt werden. Vorzugsweise wird eine Verbindung der Formel IV, in der D eine Carbonsäurehalogenidgruppe, z.B. die Gruppe -COCl darstellt, mit einer Verbindung der Formel V, in der E -NH₂ ist, zu einer Verbindung der Formel I in der M -CONH- ist, umgestzt, oder ein Amin der Formel IV mit einem Carbonsäurehalogenid der Formel V zu einer Verbindung der Formel I umgesetzt, in der M -NH-CO- ist.

Diese Acylierungen werden zweckmässig in Gegenwart einer Base, z.B. einer organischen Base, wie Pyridin durchgeführt.

Die Umsetzung der Verbindungen der Formeln VI und VII kann in an sich bekannter Weise unter den Bedingungen einer Grignard-Reaktion vorgenommen werden, z.B. in einem Aether, wie Diäthyläther oder Tetrahydrofuran, bei Raumtemperatur und anschliessender Wasserabspaltung mit sauren Agentien, z.B. mit organischen Säuren, wie p-Toluolsulfonsäure.

Nach dieser Verfahrensvariante erhält man Verbindungen der Formel I, in denen M eine Gruppe -C(CH₃)=CH- und R¹ einen wie oben definierten Rest OR¹³ darstellt.

Als funktionelle Abwandlung eines Substituenten R¹ in einer erhaltenen Verbindung der Formel I kommen z.B. die Verseifung eines Carbonsäureesters oder dessen Reduktion zur Hydroxymethylgruppe in Betracht. Die Hydroxymethylgruppe kann auch zur Formylgruppe oxidiert, oder verestert oder veräthert werden. Eine Carboxylgruppe kann dann weiterhin in ein Salz, einen Ester, ein Amid oder die Hydroxymethylgruppe umgewandelt werden.

Alle diese Abwandlungen können nach an sich bekannten Methoden vorgenommen werden.

Ein Carbonsäureester der Formel I kann wie nachstehend beschrieben, unmittelbar amidiert werden, oder in an sich bekannter Weise, z.B. durch Behandlung mit Alkalien, insbesondere durch Behandeln mit wässriger alkoholischer Natron- oder Kalilauge in einem zwischen Raumtemperatur und dem Siedepunkt des Reaktionsgemisches liegenden Temperaturbereich zur Carbonsäure hydrolysiert werden, die wiederum über ein Säurehalogenid amidiert werden kann.

Eine Carbonsäure der Formel I kann in an sich bekannter Weise, z.B. durch Behandeln mit Thionylchlorid, oder Phosphortrichlorid in Toluol oder Oxalylchlorid in DMF/Benzol in das Säurechlorid übergeführt werden, das durch Umsetzen mit Alkoholen in Ester, mit Aminen in das entsprechende Amid umgewandelt werden kann.

Ein Carbonsäureester der Formel I kann z.B. durch Behandeln mit Lithiumamid direkt in das entsprechende Amid umgewandelt werden. Das Lithiumamid wird vorteilhaft bei Raumtemperatur mit dem betreffenden Ester zur Reaktion gebracht.

Eine Carbonsäure oder ein Carbonsäureester der Formel I kann in an sich bekannter Weise zu dem entsprechenden Alkohol der Formel I reduziert werden. Die Reduktion wird voreilhaft mit Hilfe eines Metallhydrids oder Alkylmetallhydrids in einem inerten Lösungsmittel durchgeführt. Als Hydride haben sich vor allem gemischte Metallhydride, wie Lithiumaluminiumhydrid oder bis-[Methoxy-äthoxy]-natriumaluminiumdihydrid als geeignet erwiesen. Als Lösungsmittel verwendbar sind u.a. Aether, Tetrahydrofuran oder Dioxan, wenn Lithiumaluminiumhydrid verwendet wird; und Aether, Hexan, Benzol oder Toluol, wenn Diisobutylaluminiumhydrid oder bis-[Methoxy-äthoxy]natriumaluminiumdihydrid eingesetzt werden.

Ein Alkohol der Formel I kann z.B. in Gegenwart einer Base, vorzugsweise in Gegenwart von Natriumhydrid, in einem organischen Lösungsmittel wie Dioxan, Tetrahydrofuran, 1,2-Dimethoxyäthan, Dimethylformamid, in einem zwischen 0° und Raumtemperatur liegenden Temperaturbereich mit einem Alkylhalogenid, z.B. mit Methyljodid, veräthert werden.

Ein Alkohol der Formel I kann auch durch Behandeln mit einem Alkanoylhalogenid oder Anhydrid, zweckmässig in Gegenwart einer Base, beispielsweise in Gegenwart von Pyridin oder Triäthylamin in einem zwischen Raumtemperatur und dem Siedepunkt des Reaktionsgemisches liegenden Temperaturbereich verestert werden.

Die Carbonsäuren der Formel I bilden mit Basen, insbesondere mit den Alkalimetallhydroxyden, vorzugsweise mit Natrium- oder Kaliumhydroxyd, Salze.

Eine Verbindung der Formel I, in der X und/oder Y für -S- stehen, kann mit an sich bekannten Methoden zu einer Verbindung der Formel I, in der X und/oder Y für =SO oder =SO₂ stehen, oxidiert werden. Die Oxidation zur Sulfoxidgruppe kann mit Oxidationsmitteln wie Perjodaten. z.B. NaJO₄ oder mit organischen Persäuren, wie m-Chlorperbenzoesäure vorgenommen werden. Bei der Oxidation mit organischen Persäuren setzt man etwa ein Aequivalent Persäure ein, um eine Sulfoxidverbindung (X/Y = SO) zu erhalten, wogegen die Verwendung von zwei Aequivalenten Persäure zu Sulfonen (X/Y = SO₂) führt.

Die Verbindungen der Formel I in denen M eine C-C-Doppelbindung beinhaltet, können in trans- oder cis-Form vorliegen. Bei der Herstellung fallen sie mehrheitlich in der trans-Form an. Gegebenenfalls anfallende cis-Anteile können in an sich bekannter Weise, falls erwünscht, abgetrennt oder isomerisiert werden.

Die als Ausgangsmaterial für die Herstellung der Verbindungen der Formel I verwendeten Verbindungen der Formeln II-V können, soweit sie nicht bekannt oder nachstehend beschrieben sind, in Analogie zu bekannten oder den nachstehend beschriebenen Methoden hergestellt werden.

Die Dokumente Tetrahedron 31, (1975) Seiten 449-454; Chemical Abstracts (1975) 83:97160 b; J. Chem. Eng. Data 21, Nr.1 (1976), Seiten 125-131; und J. Org. Chem. 39, Nr. 9 (1974), Seiten 1242-1247 beschreiben Styryl-1,3-benzodioxolan- und -1,4-benzodioxanderivate.

Die Dokumente Tetrahedron Letters 29, Nr. 22 (1988), Seiten 2741-2744; und Chemical Abstracts (1976) 84:150 547 g beschreiben Phenylcarbamoyl-1,3-benzodioxolanderivate. Das Dokument DE-A-2 015 351 beschreibt Benzamido-tetrahydrochinolinderivate. Von den in den obengenannten Dokumenten beschriebenen Verbindungen war keine medizinische Anwendung bekannt. Die Dokumente GB-A-2 119 801, EP-A-0 130 795 und WO-A-85 00806 beschreiben Phenylpropenyl- (oder Styryl)benzopyran, benzothiopyran-, benzothiopyran-1-oxid-, benzothiopyran-1,1-dioxid-, und tetrahydrochinolin-Derivate mit ähnlicher Wirkungsrichtung wie die vorliegenden Verbindungen. Das Dokument LU-A-0 086 259 beschreibt Phenylcarbamoyl-tetrahydronaphthalin-, benzopyran- und benzothiopyran-Derivate mit ähnlicher Wirkungsrichtung wie die vorliegenden Verbindungen.

Die Verfahrensprodukte der Formel I und ihre physiologisch verträglichen Salze stellen pharmakodynamisch wertvolle Verbindungen dar. Sie können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen sowie lichtgeschädigter (Alters)-haut geeignet. Die Verfahrensprodukte der Formel I können ferner auch zur Bekämpfung von Schleimhauterkrankungen mit entzündlichen oder degenerativen bzw. metaplastischen Veränderungen eingesetzt werden. Im Papillomtest (Europ.J.Cancer Vol. 10, pp.731-737, 1974) zeigten der p-[2-(3,4-Dihydro-4,4-dimethyl-2H-1-benzopyran -7-yl)propenyl]benzoesäureäthylester (Beispiel 1) und die p-[2-(1,2,3,4-Tetrahydro-1,1,4-trimethyl -7-chinolinyl)propenyl]benzoesäure (Beispiel 5) in einer Dosierung von 6 mg/kg/Woche eine Papillomregression von 66 bzw. 42%; die p[2-(3,4-Dihydro-4,4-dimethyl-2H-1-thiobenzopyran -7-yl)propenyl]benzoesäure (Beispiel 3) mit 3 mg/kg/Woche eine Papillomregression von 61%, und der p[(E)-2-(3,4-Dihydro-3,3-dimethyl-2H-1,5-benzodithiepin -7-yl)propenyl]benzosäureäthylester mit 50 mg/kg/Woche eine Papillomregression von 50%.

Die Verbindungen der Formel I können auch zur Behandlung entzündlicher, allergischer, rheumatischer und immunologischer Erkrankungen der verschiedensten Organe verwendet werden. Beispiele solcher Krankheiten sind: Primär-chronische Polyarthritis, Spondylarthritis ancylopoetica, Osteoarthritiden, Arthritiden und Arthrosen; Ekzeme, atopische Dermatitis, Rhinitis allergica, Asthma bronchiale; Autoimmunkrankheiten wie z.B. Lupus erythematosus, Reiter's Syndrom.

Die Verbindungen der Formel I und deren Salze können deshalb als Heilmittel, z.B. in Form pharmazeutischer Präparate, Anwendung finden.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Die Dosierungen, in denen die Präparate verabreicht werden, können je nach Anwendungsart und Anwendungsweg sowie nach den Bedürfnissen der Patienten variieren.

Bei oraler Verabreichung der erfindungsgemässen Verbindungen kommen beim Erwachsenen Dosierungen von etwa 0,1-100 mg/kg, vorzugsweise 0,5-50 mg/kg pro Tag in Betracht.

Die Präparate können in einer oder mehreren Dosierungen verabreicht werden. Eine bevorzugte Darreichungsform sind Kapseln mit einem Gehalt von ca. 5-500 mg Wirkstoff.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen, Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-γ-tocopheramin sowie t-Butyl-Hydroxyanisol oder t-Butyl-Hydroxytoluol beigemischt sein.

### Beispiel 1

0,5 g einer 50%igen Suspension von Natriumhydrid in Mineralöl wurden in 15 ml Dimethylsulfoxid suspendiert und unter Argon mit einer Lösung von 3,2 g Diäthyl(4-carbäthoxybenzyl)phosphonat in 10 ml Dimethylsulfoxid versetzt. Man erwärmte anschliessend 20 Minuten auf 40°C, kühlte auf Raumtemperatur ab, tropfte eine Lösung von 0,87 g 3,4-Dihydro-4,4-dimethyl-7-acetyl-2H -1-benzopyran in 5 ml Dimethylsulfoxid hinzu und erwärmte 1 Stunde auf 40°C. Das erhaltene Reaktionsgemisch wurde auf Eiswasser gegossen, mit 1N Salzsäure angesäuert unhd mehrfach mit Aether extrahiert. Die organische Phase wurde mit Wasser gewaschen, getrocknet und eingedampft. Man erhielt ein gelbliches Oel, das nach Filtration über eine kleine Säule (Kieselgel, Eluierungsmittel Hexan/Essigester = 9:1) aus Hexan kristallisierte und 0,9 g p-[2-(3,4-Dihydro-4,4-dimethyl-2H-1-benzopyran -7-yl)propenyl]benzoesäure-äthylester, Smp. 48-50°C, lieferte.

Das als Ausgangsprodukt verwendete 3,4-Dihydro-4,4-dimethyl-7-acetyl-2H-1-benzopyran kann wie folgt hergestellt werden:

Ein Gemisch aus 20 g 3-Bromphenol, 92 g Acrylsäureäthylester und 1,8 ml Triton B (35% in Methanol) wurde über Nacht am Rückfluss gekocht. Nach dem Abdestillieren des überschüssigen Acrylesters bei Normaldruck wurde der Rückstand im Hochvakuum destilliert. Man erhielt 16,7 g 3-(m-Bromphenoxy)propionsäure-äthylester als farbloses Oel, Siedepunkt 107-110°C/67 Pa.

4,4 g Magnesium-Späne wurden mit 40 ml Aether überdeckt und innerhalb 30 Minuten mit einer Lösung von 32 g Methyljodid in 70 ml Aether versetzt. Nach weiterem 30-minütigem Kochen unter Rückfluss tropfte man unter Kühlung eine Lösung von 16,7 g 3-(m-Bromphenoxy)propionsäure-äthylester in 70 ml Benzol hinzu. Das Reaktionsgemisch wurde 1,5 Stunden zum Rückfluss erhitzt und anschliessend unter Eiskühlung mit 210 ml gesättigter Ammoniumchloridlösung versetzt. Nach mehrmaligem Extrahieren des Reaktionsgemisches mit Aether, Waschen der organischen Phase mit Wasser, Trocknen und Eindampfen erhielt man ein Oel, das durch Filtration über eine kleine Säule (SiO₂, Eluierungsmittel Hexan/Essigester = 9:1) weiter gereinigt wurde. Man erhielt 14 g 4-(m-Bromophenoxy)-2-methyl -2-butanol als farbloses Oel, Siedepunkt 106°C/40 Pa.

9,8 g Aluminiumchlorid wurden in 100 ml Nitromethan gelöst und im Laufe von 40 Minuten bei Raumtemperatur mit einer Lösung von 14 g 4-(m-Bromophenoxy)-2-methyl -2-butanol in 100 ml Nitromethan versetzt. Man rührte weitere 1,5 Stunden bei Raumtemperatur, kühlte auf 0°C und tropft nacheinander 200 ml 2N Salzsäure und 300 ml Wasser hinzu.

Nach dem Extrahieren des Reaktionsgemisches mit Aether, Trocknen und Eindampfen der organischen Phase wurde das so erhaltene rotbraune Oel zunächst über eine Säule filtriert (SiO₂, Eluierungsmittel Hexan/Essigester = 9:1) und anschliesend am Hochvakuum destilliert. Man erhielt 8 g 7-Bromo-3,4-dihydro-4,4-dimethyl-2H-1-benzopyran als farbloses Oel, Siedepunkt 82-86°C/0,5 nm. Die Substanz enthielt noch etwas 5-Bromo-3,4-dihydro-4,4-dimethyl -2H-1-benzopyran, und wurde in dieser Form in der nächsten Stufe eingesetzt.

1 g Magnesium-Späne wurden mit 10 ml absolutem Tetrahydrofuran überschichtet und bei 50-60°C unter Verwendung eines Ultraschallbades mit einer Lösung von 10 g 7-Bromo-3,4-dihydro-4,4-dimethyl -2H-1-benzopyran in 50 ml Tetrahydrofuran tropfenweise versetzt. Danach erwärmte man weitere 2 Stunden auf 70°C, kühlte auf 0°C ab und tropfte eine Lösung von 9,5 g Acetaldehyd in 30 ml Tetrahydrofuran hinzu. Man rührte eine weitere Stunde bei Raumtemperatur, goss auf Eis/gesättigte Ammoniumchloridlösung, extrahierte mit Aether, wusch mit Wasser, trocknete und dampfte ein. Nach Chromatographie (Kieselgel, Eluierungsmittel Hexan/Essigester 4:1) erhielt man 6,9 g 3,4-Dihydro-α,4,4-trimethyl -7-(2H-1-benzopyran)-methanol als viskoses Oel.

5,9 g 3,4-Dihydro-α,4,4-trimethyl -7-(2H-1-benzopyran)-methanol wurden in 200 ml Methylenchlorid gelöst und mit 25 g Braunstein versetzt. Nach 3-stündigem Rühren bei Raumtemperatur filtrierte man das Reaktionsgemisch und dampfte das Filtrat ein. Man erhielt 5,8 g 3,4-Dihydro-4,4-dimethyl-7-acetyl-2H-1-benzopyran als farbloses Oel.

### Beispiel 2

0,5 g Aethylester aus Beispiel 1 wurden in 20 ml Aethanol gelöst und mit einer Lösung von 0,8 g Kaliumhydroxid in 5 ml Wasser versetzt. Nach 2-stündigem Rühren bei 50°C wurde auf Eiswasser gegossen, mit 2N Salzsäure angesäuert und mehrfach mit Essigester extrahiert. Nach Waschen der organischen Phase mit Wasser, Trocknen über Natriumsulfat, Eindampfen und Kristallisation aus Essigester wurden 350 mg p-[2-(3,4-Dihydro-4,4-dimethyl-2H-1-benzopyran -7-yl)propenyl)benzoesäure, Smp. 242-244°C, erhalten.

### Beispiel 3

18,9 g [1(3,4-Dihydro-4,4-dimethyl-2H-1-benzothiopyran -7-yl)äthyl]]triphenylphosphoniumbromid wurden in 200 ml 1,2-Butylenoxid suspendiert und mit 5,7 g 4-Formyl-bensoesäureäthylester während 16 Stunden am Rückfluss erhitzt. Nach dem Abkühlen wurde das klare Reaktionsgemisch auf ein Gemisch aus Methanol und Wasser (6:4), gegossen, dreimal mit Hexan extrahiert, mit Methanol/Wasser (6:4) und Wasser gewaschen, getrocknet und eingedampft. Das erhaltene gelbe Oel wurde über Kieselgel filtriert (Eluierungsmittel Hexan/Essigester = 19:1) und aus Hexan/Essigester kristallisiert. Man erhielt 5,9 g Aethyl p-[2-(3,4-dihydro-4,4-dimethyl-2H-1-benzothiopyran -7-yl)propenyl]benzoat in farblosen Kristallen, Smp. 64-65°C.

1 g des so erhaltenen Esters wurden in 20 ml Aethanol gelöst und mit einer Lösung von 1,6 g Kaliumhydroxid in 10 ml Wasser versetzt. Nach 2-stündigem Erwärmen auf 50°C wurde auf Eis gegossen, mit 2N Salzsäure angesäuert und mehrfach mit Essigester extrahiert. Der nach dem Eindampfen erhaltene kristalline Rückstand wurde aus Essigsäure umkristallisiert. Man erhielt 0,6 g p-[2-(3,4-Dihydro-4,4-dimethyl -2H-1-benzothiopyran-7-yl)propenyl]benzoesäure, Smp. 255-257°C.

Das als Ausgangsmaterial verwendete Phosphoniumsalz lässt sich wie folgt herstellen:

36 g 3-Bromthiophenol wurden in 400 ml Dimethylformamid gelöst. Nach Zugabe von 27 g fein pulverisiertem Kaliumcarbonat und 29 g 3,3-Dimethyl-allylbromid rührte man 1 Stunde bei Raumtemperatur, verdünnte mit 500 ml Wasser, säuerte unter Kühlung mit 3N Salzsäure an und extrahierte mit Aether. Das nach dem Eindampfen erhaltene gelbbraune Oel wurde am Hochvakuum destilliert. Man erhielt 48 g m-Bromphenyl -3-methyl-2-butenylsulfid als farblose Flüssigkeit, Siedepunkt 85°C/13,3 Pa.

46,5 g dieses Produkts wurden in 800 ml Toluol gelöst und nach Zugabe von 45 g p-Toluolsulfonsäure-monohydrat am Wasserabscheider während 10 Stunden gekocht. Das erkaltete Reaktionsgemisch wurde mit Wasser verdünnt, durch Zugabe von wässriger Natriumbicarbonat-Lösung neutralisiert und mit Essigester extrahiert. Das nach dem Eindampfen des Lösungsmittels erhaltene gelbbraune Oel wurde am Hochvakuum destilliert. Man erhielt 39 g 7-Brom-3,4-dihydro-4,4-dimethyl-2H-1-benzothiopyran als schwach gelbliches Oel, Siedepunkt 90-93°C/13,3 Pa. Die Die Substanz enthielt ca. 15% 5-Brom-3,4-dihydro-4,4-dimethyl -2H-1-benzothiopyran und wurde so in der nächsten Stufe eingesetzt.

1,85 g Magnesium-Späne wurden mit 20 ml abs. Tetrahydrofuran überschichtet. Unter Erwärmen zum Rückfluss tropfte man eine Lösung von 10,8 g des in der vorigen Stufe erhaltenen Gemisches in 80 ml Tetrahydrofuran hinzu und kochte, bis praktisch alles Magnesium gelöst war. Nach dem Abkühlen auf 0°C tropfte man eine Lösung von 8,5 g Acetaldehyd in 50 ml Tetrahydrofuran hinzu und rührte noch 30 Minuten bei Raumtemperatur. Das Reaktionsgemisch wurde auf eiskalte Ammoniumchlorid-Lösung gegossen, mit Essigester extrahiert, mit Wasser gewaschen und eingedampft. Nach Filtration über Kieselgel (Eluierungsmittel Hexan/Essigester = 19:1) erhielt man 13,5 g 3,4-Dihydro-α,4,4-trimethyl -7-(2H-1-benzothiopyran)methanol als schwachgelbes, viskoses Oel.

Dieses Oel wurde in 250 ml Acetonitril gelöst und mit 23 g Triphenylphosphin-Hydrobromid versetzt. Nach 20-stündigem Erwärmen auf 60°C dampfte man das Reaktionsgemisch ein, nahm den Rückstand mit 500 ml 80%-igem wässrigen Aethanol auf, extrahierte dreimal mit Hexan, dampfte die Aethanol-Lösung ein, löste erneut in Methylenchlorid, trocknete über Natriumsulfat und dampfte ein. Man erhielt einen schaumigen Rückstand, der durch Verreiben mit Aether in eine amorphe, filtrierbare Substanz überführt wurde, wobei 26 g [1-(3,4-Dihydro-4,4-dimethyl -2H-1-benzothiopyran-7-yl)äthyl]triphenylphosphoniumbromid erhalten wurde.

### Beispiel 4

2,5 g Aethyl p-[2-(3,4-dihydro-4,4-dimethyl -2H-1-benzothiopyran-7-yl)propenyl]benzoat wurden in 30 ml Chloroform gelöst und bei 0°C tropfenweise mit einer Lösung von 2,7 g 90%iger m-Chlorperbenzoesäure in 40 ml Chloroform versetzt. Je nach Gehalt der Persäure muss noch etwas m-Chlorperbenzoesäure zugegeben werden bis zum völligen Verschwinden des intermediär entstehenden Sulfoxides. Das Reaktionsgemisch wurde mit Chloroform verdünnt, zweimal mit eiskalter verdünnter, wässriger Natriumcarbonat-Lösung ausgeschüttelt, zweimal mit Wasser gewaschen, getrocknet und eingedampft. Nach Chromatographie des Rohproduktes (Kieselgel, Eluierungsmittel Hexan/Essigester = 4:1) und Kristallisation aus Essigester/Hexan erhielt man 1,4 g Aethyl-p-[2-(3',4'-dihydro -4',4'-dimethyl-2'H-1-benzothiopyran -7'-yl)propenyl]benzoat-1',1'-dioxid in farblosen Kristallen, Smp. 90-91°C.

Hydrolyse des so erhaltenen Aethylesters mit Kaliumhydroxid in wässrig-äthanolischer Lösung in Analogie zu Beispiel 3 ergab nach Umkristallisation aus Tetrahydrofuran/Essigester/Hexan p-[2-(3',4'-Dihydro-4',4'-dimethyl-2'H -1-benzothiopyran-7'-yl)propenyl]benzosäure 1',1'-dioxid in farblosen Kristallen, Smp. 263-265°C.

### Beispiel 5

2,9 g einer 50%-igen Suspension von Natriumhydrid in Mineralöl wurden nach 2-maligem Waschen mit Pentan in 30 ml Dimethylformamid suspendiert und bei Raumtemperatur tropfenweise mit einer Lösung von 17,3 g Diäthyl(4-carbäthoxybenzyl)phosphonat in 50 ml Dimethylformamid versetzt. Nach einstündigem Rühren bei Raumtemperatur tropfte man eine Lösung von 5 g 1,2,3,4-Tetrahydro-1,4,4-trimethyl -7-chinolinyl-methylketon in 50 ml Dimethylformamid hinzu. Das Reaktionsgemisch wurde 1 Stunde auf 50°C erwärmt, auf Eiswasser gegossen und mit Essigester extrahiert. Das nach dem Trocknen und Eindampfen der organischen Phase erhaltene bräunliche Oel wurde chromatographiert (Kieselgel, Eluierungsmittel Hexan/Essigester = 9:1) und ergab 4,2 g Aethyl p-[2-(1,2,3,4-Tetrahydro-1,4,4-trimethyl -7-chinolinyl)propenyl]benzoat als schwachgelbes, viskoses Oel.

3,2 g dieses Oeles wurden in 30 ml Aethanol gelöst und mit einer Lösung von 5 g Kaliumhydroxid in 20 ml Wasser versetzt. Nach 2-stündigem Erwärmen auf 50°C wurde auf Eiswasser gegossen, die Lösung durch Zugabe von 1N Salzsäure auf pH 5 gestellt und mit Methylenchlorid extrahiert. Das nach Trocknen und Eindampfen der organischen Phase erhaltene Rohprodukt wurde aus Essigester/Hexan umkristallisiert und ergab 1,4 g p-[2-(1,2,3,4-Tetrahydro-1,4,4-trimethyl -7-chinolinyl)propenyl]benzoesäure in grauen Kristallen, Smp. 194-196°C.

Das als Ausgangsmaterial verwendete 1,2,3,4-Tetrahydro-1,4,4-trimethyl -7-chinolinyl-methylketon lässt sich wie folgt herstellen:
55,4 g 3-Bromanilin wurden in 300 ml Hexan gelöst und mit 76 ml Triäthylamin versetzt. Unter Eiskühlung tropfte man eine Lösung von 40 g 3,3-Dimethylacrylsäurechlorid in 300 ml Hexan hinzu. Nach 3-stündigem Kochen am Rückfluss goss man auf Eiswasser und extrahierte mit Aether. Die organische Phase wurde mit Wasser gewaschen, getrocknet und eingedampft. Man erhielt ein braunes Oel, das am Hochvakuum destilliert wurde. Die Ausbeute betrug 51 g N-(3-Bromphenyl)-3,3-dimethyl-acrylsäureamid (gelbes Oel), Siedepunkt 146-152°C/93 Pa.

28,7 g pulverisiertes Kaliumhydroxid wurden in 300 ml Dimethylsulfoxid suspendiert und eine Lösung von 32,6 g N-(3-Bromphenyl)-3,3-dimethyl-acrylsäureamid in 200 ml DMSO tropfenweise dazugegeben. Man rührte 30 Minuten bei Raumtemperatur und tropfte anschliessend eine Lösung von 27,5 g Methyljodid in 150 ml DMSO hinzu. Nach 2,5 Stunden goss man auf 1 L Eiswasser und extrahierte mit Essigester. Die organische Phase wurde mehrfach mit Wasser gewaschen, getrocknet und eingedampft. Der ölige Rückstand wurde am Hochvakuum destilliert. Man erhielt 29,3 g N-Methyl-N-(3-Bromphenyl) -3,3-dimethyl-acrylsäureamid als schwachgelbes Oel, Siedepunkt 112-114°C/67 Pa.

29,3 g dieses Oeles wurden in 1 L hochsiedendem Petroläther gelöst. Unter Rühren gab man 30 g Aluminiumchlorid hinzu und kochte anschliessend 3 Stunden am Rückfluss. Nach dem Abkühlen auf 0-5°C tropfte man langsam 700 ml 2N Salzsäure hinzu. verdünnte mit Wasser und extrahierte mit Aether. Das nach Trocknen und Eindampfen des Lösungsmittels erhaltene gelbbraune Oel war ein 1:1-Gemisch von 5-Brom- und 7-Brom-3,4-dihydro-1,4,4-trimethyl-2(1H)-chinolinon. Durch Säulenchromatographie (Kieselgel, Eluierungsmittel Hexan/Essigester 9:1) erhielt man zuerst die 7-Brom-Verbindung die aus Hexan kristallisierte, Ausbeute 14,2 g, Schmelzpunkt 92-94°C.

14,2 g der 7-Brom-Verbindung wurden in 300 ml Tetrahydrofuran gelöst und bei 0°C tropfenweise mit einer Lösung von 5,7 ml Boran-Dimethylsulfid-Komplex (ca. 10 Molar in überschüssigem Dimethylsulfid) in 250 ml Tetrahydrofuran versetzt. Das Reaktionsgemisch wurde anschliessend unter Argon während 2,5 Stunden zum Rückfluss erwärmt, auf 0°C abgekühlt und tropfenweise nacheinander mit 700 ml Methanol und 300 ml 6N Salzsäure versetzt. Nach 30 Minuten wurde das Gemisch im Wasserstrahlvakuum eingedampft, der flüssige Rückstand mit Eis versetzt, durch Zugabe von eiskalter 3N Natronlauge alkalisch gestellt und mit Aether extrahiert. Nach Trocknung und Eindampfen des Lösungsmittels erhielt man ein gelbliches Oel, das am Hochvakuum destilliert wurde und 11,8 g 7-Brom-1,2,3,4-tetrahydro-1,4,4-trimethylchinolin als farbloses Oel, Siedepunkt 124-127°C/120 Pa lieferte. Die Substanz erstarrte beim Stehen im Kühlschrank.

2 g Magnesium-Späne wurden mit 20 ml Tetrahydrofuran überschichtet. Unter Verwendung eines Ultraschallbades tropfte man bei 55-60°C eine Lösung von 20,8 g 7-Brom-1,2,3,4-tetrahydro-1,4,4-trimethylchinolin in 80 ml Tetrahydrofuran hinzu. Danach kochte man noch 2 Stunden am Rückfluss, kühlte auf 0°C ab und tropfte eine Lösung von 10 g Acetaldehyd in 80 ml Tetrahydrofuran dazu. Nach 1-stündigem Rühren bei Raumtemperatur goss man aus Eis/gesättigte Ammoniumchlorid-Lösung und extrahierte mit Aether. Das nach Trocknen und Eindampfen des Lösungsmittels erhaltene Oel wurde über eine Kieselgelsäule filtriert (Eluierungsmittel Hexan/Essigester 9:1) und ergab 14,5 g 1,2,3,4-Tetrahydro-α,1,4,4-tetramethyl -7-chinolin-methanol als gelbliches Oel.

5,5 g Oxalylchlorid wurden in 50 ml Methylenchlorid gelöst. Bei -60°C tropfte man ein Gemisch von 6 ml Dimethylsulfoxid und 35 ml Methylenchlorid hinzu und nach 5 Minuten eine Lösung von 8,5 g des in der vorigen Stufe erhaltenen Oeles in 85 ml Methylenchlorid. Man rührte noch 15 Minuten bei -60°C und tropfte dann bei dieser Temperatur 28 ml Trimethylamin dazu. Nach Entfernen des Kühlbades wurde das Reaktionsgemisch 2 Stunden bei Raumtemperatur gerührt, anschliessend auf Eis gegossen und mit Aether extrahiert. Die organische Phase wurde mit Wasser gewaschen, getrocknet und eingedampft. Nach Filtration des Rohproduktes über eine kleine Säule (Kieselgel, Eluierungsmittel Hexan/Essigester = 9:1) und Kristallisation aus Hexan erhielt man 5 g 1,2,3,4-Tetrahydro-1,4,4-trimethyl-7-chinolinyl-methylketon in schwach gelblichen Kristallen, Smp. 46-48°C.

### Beispiel 6

In Analogie zu Beispiel 5 erhielt man aus 6,5 g 6-Acetyl-1,4-benzodioxan und 16,4 g Diäthyl 4-carbäthoxybenzyl)phosphonat nach Filtration des Rohproduktes über eine Säule (Kieselgel, Eluierungsmittel Hexan/Essigester = 9:1) und Umkristallisation aus Hexan/Essigester 7,3 g Aethyl p-[(E)-2-(1,4-benzodioxan-6-yl)propenyl]benzoat in farblosen Kristallen, Smp. 64-66°C.

In Analogie zu Beispiel 2 erhielt man durch Hydrolyse des so erhaltenen Esters p-[(E)-2-(1,4-Benzodioxan-6-yl)propenyl]benzoesäure in weissen Kristallen, Smp. 172-173°C (aus Essigester).

### Beispiel 7

In Analogie zu Beispiel 5 erhielt man aus 3,2 g 6-Acetyl-1,4-benzodithian und 7,4 g Diäthyl 4-carbäthoxybenzyl)phosphonat nach Filtration des Rohproduktes über eine Säule (Kieselgel, Hexan/Essigester = 4:1) und Umkristallisation aus Hexan/Essigester 3,1 g Aethyl p-[(E)-2-(1,4-benzodithian-6-yl)propenyl]benzoat in farblosen Kristallen, Smp. 90-92°C.

In Analogie zu Beispiel 2 erhält man durch Hydrolyse des so erhaltenen Esters mit Kaliumhydroxid und Umkristallisation aus Essigester/Hexan p-[(E)-2-(1,4-Benzodithian-6-yl)propenyl]benzoesäure in weissen Kristallen, Smp. 236-237°C.

### Beispiel 8

15,1 g α,2,2-Trimethyl-1,3-benzodioxol-5-methanol wurden in 450 ml Acetonitril gelöst und mit 24,6 g Triphenylphosphin-hydrobromid 3,5 Stunden bei 50°C gerührt. Danach wurde im Vakuum eingedampft. Der Rückstand wurde zwischen 80-proz. Aethanol und Hexan (je dreimal 600 ml) verteilt; die wässrige Aethanolphase im Vakuum eingedampft, in Dichlormethan aufgenommen, über Natriumsulfat getrocknet, eingedampft und im Vakuum getrocknet: 39,6 g [α-(2,2-Dimethyl-1,3-benzodioxol-5-yl)äthyl] triphenylphosphoniumbromid als beiger Schaum, der nicht kristallisierte, aber nach Dünnschichtchromatographie (CH₂Cl₂+5% MeOH) praktisch rein war.

12,5 g dieses Phosphoniumsalzes und 4,7 g 4-Formylbenzoesäuremethylester wurden in 80 ml 1,2-Butylenoxid unter Argon 16 Stunden unter Rückfluss gekocht. Nach dem Abkühlen wurde das Produkt zwischen Hexan (dreimal 300 ml) und 70-proz. Aethanol (dreimal 150 ml) verteilt. Die wässrigen Aethanolphasen wurden anschliessend noch dreimal mit Hexan und 10% Aethylacetat extrahiert. Nach dem Eindampfen wurden 5,46 g Hexan-Extrakt (A) und 1,23 g Hexan-Aethylacetat-Extrakt (B) erhalten. Extrakt A lieferte aus Hexan 2,39 g kristallinen, reinen p-[(E)-2-(2,2-Dimethyl-1,3-benzodioxol-5-yl)propenyl] benzoesäuremethylester, Smp. 72-74°C. Die Mutterlaugen von Extrakt A und Extrakt B wurden gemeinsam an Kieselgel mit Hexan+5% Aethylacetat chromatographiert, wobei noch 0,7 g Produkt vom Schmelzpunkt 73-75°C erhalten wurden

### Beispiel 9

A. Zu 3,87 g Natriumhydrid (50% in Mineralöl) in 50 ml Dimethylformamid wurden unter Argon und unter Eiskühlung 24,2 g α-(Diäthoxyphosphinyl)-p-toluylsäureäthylester gegeben. Das Gemisch wurde bei Raumtemperatur bis zum Aufhören der Wasserstoffentwicklung gerührt. Danach wurde unter Eis/Methanolkühlung eine Lösung von 15 g 3,4-Dihydro-3,3-dimethyl-2H-1,5-benzodithiepin -7-yl-methylketon in 50 ml Dimethylformamid langsam zugetropft, wobei die Innentemperatur auf 35°C stieg. Nach einer weiteren halben Stunde wurde das Reaktionsgemisch auf Eis/Kochsalz gegeben und mit Aether extrahiert. Die ätherische Lösung wurde mit Wasser gewaschen, über Natriumsulfat getrocknet und unter vermindertem Druck eingedampft. Chromatographie an Kieselgel (Petroläther/Aethylacetat; 97/3) lieferte 16,2 g Rohprodukt, das nach Umkristallisation aus Hexan 12,6 g p[(E)-2-(3,4-Dihydro-3,3,-dimethyl-2H-1,5-benzodithiepin -7-yl)propenyl]benzosäureäthylester in Form blassgelber Kristalle lieferte. Smp. 99°C.
   Das Ausgangsmaterial wurde wie folgt hergestellt:
B. Zu 5,55 g Natriumhydrid (50% in Mineralöl) in 150 ml Dimethylsulfoxid wurde unter Kühlung 15 g Dimercaptobenzol langsam zugetropft, wobei die Innentemperatur zwischen 15 und 23°C gehalten wurde. Anschliessend wurde 1 Stunde bei Raumtemperatur gerührt und das Reaktionsgemisch mit Eis/Methanol gekühlt. Danach wurden 50 g 2,2-Dimethylpropandiol-ditosylat in fester Form in einer Portion zugegeben und das Reaktionsgemisch wurde 3 Stunden lang auf 80°C erwärmt. Danach wurde das Reaktionsgemisch abgekühlt, auf Eis gegossen, mit Aether extrahiert, der Extrakt mit Wasser gewaschen und getrocknet. Nach Eindampfen des Lösungsmittels wurde ein gelbes Oel erhalten, das an Kieselgel chromatographiert wurde (Petroläther/Aethylacetat; 99:1). Man erhielt 14 g 3,4-Dihydro-3,3-dimethyl-2H-1,5-benzodithiepin als farbloses Oel.
C. 13,3 g 3,4-Dihydro-3,3-dimethyl-2H-1,5-benzodithiepin wurden unter Argon in 110 ml Aethylenchlorid gelöst und bei -10°C nacheinander mit 10,1 ml Acetylchlorid und 19 g Aluminiumtrichlorid portionsweise versetzt. Danach wurde 2 1/2 Stunden bei Raumtemperatur gerührt, das Reaktionsgemisch auf Eis gegossen und mit Aether extrahiert. Der Extrakt wurde mit verdünnter Natronlauge und Wasser gewaschen, getrocknet und zur Trockne eingedampft. Man erhielt 15,6 g 3,4-Dihydro-3,3-dimethyl-2H-1,5-benzodithiepin -7-yl-methylketon in Form bräunlicher Kristalle.

### Beispiel 10

In Analogie zu Beispiel 9 wurde aus 3,4-Dihydro-3,3-dimethyl-2H-1,5-benzodioxepin -7-yl methylketon der p-[(E)-2-(3,4-Dihydro-3,3-dimethyl-2H-1,5-benzodioxepin -7-yl)propenyl]benzoesäureäthylester, Smp. 50-51°C erhalten. Das Ausgangsmaterial wurde in Analogie zu Beispiel 9, Abschnitt B und C ausgehend von Brenzcatechin über das 3,4-Dihydro-3,3-dimethyl-2H-1,5-benzodioxepin hergestellt.

### Beispiel 11

In Analogie zu Beispiel 9 wurde aus 3,4-Dihydro-3-methyl-2H-1,5-benzodithiepin-7-yl methylketon der p-[(E)-2-(3,4-Dihydro-3-methyl-2H-1,5-benzodithiepin -7-yl)propenyl]benzoesäureäthylester, Smp. 68-70°C, erhalten. Das Ausgangsmaterial wurde in Analogie zu Beispiel 9, Abschnitt B und C über das 3,4-Dihydro-3-methyl-2H-1,5-benzodithiepin hergestellt.

### Beispiel 12

In Analogie zu Beispiel 9 wurde aus 3,4-Dihydro-2H-1,5-benzodithiepin-7-yl methylketon der p-[(E)-2-(3,4-Dihydro-2H-1,5-benzodithiepin -7-yl-propenyl]benzoesäureäthylester, Smp. 98-99°C, erhalten. Das Ausgangsmaterial wurde in Analogie zu Beispiel 9, Abschnitt B und C, über das 3,4-Dihydro-2H-1,5-benzodithiepin hergestellt.

### Beispiel 13

A. 4,9 g 2,3,4,5-Tetrahydro-α-methyl-1-benzoxepin-8-methanol wurden in 50 ml Acetonitril gelöst und mit 13 g Triphenylphosphinhydrobromid versetzt. Das Reaktionsgemisch wurde 24 Stunden bei 40°C gerührt, danach wurde die Hauptmenge des Lösungsmittels unter vermindertem Druck entfernt und der Rückstand zwischen Hexan und Aethanol/Wasser (8:2) verteilt. Die schwere Phase wurde eingedampft und getrocknet wobei 11,96 g weisses Phosphoniumsalz erhalten wurden. 6,29 g Phosphoniumsalz wurden in 13 ml Butylenoxid gelöst, mit 2,6 g 4-Formylbenzoesäureäthylester versetzt und 18 Stunden zum Rückfluss erhitzt. Danach wurde die Hauptmenge des Lösungsmittels unter vermindertem Druck entfernt und der Rückstand zwischen Hexan und Aethanol/Wasser (8:2) verteilt. Die leichte Phase wurde über Magnesiumsulfat getrocknet und eingedampft. Chromatographie an Kieselgel (Petroläther/Aethylacetat (97:3) und Umkristallisation aus Hexan lieferte 1,4 g p-[(E)-2-(2,3,4,5-Tetrahydro-1-benzoxepin -8-yl)propenyl]benzoesäureäthylester, Smp. 71-72°C.
   Das Ausgangsmaterial wurde wie folgt hergestellt:
   m-Bromphenol wurde mit γ-Butyrolacton in Analogie zu Beispiel 15, Abschnitt B, C und D zu 8-Brom-2,3,4,5-tetrahydro-1-benzoxepin umgesetzt.
B. 7,3 g 8-Brom-2,3,4,5-tetrahydro-1-benzoxepin und 30 ml abs. Tetrahydrofuran wurden unter Argon zu 930 mg Mg-Spänen und einem Körnchen Jod gegeben. Die Reaktion wurde durch Zusatz von einigen Tropfen 1,2-Dibromäthan in Gang gebracht und war nach 2 Stunden beendet. Danach wurde das Reaktionsgemisch auf -10°C gekühlt und überschüssiger Acetaldehyd wurde in das Reaktionsgefäss hineindestilliert. Nach 10 Minuten wurde mit gesättigter Na₄Cl-Lösung hydrolysiert, mit Aether extrahiert, mit Wasser gewaschen, getrocknet und zur Trockene eingedampft. Chromatographie an Kieselgel (Petroläther/Aethylacetat; 3:1) lieferte 4,93 g 2,3,4,5-Tetrahydro-α-methyl-1-benzoxepin -8-methanol als farbloses Oel.

### Beispiel 14

In Analogie zu Beispiel 13 wurde aus 2,3,4,5-Tetrahydro-α-methyl-3,3-dimethyl-2H-1-benzothiepin-8-methanol der p-[(E)-2-(2,3,4,5-Tetrahydro-3,3-dimethyl-2H -1-benzothiepin-8-yl)propenyl]benzoesäuremethylester, Smp. 82-84°C, erhalten. Das Ausgangsmaterial wurde in Analogie zu Beispiel 13, Abschnitt B, aus 8-Brom-2,3,4,5-tetrahydro-1-benzothiepin hergestellt.

### Beispiel 15

Zu 1,3 g Natriumhydrid (50% in Mineralöl) in 28 ml Dimethylformamid wurden langsam 8,85 g α-(Diäthoxyphosphinyl)-p-toluylsäureäthylester zugetropft. Das Reaktionsgemisch wurde bei Raumtemperatur bis zum Aufhören der Wasserstoffentwicklung gerührt. Danach wurden 4,2 g 2,3,4,5-Tetrahydro-3,3-dimethyl-1-benzothiepin -7-yl-methylketon zugesetzt und das Reaktionsgemisch wurde 2 Stunden bei Raumtemperatur gerührt. Danach wurde auf Eis/Kochsalz gegossen, mit Aether extrahiert, die ätherische Lösung mit Wasser gewaschen und getrocknet und eingedampft. Chromatographie an Silicagel (Petroläther/Aethylacetat (95:5)) und Umkristallisation aus Hexan lieferte 4 g p-[(E)-2-(2,3,4,5-Tetrahydro-3,3-dimethyl-1-benzothiepin -7-yl)propenyl]benzoesäureäthylester vom Schmelzpunkt 96-97°C. (Referenzbeispiel; nicht gegenstand der Erfindung).
A. In Analogie wurde aus 2,3,4,5-Tetrahydro-1-benzothiepin-8-yl methylketon der p-[(E)-2-(2,3,4,5-Tetrahydro-1-benzothiepin -8-yl)propenyl]benzoesäureäthylester, Smp. 63-64°C, erhalten.
   Das Ausgangsmaterial wurde wie folgt hergestellt:
B. 3,74 g Natrium wurden unter Argon in 77 ml absolutem Aethanol gelöst. Die Lösung wurde tropfenweise mit 25 g m-Bromthiophenol versetzt und zum Rückfluss erhitzt. Danach wurden 12,4 ml γ-Butyrolacton zugesetzt, und das Reaktionsgemisch wurde 5 Stunden auf 110°C erwärmt. Das ausgeschiedene Carbonsäure-Natriumsalz wurde abfiltriert, mit wenig Aether gewaschen und in 300 ml Wasser gelöst. Die Lösung wurde unter Eiskühlung mit 1N HCl auf pH 2 angesäuert, die freie Carbonsäure mit Aether extrahiert, die ätherische Lösung getrocknet und eingedampft. Man erhielt 33,1 g 4-(m-Bromphenylmercapto)buttersäure.
C. 33,1 g der so erhaltenen Säure wurden mit 275 g Polyphosphorsäure und 550 ml O-Xylol 24 Stunden unter intensivem Rühren auf 120°C erwärmt. Nach Abkühlung wurde mit Eis hydrolysiert, mit Wasser verdünnt und mit Aether extrahiert. Die organische Phase wurde mit Wasser gewaschen, getrocknet und eingedampft. Chromatographie an Kieselgel (Petroläther/Aethylacetat (9:1)) lieferte 22,1 g 8-Brom-3,4-dihydro-1-benzothiepin -5-(2H)on als bräunliches Oel.
D. 15,6 g 8-Brom-3,4-dihydro-1-benzothiepin-5-(2H)on wurden in 70 ml Diäthylenglykol gelöst. Die Lösung wurde mit 6,55 ml Hydrazinhydrat und 7,5 g festem KOH versetzt und ca. 30 Stunden auf 180-190°C unter Rückfluss erhitzt. Nach Abkühlen wurde auf Eis gegossen und mit Aether extrahiert. Die organische Phase wurde mit Wasser gewaschen, getrocknet und eingedampft. Chromatographie an Kieselgel mit Petroläther lieferte 10,7 g 8-Brom-2,3,4,5-tetrahydro-1-benzothiepin als farbloses Oel.
E. Das vorstehend erhaltene Bromid wurde in Analogie zu Beispiel 13, letzter Abschnitt, durch Grignard-Reaktion in 2,3,4,5-Tetrahydro-α-methyl-1-benzothiepin -8-methanol übergeführt.
F. 7,80 g 2,3,4,5-Tetrahydro-α-methyl-1-benzothiepin-8-methanol wurden in 100 ml Methylenchlorid gelöst, mit 50 g MnO₂ versetzt und das Reaktionsgemisch über Nacht gerührt. Danach wurde über ein Filterhilfsmittel abfiltriert und die Lösung eingedampft. Man erhielt 7,30 g 2,3,4,5-Tetrahydro-1-benzothiepin-8-yl methylketon als farbloses Oel.

### Beispiel 16

A. 4,0 g p-[(E)-2-(2,3,4,5-Tetrahydro-1-benzothiepin -7-yl)propenyl]benzoesäureäthylester wurden in 30 ml Methylenchlorid mit 6,05 g m-Chlorperbenzoesäure (85%) versetzt und 24 Stunden bei ca. +5°C gehalten. Danach wurde mit Methylenchlorid verdünnt, mit Bisulfitlösung und Sodalösung gewaschen, getrocknet und eingedampft. Umkristallisation aus Aethylacetat/Hexan lieferte 3,7 g p-[(E)-2-(2',3',4',5'-Tetrahydro-1-benzothiepin -7'-yl)propenyl]benzoesäureäthylester-1',1'-dioxid als blassgelbe Kristalle vom Schmelzpunkt 147-148°C. (Referenzbeispiel; nicht gegenstand der Erfindung).
B. In Analogie wurden hergestellt:
   p-[(E)-2-(2',3',4',5'-Tetrahydro -5',5'-dimethyl-1-benzothiepin-7'-yl)propenyl] benzoesäureäthylester-1',1'-dioxid, Smp. 142-143°C, und
   p-[(E)-2-(3',4'-Dihydro-3',3'-dimethyl-2'-H-1,5-benzodithiepin-7'-yl)propenyl]benzoesäureäthylelester -1',1',5',5'-tetroxid, Smp. 175-176°C.

### Beispiel 17

A. Eine Lösung von 4,47 g 2,3,4,5-Tetrahydro-α-methyl-1-methyl -1H-1-benzazepin -7-methanol in 50 ml Acetonitril wurde mit 9,73 g Triphenylphosphinhydrobromid versetzt. Das Reaktionsgemisch wurde 20 Stunden bei 30°C gerührt, eingeengt und der Rückstand zwischen Hexan und wässrigem Aethanol (8:2) verteilt. Die untere Phase wurde eingedampft, das Produkt in Methylenchlorid aufgenommen, die Lösung getrocknet und eingedampft, wobei 12,3 g Phosphoniumsalz in Form rötlicher Kristalle erhalten wurden. 10,7 g Phosphoniumsalz wurden unter Argon in 40 ml absolutem Tetrahydrofuran suspendiert und bei 0°C mit 20,7 ml 1,4M n-Butyllithiumlösung deprotoniert. Nach 15 Minuten wurde die dunkelrote Lösung mit 4,76 g 4-Formylbenzoesäuremethylester versetzt und 1 1/2 Stunden bei Raumtemperatur gerührt. Danach wurde auf Eis gegossen, mit Aether extrahiert, mit Wasser gewaschen und getrocknet und eingedampft. Chromatographie an Kieselgel (Petroläther/Aethylacetat (95:5) und Umkristallisation aus Hexan/Aethylacetat lieferte 3.06 g p-[(E)-2-(2,3,4,5-Tetrahydro-1-methyl-1-benazepin -7-yl)propenyl]benzoesäuremethylester in Form blassgelber Kristalle vom Schmelzpunkt 106°C. (Referenzbeispiel; nicht Gegenstand der Erfindung)
B. In Analogie wurde aus 3,4-Dihydro-α-3,3-trimethyl-2H-1,5-benzodithiepin -7-yl-methanol der p-[(E)-2-(3,4-Dihydro-3,3-dimethyl-2H-1,5-benzodithiepin -7-yl)propenyl]benzoesäuremethylester, Smp. 142-143° hergestellt. Das Ausgangsmaterial wurde aus dem in Beispiel 9C. hergestellten 3,4-Dihydro-3,3-dimethyl-2H-1,5-benzodithiepin -7-yl-methylketon durch Natriumborhydrid-Reduktion synthetisiert.

### Beispiel 18

A. Eine Lösung von 1,2 g p-[(E)-2-(2,3,4,5-Tetrahydro -5-methyl-1-benzothiepin -7-yl)propenyl]benzoesäureäthylester in 30 ml Aethanol wurde mit 5 ml Wasser, die 0,8 g NaOH enthielten, versetzt. Das Reaktionsgemisch wurde über Nacht bei 35°C gerührt, auf Eis gegossen, mit HCl angesäuert und mit Aether extrahiert. Die organische Phase wurde mit Wasser gewaschen, getrocknet und eingeengt. Nach Umkristallisation aus Aethylacetat wurden 885 mg p-[(E)-2-(2,3,4,5-Tetrahydro-5-methyl -1-benzothiepin-7-yl)propenyl]bensoesäure in Form weisser Kristalle vom Schmelzpunkt 186°C erhalten. (Referenzbeispiel; nicht gegenstand der Erfindung).
B. In analoger Weise wurden hergestellt:
   p-[(E)-2-(3,4-dihydro-3-methyl-2H-1,5-benzodithiepin -7-yl)propenyl]benzoesäure, Smp. 188-189°C,
   p-[(E)-2-(3,4-Dihydro-3,3-dimethyl-2H-1,5-benzodithiepin -7'-yl)propenyl]benzoesäure, Smp. 212-213°C,
   p-[(E)-2-(2',3',4',5'-Tetrahydro-1-benzothiepin -8'-yl)propenyl]bensoesäure-1',1'-dioxid, Smp. 219-220°C,
   p-[(E)-2-(3,4-Dihydro-3,3-dimethyl-2H-1,5-benzodioxepin-7-yl)propenyl]benzoesäure, Smp. 198-199°C, und
   p-[(E)-2-(2,3,4,5-Tetrahydro-1-benzothiepin-8-yl)propenyl]benzoesäure, Smp. 217-218°C.

### Beispiel 19

In Analogie zu Beispiel 13 (Abschnitt A und B), aber mit 4-Formylbenzoesäuremethyl- statt äthylester als Carbonylkomponente, wurde p-[(E)-2-(2,3,4,5-Tetrahydro-5-methyl-1-benzothiepin-8-yl)propenyl]bensoesäuremethylester, Smp. 88-89°, hergestellt.

Das Ausgangsmaterial wurde wie folgt hergestellt:
Zunächst wurde, wie in Beispiel 15 beschrieben, m-Bromthiophenol in 8-Brom-3,4-dihydro-1-benzothiepin-5-(2H)-on überführt. Dieses Keton wurde mit MeMgI in Aether methyliert und der resultierende tertiäre Alkohol wie folgt deoxygeniert: 22,85 g wurden unter Argon in 250 ml Hexan vorgelegt und nacheinander mit 75,1 g NaI, 26,4 ml Acetonitril und 63,5 ml Me₃SiCl versetzt. Man rührte über Nacht bei Raumtemperatur, goss auf Eis, extrahiert mit Aether, wusch mit Bisulfit-Lösung und Wasser, trocknete und dampfte ein. Chromatographie an Kieselgel mit Petroläther ergab 18,1 g 8-Brom-2,3,4,5-tetrahydro-5-methyl-1-benzothiepin als blassgelbes Oel.

### Beispiel 20

In Analogie zu Beispiel 15 wurde aus 2,3-Dihydro-3,3-dimethyl-1,4-benzoxathiin-7-yl-methylketon der p-[(E)-2-(2,3-Dihydro-3,3-dimethyl-1,4-benzoxathiin -7-yl)propenyl]benzoesäureäthylester, Smp. 69-70° hergestellt.

Das Ausgangsmaterial wurde wie folgt hergestellt:
4,11 ml 2-Mercaptophenol und 27,4 g fein gepulvertes K₂CO₃ wurden in 40 ml DMF vorgelegt und bei 0° unter Argonatmosphäre mit 4,08 ml β-Methallylchlorid versetzt. Man liess 1/2 Stunde bei Raumtemperatur nachreagieren, goss auf Eis und extrahierte mit Aether. Nach Waschen mit Wasser, Trocknen und Eindampfen erhielt man 7,80 g blassgelbes Oel, das in 60 ml Chloroform gelöst und nach Zusatz von 1 g p-Toluolsulfonsäure über Nacht zum Rückfluss erhitzt wurde. Extraktive Aufarbeitung (Aether) ergab 6,96 g DC-einheitliches 2,3-Dihydro-3,3-dimethyl-1,4-benzoxathiin als farbloses Oel. Dieses wurde in Analogie zu Beispiel 18(B) regioselektiv zum 2,3-Dihydro-3,3-dimethyl-1,4-benzoxathiin-7-yl-methylketon acetyliert.

### Beispiel 21

In Analogie zu Beispiel 17 wurde aus 2,3,4,5-Tetrahydro-1,3,3,5-tetramethyl-1H-1,5-benzodiazepin der p-[(E)-2-(2,3,4,5-Tetrahydro-1,3,3,5-tetramethyl-1H-1,5-benzodiazepin-7-yl)propenyl]benzoesäuremethylester, Smp. 120-121° hergestellt.

Das Ausgangsmaterial wurde wie folgt hergestellt:
Unter Argon wurden 120 mMol NaNH₂ (50% Suspension in Toluol) in 40 ml abs. THF vorgelegt. Man gab 40 mMol t-BuOH in 10 ml THF zu und rührte 2 Stunden bei 50°. Dann wurden 40 mMol (5,2 g) 1,3-Diamino-N,N,2,2-tetramethylpropan, gelöst in 20 ml THF, zugetropft und eine weitere Stunde bei 50° gerührt. Man kühlt auf 30°. verdünnte mit 200 ml THFund gab 40 mMol (4,6 ml) 1,2-Dichlorbenzol zu. Nach 18 Stunden wurde extraktiv aufgearbeitet (Aether). Schnelle Chromatographie des Rohprodukts an Kieselgel mit Hexan lieferte 4,4 g rotbraune Kristalle.

### Beispiel 22

4,2 g 3,4-Dihydro-3,3-dimethyl-2H-1,5-benzodioxepin-7-carbonsäure wurden mit 3 ml SOCl₂ versetzt und 1 Stunde unter Rückfluss erhitzt. Das überschüssige Reagens wurde im Vakuum abgedampft und das resultierende Säurechlorid am Hochvakuum getrocknet. Dann löste man es in 70 ml Pyridin und tropfte bei 0° unter Ar-Atmosphäre eine Lösung von 4,05 g p-Aminobenzoesäureäthylester in 70 ml Pyridin langsam zu. Man liess bei Raumtemperatur 2 Stunden nachreagieren, engte auf 1/4 des Volumens ein und verteilte zwischen Aether und verdünnter HCl. Man wusch die organische Phase gründlich mit H₂O, trocknete und engte ein. Dabei kristallisierte das Produkt aus. Nach Kühlen, Abnutschen und Trocknen erhielt man 5,9 g p-(3,4-Dihydro-3,3-dimethyl-2H-1,5-benzodioxepin -7-carboxamido)benzoesäureäthylester als farblose Kristalle vom Smp. 181-182°.

Das Ausgangsmaterial wurde wie folgt hergestellt:
A. 8,55 g 3,4-Dihydro-3,3-dimethyl-2H-1,5-benzodioxepin (vgl. Beispiel 10) wurden in 70 ml CH₂Cl₂ gelöst und bei 0° unter Ar-Atmosphäre mit einer Lösung von 8,0 g Br₂ in 30 ml CH₂Cl₂ versetzt. Man erwärmte auf Raumtemperatur und liess 1 Stunde weiterreagieren. Dann wurde auf Eis gegossen und mit Aether extrahiert. Waschen mit NaHCO₃-Lösung, Wasser, Trocknen und Eindampfen ergab 13,35 g 7-Brom-3,4-dihydro-3,3-dimethyl-2H-1,5-benzodioxepin als farbloses Oel, das 94,5% rein war und 4,7% Ausgangsmaterial enthielt; es wurde roh weiterverwendet.
B. Aus 6,0 g des so erhaltenen Bromids und 675 mg Mg-Spänen wurde unter Ar-Atmosphäre in 25 ml THF die Grignard-Verbindung hergestellt. Nach 3 Stunden war die Metallierung beendet. Man kühlte auf -10° und leitete während 30 Minuten einen kräftigen CO₂-Strom ein. Man gab vorsichtig Eis zu, verteilte zwischen verd. NaOH und Aether, stellte die wässrige Phase mit HCl konz. auf pH = 1 und extrahierte wieder mit Aether. Waschen mit Wasser, Trocknen und Eindampfen lieferte 4,2 g 3,4-Dihydro-3,3-dimethyl-2H-1,5-benzodioxepin-7-carbonsäure als farblose Kristalle vom Smp. 172-173°.

### Beispiel 23

In Analogie zu Beispiel 22 wurde aus 3,4-Dihydro-3,3-dimethyl-2H-1,5-benzodithiepin -7-carbonsäurechlorid der p-(3,4-Dihydro-3,3-dimethyl-2H-1,5-benzodithiepin -7-carboxamido)benzoesäureäthylester, Smp. 180-181°, hergestellt.

Das Ausgangsmaterial wurde in Analogie zu Beispiel 30, Abschnitt A und B hergestellt, wobei bei der Bromierung Fe-Pulver als Katalysator verwendet wurde und das Säurechlorid aus der Säure mit Oxalylchlorid hergestellt wurde.

### Beispiel 24

In Analogie zu Beispiel 22 wurde aus 2,3,4,5-Tetrahydro-5-methyl-1-benzothiepin-8-carbonsäurechlorid der p-(2,3,4,5-Tetrahydro-5-methyl-1-benzothiepin -8-carboxamido)benzoesäureäthylester, Smp. 132-133°, hergestellt.

### Beispiel 25

In Analogie zu Beispiel 22 wurde aus 2,3,4,5-Tetrahydro-1-benzothiepin-8-carbonsäurechlorid der p-(2,3,4,5-Tetrahydro-1-benzothiepin -8-carboxamido)benzoesäureäthylester, Smp. 100°, hergestellt.

### Beispiel 26

10,0 g 3,4-Dihydro-3,3-dimethyl-2H-1,5-benzodithiepin-7-yl-methylketon (siehe Beispiel 9) wurden in 100 ml EtOH gelöst und portionenweise mit 2,0 g NaBH₄ versetzt. Man liess über Nacht bei Raumtemperatur nachreagieren, verdünnte mit Wasser und extrahierte mit Aether. Waschen mit Wasser, Trocknen und Eindampfen lieferte 10,0 g sekundären Alkohol, der roh weiterverarbeitet wurde.

Dieser wurde in 150 ml Acetonitril gelöst und mit 19,5 g Triphenylphosphinhydrobromid versetzt. Man rührte über Nacht bei 40° und dampfte dann die Hauptmenge des Lösungsmittels im Vakuum ab. Der Rückstand wurde zwischen Hexan und EtOH/H₂O = 8/2 verteilt, die schwere Phase eingedampft und wieder in CH₂Cl₂ gelöst. Trocknen und Eindampfen ergab einen Schaum, der während mehreren Stunden in Hexan/Aether = 2/1 digeriert wurde; dabei resultierten 20,6 g farblose Kristalle.

4,64 g davon wurden in 25 ml THF vorgelegt und bei 0° mit 5,8 ml 1,6N nBuLi (Hexan) in das Ylid überführt. Man rührte 1/4 Stunde bei 0° und gab dann 1,90 g 4-(2-Morpholinoäthoxy)benzaldehyd unverdünnt zu. Man liess auf Raumtemperatur erwärmen und arbeitete nach 1 Stunde wie folgt auf: Man verteilte zwischen EtOH/H₂O = 8/2 und Hexan/AcOEt = 95/5 und dampfte die leichtere Phase ein. Säulenchromatographie an Kieselgel (Aethylacetat) des so erhaltenen Rohprodukts und Kristallisation aus Aether lieferten schliesslich 1,20 g 4-[2-[p-[(E)-2-(3,4-Dihydro-3,3-dimethyl-2H -1,5-benzodithiepin-7-yl)propenyl]phenoxy]äthyl]morpholin in Form weisser Kristalle vom Smp. 117-118°.

### Beispiel 27

In Analogie zu Beispiel 26 wurde aus 3,4-Dihydro-3,3-dimethyl-2H-1,5-benzodioxepin-7-yl-methylketon das 4-[2-[p-[(E)-2-(3,4-Dihydro-3,3-dimethyl-2H -1,5-benzodioxepin-7-yl)propenyl]phenoxy]äthyl]morpholin, Smp. 104-105° hergestellt.

### Beispiel 28

In Analogie zu Beispiel 26 wurde mit p-Aethoxycarbonyloxybenzaldehyd als Carbonylkomponente das p-[(E)-2-(3,4-Dihydro-3,3-dimethyl-2H-1,5-benzodioxepin -7-yl)propenyl]phenyläthylcarbonat hergestellt.

590 mg davon wurden in 15 ml Aethanol gelöst und mit 7 ml Wasser, enthaltend 700 mg NaOH-Plätzchen, versetzt. Man rührte über Nacht bei 50°, goss auf Eis, extrahierte mit Aether, wusch mit Wasser, trocknete und dampfte ein. Säulenchromatographie an Kieselgel (Petroläther/Aethylacetat = 85/15) und anschliessende Kristallisation aus Hexan/Aether lieferten 270 mg p-[(E)-2-(3,4-Dihydro-3,3-dimethyl-2H-1,5-benzodioxepin -7-yl)propenyl]phenol als weisse Kristalle vom Smp. 70-71°.

### Beispiel 29

8,30 g 3,4-Dihydro-2H-1,5-benzothiazepinhydrobromid wurden in 150 ml THF vorgelegt und mit 48 ml 1,55M nBuLi (Hexan) unter Ar-Atmosphäre bei -10° deprotoniert. Nach 1/4 Stunde tropfte man zur gelben Lösung des Li-Amids 4,64 ml MeI zu und rührte 1 Stunde nach. Dann goss man auf Eis, extrahierte mit Aether, wusch mit Wasser, trocknete und dampfte ein. Säulenchromatographie an Kieselgel (Petroläther/Aethylacetat (96/4)) lieferte 5,70 g 3,4-Dihydro-5-methyl-2H-1,5-benzothiazepin als farbloses Oel, das wie folgt formyliert wurde:

Man legte unter Ar-Atmosphäre 200 ml DMF vor und tropfte unter Kühlung 11,3 ml POCl₃ zu. Man rührte 1/4 Stunde bei Raumtemperatur nach, gab 5,70 g des aromatischen Substrats zu und erwärmte 1,5 Stunden auf 60-70°. Nach Abkühlen wurde auf Eis gegossen, mit Aether extrahiert, mit Wasser gewaschen, getrocknet und eingedampft. Kristallisation aus Hexan/Aethylacetat lieferte 5,20 g Aldehyd als gelbe Kristalle vom Smp. 56-57°, der folgendermassen umgesetzt wurde:
Man tropfte 80 ml Aether, in dem 5,20 g dieses Aldehyds gelöst waren, bei Raumtemperatur unter Ar-Atmosphäre langsam zu einer ätherischen MeMgI-Lösung, die nach Standardverfahren aus 968 mg Mg-Spänen und 2,5 ml MeI hergestellt worden war. Man rührte noch 2 Stunden bei Raumtemperatur, hydrolysierte dann mit NH₄Cl-Lösung und extrahierte mit Aether. Waschen mit Wasser, Trocknen und Entfernen des Lösungsmittels im Vakuum lieferte 5,50 g sekundären Alkohol als Oel, das roh weiterverwendet wurde.

Dieses wurde in 40 ml Acetonitril vorgelegt und mit 10,1 g Triphenylphosphinhydrobromid versetzt. Man rührte über Nacht bei 40° und engte dann die klare Lösung im Vakuum ein. Der Rückstand wurde zwischen Hexan und EtOH/H₂O = 8/2 verteilt und die schwere Phase eingedampft. Das so erhaltene Rohprodukt wurde nochmals in CH₂Cl₂ gelöst, über Na₂SO₄ getrocknet und eingedampft. Mehrstündiges Verrühren in Hexan/Aether (2/1) lieferte schliesslich 14,4 g kristallines Phosphoniumsalz.

Diese 14,4 g Phosphoniumsalz wurden in 60 ml THF unter Ar-Atmosphäre vorgelegt und bei 0° mit 22,1 ml 1,5M nBuLi (Hexan) ins tiefrote Ylid überführt. Nach 5 Minuten gab man 4,65 g 4-Formyl-benzoesäuremethylester als Feststoff zu und entfernte anschliessend das Kühlbad. Nach 1,5 Stunden wurde auf Eis gegossen, mit Aether extrahiert, mit Wasser gewaschen, getrocknet und eingedampft. Sorgfältige Chromatographie an Kieselgel (Petroläther/Aethylacetat; 92/8) und Umkristallisation aus Hexan/Aethylacetat lieferte schliesslich 2,65 g p-[(E)-2-(3,4-Dihydro-5-methyl-2H-1,5-benzothiazepin -8-yl)propenyl]benzoesäuremethylester als gelbe Kristalle vom Smp. 97-98°.

### Beispiel 30

In Analogie zu Beispiel 29 wurde der p-[(E)-2-(3,4-Dihydro-3,5-dimethyl-2H-1,5-benzothiazepin -8-yl)propenyl]benzoesäuremethylester, Smp. 101-102° hergestellt.

Das Ausgangsmaterial wurde wie folgt synthetisiert:
18,8 g 2-Aminothiophenol wurden in 75 ml Aceton vorgelegt und bei 0° mit 41,6 g gepulvertem K₂CO₃ und 18,2 ml 1-Brom-3-chlor-2-methylpropan versetzt, wobei sofort eine stark exotherme Reaktion einsetzte. Nach 2 Stunden wurde auf Eis gegossen, mit Aether extrahiert, mit Wasser gewaschen, getrocknet und eingedampft. Es resultierten 32,0 g S-alkyliertes Produkt, das in 80 ml Aceton gelöst wurde. Die Lösung wurde mit 111 g NaJ versetzt und während 3 Tagen erhitzt (Oelbad 90°). Man kühlte, goss auf Eis, stellte mit Natronlauge basisch, extrahierte mit Aether, wusch mit Wasser, trocknete und dampfte ein. Säulenchromatographie an Kieselgel (Petroläther/Aethylacetat 95/5) lieferte 10,0 g 3,4-Dihydro-3-methyl-2H-1,5-benzothiazepin als farbloses Oel, allerdings mit 14% einer unbekannten Verbindung verunreinigt.

Die weitere Umsetzung erfolgte wie in Beispiel 29 durch Formylierung, Reaktion mit Methylmagnesiumjodid und Herstellung des Phosphoniumsalzes.

### Beispiel 31

In Analogie zu Beispiel 26 wurde mit 4-(2-Dimethylaminoäthoxy)benzaldehyd als Carbonylkomponente das N,N-Dimethyl-2-[p-[(E)-2-(3,4-dihydro-3,3-dimethyl -2H-1,5-benzodithiepin-7-yl)propenyl]phenoxy]äthylamin, Smp. 46-47 erhalten°.

### Beispiel 32

In analoger Weise zu Beispiel 18 wurden hergestellt:
p-[(E)-2-(2,3-Dihydro-3,3-dimethyl-1,4-benzoxathiin-7-yl)propenyl]benzoesäure, Smp. 217-218°;
p-[(E)-2(3,4-Dihydro-2H-1,5-benzodithiepin-7-yl)propenyl]benzoesäure, Smp. 202-203°;
p-[(E)-2-(3,4-Dihydro-3,3-dimethyl-2H-1,5-benzodioxepin-7-yl)propenyl]benzoesäure, Smp. 198-199°;
p-[(E)-2-(3,4-Dihydro-5-methyl-2H-1,5-benzothiazepin-8-yl)propenyl]benzoesäure, Smp. 197-198°;
und
p-[(E)-2-(3,4-Dihydro-3,5-dimethyl-2H-1,5-benzothiazepin-8-yl)propenyl]benzoesäure, Smp. 213-214°.

### Beispiel 33

1,20 g p-(2,3,4,5-Tetrahydro-1-benzothiepin -8-carboxamido)benzoesäureäthylester wurden in 40 ml Aethanol gelöst und mit 14 ml Wasser, enthaltend 1,40 g NaOH, versetzt. Man rührte über Nacht bei Raumtemperatur, goss auf Eis und säuerte mit konz. HCl an. Dann wurde zweimal mit Aethylacetat extrahiert, mit Wasser gewaschen, getrocknet und eingedampft. Umkristallisation aus Aethylacetat ergab 925 mg p-(2,3,4,5-Tetrahydro-1-benzothiepin-8-carboxamido)benzoesäure als farblose Kristalle vom Smp. 261-262°.

### Beispiel 34

Zu 16,8 ml Acetylchlorid in 360 ml Methylenchlorid wurden bei 0°C 28,9 g Aluminiumchlorid portionenweise zugegeben. Nach weiteren 30 Minuten bei 0°C wurde eine Lösung von 35,9 g 2,2-Dimethyl-1,3-benzodithiol in 180 ml Methylenchlorid langsam zugetropft und weitere 12 Stunden bei 0°C, danach 5 Stunden bei 20°C gerührt. Danach wurde auf Eis gegossen, mit Methylenchlorid extrahiert und die Extrakte mit verdünnter Natronlauge und Wasser neutral gewaschen, über Natriumsulfat getrocknet und eingedampft. Nach Umkristallisation aus Methanol/Wasser erhielt man 41,8 g (2,2-Dimethyl-1,3-benzodithiol-5-yl)methylketon, Schmelzpunkt 75-76°C. 41,8 g dieses Ketons wurden in 1 l Aethanol gelöst, auf 0°C gekühlt und 7 g Natriumborhydrid portionenweise zugegeben. Danach wurde 3 Stunden bei 20°C gerührt, im Vakuum weitgehend eingedampft, 200 ml Wasser zugegeben, mit 1N Schwefelsäure unter Kühlung langsam auf pH 4 gestellt und dreimal mit Aether extrahiert. Die Extrakte wurden mit Wasser gewaschen, getrocknet und eingedampft. Man erhielt 42,6 g α,2,2,-Trimethyl-1,3-benzodithiol-5-methanol. 39,5 g dieses Alkohols wurden mit 65,9 g Triphenylphosphinhydrobromid in 500 ml Acetonitril wie in Beispiel 8 beschrieben umgesetzt und aufgearbeitet. Man erhielt 96,3 g [1-(2,2-Dimethyl-1,3-benzodithiol -5-yl)äthyl]triphenylphosphoniumbromid. 17,0 g dieses Phosphoniumsalzes wurden in Analogie zu Beispiel 8 mit Methyl-4-formylbenzoat umgesetzt. Nach Aufarbeitung und Umkristallisation aus Essigester und Hexan erhielt man 4,9 g Methyl p-[(E)-2-(2,2-dimethyl-1,3-benzodithiol -5-yl)propenyl]benzoat in farblosen Kristallen, Schmelzpunkt 86-87°C.

### Beispiel 35

6,6 g [1-(2,2-Dimethyl-1,3-benzodithiol -5-yl)äthyl]triphenylphosphoniumbromid wurden in 40 ml Tetrahydrofuran gelöst und bei 0°C langsam mit 9 ml einer 1,5 molaren Lösung von n-Butyllithium in Hexan versetzt. Nach 40 Minuten wurden zur dunkelroten Lösung 3,5 g 4-(2-Morpholinoäthoxy)benzaldehyd in 10 ml Tetrahydrofuran zugetropft und 16 Stunden bei 20°C gerührt. Dann wurde auf Eis gegossen und mit Essigester extrahiert. Die vereinigten Extrakte wurden mit Wasser gewaschen, getrocknet und eingedampft. Nach Chromatographie (Kieselgel, Eluierungsmittel: Essigester) und Kristallisation aus Aether/Hexan erhielt man 1,8 g 4-[2-[p-[(E)-2-(2,2-Dimethyl -1,3-benzodithiol-5-yl)propenyl]phenoxy]äthyl]morpholin in farblosen Kristallen, Schmelzpunkt 90-91°C.

### Beispiel 36

In Analogie zu Beispiel 35 erhielt man durch Umsetzung von [1-(2,2-Dimethyl-1,3-benzodithiol -5-yl)äthyl]triphenylphosphoniumbromid mit Benzaldehyd 2,2-Dimethyl-5-[(E)-α-methylstyryl]-1,3-benzodithiol, Schmelzpunkt 57-58°C.

### Beispiel 37

In Analogie zu Beispiel 35 erhielt man durch Umsetzung von [1-(2,2-Dimethyl-1,3-benzodithiol -5-yl)äthyl]triphenylphosphoniumbromid mit Aethyl 4-formylphenylcarbonat Aethyl p-[(E)-2-(2,2-dimethyl-1,3-benzodithiol -5-yl)propenyl]phenylcarbonat, Schmelzpunkt 93-94°C. Hydrolyse dieser Verbindung mit wässriger Kalilauge in Aethanol ergab p-[(E)-2-(2,2-Dimethyl-1,3-benzodithiol -5-yl)propenyl]phenol, Schmelzpunkt 123-124°C.

### Beispiel 38

In Analogie zu Beispiel 18 erhielt man durch Hydrolyse von Methyl p-[(E)-2-(2,2-dimethyl-1,3-benzodithiol -5-yl)propenyl]benzoat p-[(E)-2-(2,2-Dimethyl-1,3-benzodithiol-5-yl)propenyl] benzoesäure, Schmelzpunkt 204-106°C.

### Beispiel 39

33 g [1-(3,4-Dihydro-4,4-dimethyl-2H-1-benzothiopyran -7-yl)äthyl]triphenylphosphoniumbromid und 6,5 g Benzaldehyd wurden in 300 ml Butylenoxid während 16 Stunden rückflussiert. Nach Aufarbeitung und Umkristallisation aus Hexan erhielt man 7,9 g 3,4-Dihydro-4,4-dimethyl-7-(α-methylstyryl)-2H -1-benzothiopyran, Schmelzpunkt 67-69°C.

### Beispiel 40

Oxidation der nach Beispiel 39 erhaltenen Verbindung mit m-Chloroperbenzoesäure in Analogie zu Beispiel 4 ergab 3,4-Dihydro-4,4-dimethyl -7-(α-methylstyryl)-2H-1-benzothiopyran 1,1-dioxid, Schmelzpunkt 148-150°C (aus Essigester).

### Beispiel 41

1,7 g Magnesiumspäne wurden mit 10 ml Aether überschichtet. Unter leichtem Rückfluss wurde eine Lösung von 7,7 g Benzylchlorid in 80 ml Aether hinzugetropft und anschliessend eine weitere Stunde bis zur vollständigen Lösung des Magnesiums am Sieden gehalten. Nach dem Abkühlen auf Raumtemperatur wurde eine Lösung von 7 g 3,4-Dihydro-4,4-dimethyl-7-acetyl -2H-1-benzopyran in 50 ml Aether zugetropft und das Reaktionsgemisch weitere 2,5 Stunden zum Rückfluss erhitzt. Nach dem Abkühlen goss man auf Eis/2N Salzsäure, extrahierte mit Aether, wusch die organische Phase mit Wasser und verdünnte Natriumbicarbonatlösung, trocknete und dampfte ein. Das so erhaltene schwachgelbe Oel wurde in 10 ml Essigsäure gelöst und nach Zugabe von 0,5 g p-Toluolsulfonsäure während 2 Stunden zum Rückfluss erhitzt. Die erhaltene Reaktionslösung wurde mit Wasser verdünnt und mehrfach mit Aether extrahiert. Die vereinigten Extrakte wurden mit Wasser verdünnt, mit Natriumbicarbonatlösung und Wasser gewaschen, getrocknet und eingedampft. Das Rohprodukt wurde chromatographiert (Kieselgel, Eluierungsmittel, Hexan/0,5% Essigester) und aus Hexan umkristallisiert. Man erhielt 4,8 g 3,4-Dihydro-4,4-dimethyl-7-(α-methylstyryl) -2H-1-benzopyran, Schmelzpunkt 44-46°C.

### Beispiel 42

In Analogie zu Beispiel 41 wurde durch Grignard-Reaktion von Benzylmagnesiumchlorid mit 3,4-Dihydro-1,4,4-trimethyl -7-acetyl-chinolin 3,4-Dihydro-1,4,4-trimethyl -7-(α-methylstyryl)chinolin hergestellt, Schmelzpunkt 66-68°C (aus Hexan).

### Beispiel 43

Eine Lösung von 14,2 g 7-Brom-3,4-dihydro-4,4-dimethyl -2H-1-benzopyran und 1,1 g 1,2-Dibromäthan in 80 ml Tetrahydrofuran wurde unter Verwendung eines heizbaren Ultraschallbades zu einer rückfluss-siedenden Suspension von 1,6 g Magnesiumspänen in 20 ml Tetrahydrofuran hinzugetropft. Nach weiteren 2 Stunden Rückfluss wurde das Reaktionsgemisch auf 0°C abgekühlt und ein starker Strom von Kohlendioxid-Gas eingeleitet (ca. 1 Stunde). Danach wurde auf Eis gegossen, mit 2N Salzsäure angesäuert und mit Essigester extrahiert. Die organischen Extrakte wurden mit Wasser gewaschen, getrocknet und eingedampft. Nach Umkristallisation aus Essigester/Hexan erhielt man 5,3 g 3,4-Dihydro-4,4-dimethyl-2H-1-benzopyran -7-carbonsäure, Schmelzpunkt 173-174°C.

1,4 g der so erhaltenen Säure wurden mit 20 ml Thionylchlorid 1 Stunde zum Rückfluss erhitzt. Das überschüssige Thionylchlorid wurde anschliesend im Wasserstrahlvakuum abdestilliert, das zurückbleibende Säurechlorid in 20 ml Tetrahydrofuran gelöst und bei Raumtemperatur zu einer Lösung von 1,2 g 4-Amino-benzoesäureäthylester in 30 ml Pyridin zugetropft. Nach einstündigem Rühren wurde auf Eiswasser gegossen und mit Essigester extrahiert. Die organischen Extrakte wurden zweimal mit 2N Salzsäure und Wasser gewaschen, getrocknet und eingedampft. Nach Filtration des Rohproduktes über eine Kieselgelsäule (Eluierungsmittel Hexan/Essigester 2:1) erhielt man 2,4 g p-(3,4-Dihydro-4,4-dimethyl -2H-1-benzopyran-7-carboxamido)benzoesäureäthylester als farbloses Oel.

Der Aethylester wurde in 20 ml Aethanol gelöst und mit einer Lösung von 1,9 g Kaliumhydroxid in 10 ml Wasser versetzt. Nach einstündigem Rühren bei 40°C wurde aus Eis gegossen, mit kalter 2N Salzsäure angsäuert und mit Essigester extrahiert. Die organischen Extrakte wurden mit Wasser gewaschen, getrocknet und eingedampft. Nach Umkristallisation des Rohproduktes aus Essigester/Hexan erhielt man 2,1 g p-(3,4-Dihydro-4,4-dimethyl -2H-1-benzopyran-7-carboxamido)benzoesäure in farblosen Kristallen, Schmelzpunkt 279-281°C.

### Beispiel 44

11 g 7-Brom-3,4-dihyro-4,4-dimethyl-2H-1-benzothiopyran wurden in einem Gemisch von 100 ml Aether und 10 ml Tetrahydrofuran gelöst und bei -78°C mit 33 ml einer 1,6 molaren Lösung von n-Butyllithium in Hexan versetzt. Die Reaktionslösung wurde 15 Minuten bei -50°C gehalten, erneut auf -78°C gekühlt und 2 Stunden mit Kohlendioxid begast. Danach wurde auf Eis gegossen, mit 6N Salzsäure angesäuert und mit Essigester extrahiert. Die organischen Phasen wurden mit Wasser gewaschen, getrocknet und eingedampft. Nach Umkristallisation des Rohproduktes aus Essigester/Hexan erhielt man 4,6 g 3,4-Dihydro-4,4-dimethyl-2H-1-benzothiopyran -7-carbonsäure, Schmelzpunkt 202-204°C.

5,4 g dieser Säure wurden mit 30 ml Oxalylchlorid versetzt und während 1 Stunde am Rückfluss erwärmt. Nach dem Abdampfen des überschüssigen Säurechlorides wurde der Rückstand in 50 ml Tetrahydrofuran gelöst und bei Raumtemperatur zu einer Lösung von 3,6 g 4-Amino-benzoesäureäthylester und 70 ml Pyridin zugetropft. Aufarbeitung in Analogie zu Beispiel 57 und Umkristallisation aus Essigester/Hexan ergab 6,6 g Aethyl p-(3,4-dihydro-4,4-dimethyl -2H-1-benzothiopyran-7-carboxamido)benzoat in weissen Kristallen, Schmelzpunkt 148-150°C.

Hydrolyse dieser Verbindung mit Kaliumhydroxid/Aethanol/Wasser in Analogie zu Beispiel 43 führte zu p-(3,4-Dihydro-4,4-dimethyl -2H-1-benzothiopyran-7-carboxamido)benzoesäure, Schmelzpunkt 274-276°C.

### Beispiel 45

Oxidation von 4 g Aethyl p-(3,4-dihydro-4,4-dimethyl-2H -1-benzothiopyran-7-carboxamido)benzoat mit m-Chloroperbenzoesäure in Analogie zu Beispiel 4 ergab nach Umkristallisation aus Essigester/Hexan 4,2 g Aethyl p-(3,4-dihydro-4,4-dimethyl-2H -1-benzothiopyran-7-carboxamido)benzoat 1,1-dioxid, Schmelzpunkt 208-210°C. Hydrolyse dieser Verbindung in Analogie zu Beispiel 43 führte zu p-(3,4-Dihydro-4,4-dimethyl-2H -1-benzothiopyran-7-carboxamido)benzoesäure-1,1-dioxid, Schmelzpunkt 314-316°C.

### Beispiel 46

2,7 g 7-Bromo-1,2,3,4-tetrahydro-1,4,4-trimethylchinolin wurden in 100 ml Aether gelöst und bei -78°C mit 27 ml n-Butyllithium (1,6 molar in Hexan) versetzt. Nach 3-stündigem Rühren bei -40°C leitete man während 1,5 Stunden Kohlendioxid-Gas ein. Die Aufarbeitung erfolgte in Analogie zu Beispiel 43 und ergab nach Umkristallisation aus Essigester/Hexan 1,9 g 1,2,3,4-Tetrahydro-1,4,4-trimethyl -7-chinolincarbonsäure, Schmelzpunkt 166-168°C.

1,4 g dieser Säure wurden in Analogie zu Beispiele 54 mit Oxalylchlorid in das Säurechlorid überführt und durch Umsetzung mit 1,1 g 4-Aminobenzoesäureäthylester in 100 ml Pyridin zu 1,7 g Aethyl p-(1,2,3,4-tetrahydro-1,4,4-trimethyl -7-chinolin-carboxamido)benzoat umgesetzt, Schmelzpunkt 118-119°C (aus Aether/Hexan).

Hydrolyse dieser Verbindung mit Kaliumhydroxid/Wasser/Aethanol ergab nach Umkristallisation aus Essigester/Hexan p-(1,2,3,4-Tetrahydro-1,4,4-trimethyl -7-chinolin-carboxamido)benzoesäure, Schmelzpunkt 279°C (Zersetzung).

### Beispiel 47

In Analogie zu Beispiel 46 wurde aus 7-Bromo-1-decyl-1,2,3,4-tetrahydro -4,4-dimethylchinolin Aethyl p-(1-decyl-1,2,3,4-tetrahydro-4,4-dimethyl -7-chinolincarboxamido)benzoat hergestellt, Schmelzpunkt 105-106°C (aus Essigester/Hexan).

Hydrolyse des Esters in Analogie zu Beispiel 43 lieferte p-(1-Decyl-1,2,3,4-tetrahydro-4,4-dimethyl -7-chinolincarboxamido)benzoesäure, Schmelzpunkt 194-196°C (aus Essigester/Hexan).

### Beispiel 48

3,7 g 1,4-Benzodioxan-6-carbonsäure wurden mit 50 ml Thionylchlorid versetzt. Nach einstündigem Kochen am Rückfluss wurde das überschüssige Thionylchlorid abgedampft, der Rückstand in 15 ml Tetrahydrofuran gelöst und bei Raumtemperatur zu einer Lösung von 3,3 g 4-Aminobenzoesäureäthylester in 80 ml Pyridin zugetropft. Nach zweistündigem Rühren wurde auf Eiswasser gegossen, mit Essigester extrahiert, die organischen Phasen mit 2N Salzsäure und Wasser gewaschen, getrocknet und eingedampft. Nach Umkristallisation aus Essigester/Hexan erhielt man 5,6 g Aethyl-p-(1,4-benzodioxan-6-carboxamido)benzoat, Schmelzpunkt 134-136°C.

Hydrolyse dieser Verbindung mit Kaliumhydroxid/Wasser/Aethanol bei 50°C während 2 Stunden lieferte p-(1,4-Benzodioxan-6-carboxamido)benzoesäure, Schmelzpunkt 278-280°C.

### Beispiel 49

2,5 ml Methyljodid wurden in 30 ml Aether gelöst und unter leichtem Rückfluss zu einer Suspension von 972 mg Magnesiumspänen in 15 ml Aether zugetropft. Nachdem alles Magnesium gelöst war, wurde eine Lösung von 6,2 g 1,2,3,4-Tetrahydro-1,4-dimethyl-6-formyl-chinoxalin in 15 ml Aether unter leichter Kühlung tropfenweise zugegeben. Nach dreistündigem Rühren bei Raumtemperatur wurde unter Eiskühlung eine wässrige Lösung von Ammoniumchlorid zugetropft und anschliessend das Reaktionsgemisch mit Aether extrahiert. Nach dem Trocknen und Eindampfen der organischen Extrakte wurde das Rohprodukt durch Flash-Chromatographie (Kieselgel, Eluierungsmittel Hexan/Essigester 1:1) weiter gereinigt und ergab 6 g eines gelben Oeles.

Dieses Oel wurde in 300 ml Acetonitril gelöst und 11 g Triphenylphosphinhydrobromid zugegeben. Nach 16-stündigem Rühren wurde eingedampft, der Rückstand in 300 ml Aethanol/Wasser (8:2) gelöst und mehrfach mit Hexan extrahiert. Die wässrige Phase wurde eingedampft, in Methylenchlorid gelöst, erneut eingedampft, wieder mit Methylenchlorid aufgenommen, über Natriumsulfat getrocknet und eingedampft. Man erhielt 12,4 g eines grünlichen, extrem hygroskopischen Phosphoniumsalzes als amorphes Pulver.

10,3 g dieses Phosphoniumsalzes wurden in 200 ml Tetrahydrofuran gelöst und bei -78°C mit 19 ml einer 1,6 molaren Lösung von n-Butyllithium in Hexan versetzt. Nach 1-stündigem Rühren bei -78°C wurde eine Lösung von 5,1 g 4-Formyl-benzoesäuremethylester in 30 ml Tetrahydrofuran dazugetropft. Man liess auf Raumtemperatur kommen und rührte noch 2 Stunden. Nach Aufarbeitung in Analogie zu Beispiel 3 wurde das Rohprodukt durch Flash-Chromatographie (Kieselgel, Eluierungsmittel Hexan/Essigester = 4:1) gereinigt und aus Hexan umkristallisiert. Man erhielt 3,6 g Methyl-p-[(E)-2-(1,2,3,4-Tetrahydro-1,4-dimethyl -6-chinoxalinyl)propenyl]benzoat in gelben Kristallen, Schmelzpunkt 91-93°C.

600 mg dieses Esters wurden durch Reaktion mit einer Lösung von 1,5 g Kaliumhydroxid in 25 ml Aethanol und 10 ml Wasser bei 50°C während 4 Stunden in die freie Säure überführt. Man erhielt nach Umkristallisation aus Essigester/Hexan 450 mg p-[(E)-2-(1,2,3,4-Tetrahydro-1,4-dimethyl -6-chinoxalinyl)propenyl]benzoesäure in orangen Kristallen, Schmelzpunkt 203-205°C.

### Beispiel 50

In Analogie zu Beispiel 33 wurde hergestellt:
p-(2,3,4,5-Tetrahydro-5-methyl-1-benzothiepin-8-carboxamido)benzoesäure, Smp. >270°;
p-(3,4-Dihydro-3,3-dimethyl-2H-1,5-benzodithiepin-7-carboxamido)benzoesäure, Smp. >250°;
p-(3,4-Dihydro-3,3-dimethyl-2H-1,5-benzodioxepin-7-carboxamido)benzoesäure, Smp. 261-262°; und

### Beispiel 51

Unter Argonatmosphäre wurde aus 473 mg Magnesiumspänen und 1,73 ml Benzylchlorid in 30 ml Tetrahydrofuran nach Standardverfahren die Grignard-Verbindung hergestellt. Bei 0°C gab man 2,12 g 3,4-Dihydro-3,3-dimethyl-2H-1,5-benzodithithiepin -7-yl-methylketon unverdünnt zu und liess 1 Stunde bei Raumtemperatur nachreagieren. Man hydrolysierte mit NH₄Cl-Lösung, extrahierte mit Aether, wusch mit Wasser, trocknete und dampfte ein. Das so gewonnene Rohprodukt wurde in 20 ml Toluol aufgenommen, mit 500 mg p-Toluolsulfonsäure versetzt und über Nacht bei 75°C gerührt. Dabei trat Dehydratisierung und Isomerisierung ein. Man entfernte das Lösungsmittel im Vakuum und reinigte mittels Säulenchromatographie an Kieselgel (Petroläther). Umkristallisation aus Hexan ergab schliesslich 1,75 g 3,4-Dihydro-3,3-dimethyl-7-[(E)-α-methylstyryl] -2H-1,5-benzodithiepin als weisse Kristalle vom Schmelzpunkt 76-77°C.

In analoger Weise wurde das 3,4-Dihydro-3,3-dimethyl -7-[(E)-α-methylstyryl)-2H-1,5-benzodioxepin, Schmelzpunkt 45-48°C, hergestellt.

### Beispiel 52

Weitere Beispiele von Verbindungen der Formel I sind die folgenden:
Methyl p-[(E)-2-(2-methyl-1,3-benzodithiol-5-yl)propenyl]benzoat, Smp. 72-73°C (Hexan);
p-[(E)-2-(2-Methyl-1,3-benzodithiol-5-yl)propenyl]benzoesäure, Smp. 193-195°C (AcOEt);
Aethyl p-(2,2-Dimethyl-1,3-benzoxathiol-5-carboxamido)benzoat, Smp. 137-138°C;
p-(2,2-Dimethyl-1,3-benzoxathiol-5-carboxamido)benzoesäure, Smp. 284-286°C;
Aethyl p-(2,2-dimethyl-1,3-benzodioxol-5-carboxamido)benzoat, Smp. 143-144°C;
2-[p-[(E)-3,4-(Isopropylidendioxy)-β-methylstyryl]phenoxy]-N,N-dimethyläthylamin, Smp. 54-56°C;
Methyl p-[(E)-2-(1,2,3,4-Tetrahydro-1,2,3,4-tetramethyl-chinoxalin-6-yl)propenyl]benzoat, Smp. 90-93°C;
p-[(E)-2-(1,2,3,4-Tetrahydro-1,2,3,4-tetramethyl-chinoxalin-6-yl)propenyl]benzoesäure, Smp. 199-200°C;
p-[(E)-2-(2,2-Dimethyl-1,3-benzodioxol-5-yl)propenyl]benzoesäure, Smp. 185-186°C;
p-(2,2-Dimethyl-1,3-benzodioxol-5-carboxamido)benzoesäure, Smp. 281-283°C;
p-(1,4-Benzodithiin-6-carboxamido)benzoesäure und deren Aethylester;
p-(1,2,3,4-Tetrahydro-1,4-dimethyl-6-chinoxalincarboxamido)benzoesäure und deren Aethylester;
p-(1,2,3,4-Tetrahyro-1,2,3,4-tetramethyl-6-chinoxalincarboxamido)benzoesäure und deren Aethylester;
4-[2-[p-[(E)-2-(1,2,3,4-Tetrahydro-1,4,4-trimethylchinolin-7-yl]propenyl]phenoxy]äthyl]morpholin;
4-[2-[p-[(E)-2-(3,4-Dihydro-4,4-dimethyl-2H-1-benzothiopyran-7-yl]propenyl]phenoxy]äthyl]morpholin;
p-[(E)-2-(3,4-Dihydro-4,4-dimethyl-2H-1-benzothiopyran-7-yl]propenyl]phenol;
p-[2-(3,4-Dihyro-4,4,6-trimethyl-2H-1-benzopyran-7-yl)propenyl]benzoesäure;
p-[1,2,3,4-Tetrahydro-1,4,4-trimethyl-7-chinolinyl)carbamoyl]benzoesäure;
p-[2-(3,4-Dihydro-4,4-dimethyl-6-methoxy-2H-1-benzothiopyran-7-yl)propenyl]benzoesäure;

### Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 200 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 3̅0̅0̅ |

Das sprühgetrocknete Pulver, das auf dem Wirkstoff, Gelatine und mikrokristalliner Cellulose basiert und eine mittlere Korngrösse des Wirkstoffes von <1 µ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

### Beispiel B

Tabletten können wie folgt hergestellt werden:

| Bestandteile | mg/Tablette |
|---|---|
| 1. Verbindung I als feingemahlenes Pulver | 500 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| | Total 8̅0̅0̅ |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

### Beispiel C

Weichgelatinekapseln können wie folgt hergestellt werden:

| Bestandteile | mg/Kapsel |
|---|---|
| 1. Verbindung I | 50 |
| 2. Triglycerid | 450 |
| | Total 5̅0̅0̅ |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 50 mg Wirkstoff verarbeitet.

### Beispiel D

Eine Lotion kann wie folgt hergestellt werden:

| Bestandteile | |
|---|---|
| 1. Verbindung I, feingemahlen | 3,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

## Patentansprüche (Patentansprüche für folgende(n) Vertragsstaat(en): AT, BE, CH, DE, FR, GB, IT, LI, LU, NL, SE)

1. Verbindungen der allgemeinen Formel 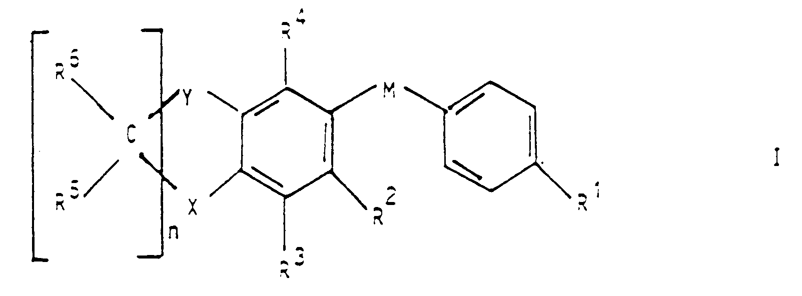 worin R¹ Wasserstoff, Acyl, nieder-Alkyl oder eine Gruppe -CHO, -CH₂OR¹⁰, -COR⁷, oder OR¹³; R², R³ und R⁴ Wasserstoff, nieder-Alkyl, nieder-Alkoxy oder Halogen; R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(R¹¹)=C(R¹²)-; -CONH-, oder -NH-CO-; R¹¹, R¹², R¹⁴ und R¹⁵ Wasserstoff oder nieder-Alkyl, R¹³ Wasserstoff, nieder-Alkyl, das durch Amino, mono- oder di-Alkylamino, Morpholino, Thiamorpholino oder Piperazino substituiert sein kann, oder nieder-Alkoxycarbonyl; und n 1, 2, 3 oder 4 bedeuten; und wobei "nieder" Gruppen mit 1-6 C-Atomen bezeichnet
und Salze von Verbindungen der Formel I in denen R¹ eine Carboxygruppe darstellt, zur Verwendung als Heilmittel.

2. Verbindungen gemäss Anspruch 1, wobei R¹³ Wasserstoff oder nieder-Alkyl, das durch Amino, mono- oder di-Alkylamino, Morpholino, Thiamorpholino oder Piperazino substituiert sein kann,
darstellt und die übrigen Symbole die in Anspruch 1 angegebene Bedeutung haben, zur Verwendung als Heilmittel.

3. Verbindungen gemäss Anspruch 1 oder 2, wobei M C(R¹¹)=C(R¹²)- darstellt, zur Verwendung als Heilmittel.

4. Verbindungen gemäss Anspruch 1 oder 2, wobei M -CONH-darstellt, zur Verwendung als Heilmittel.

5. Verbindungen gemäss Anspruch 1-4, wobei X und Y -O-, -S-, >SO, >SO₂ oder >NR¹⁸ sind, zur Verwendung als Heilmittel.

6. Verbindungen gemäss den Ansprüchen 1-5, wobei X und Y -S- sind , zur Verwendung als Heilmittel.

7. Verbindungen der allgemeinen Formel 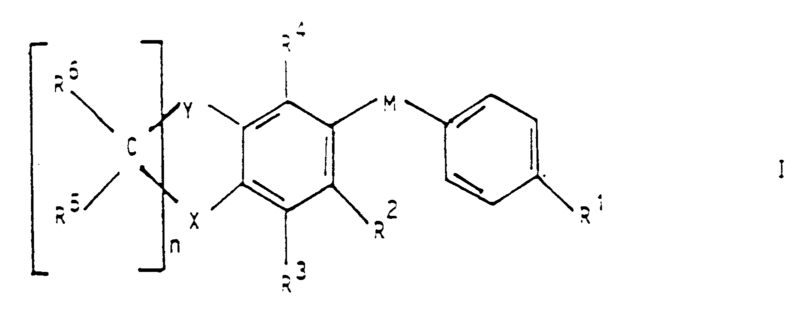 worin R¹ Acyl, oder eine Gruppe -CHO, -CH₂OR¹⁰, -COR⁷, oder OR¹³; R², R³ und R⁴ Wasserstoff, nieder-Alkyl, nieder-Alkoxy oder Halogen; R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(CH₃)=CH-, -CONH-, oder -NH-CO-; R¹⁴ und R¹⁵ Wasserstoff oder nieder-Alkyl, R¹³, nieder-Alkyl, das durch Amino, mono- oder di-Alkylamino, Morpholino, Thiamorpholino oder Piperazino substituiert ist, oder nieder-Alkoxycarbonyl; und n 1, 2, 3 oder 4 bedeuten; und wobei "nieder" Gruppen mit 1-6 C-Atomen bezeichnet
und Salze von Verbindungen der Formel I in denen R¹ eine Carboxygruppe darstellt.

8. Verbindungen gemäss Anspruch 7, wobei R¹³ nieder-Alkyl, das durch Amino, mono- oder di-Alkylamino, Morpholino, Thiamorpholino oder Piperazino substituiert ist, darstellt und die übrigen Symbole die in Anspruch 1 angegebene Bedeutung haben.

9. Verbindungen gemäss Anspruch 7 oder 8, wobei M -C(CH₃)=CH- darstellt.

10. Verbindungen gemäss Anspruch 7 oder 8, wobei M -CONH-darstellt.

11. Verbindungen gemäss Anspruch 7-10 wobei X und Y -O-, -S-, >SO, >SO₂ oder >NR¹⁸ sind.

12. Verbindungen gemäss Anspruch 11, wobei X und Y -S- sind.

13. Die Verbindungen gemäss Anspruch 7,
p-[2-(3,4-Dihydro-4,4-dimethyl-2H-1-benzopyran-7-yl)propenyl]benzoesäure-äthylester,
p-[2-(3,4-Dihydro-4,4-dimethyl-2H-1-benzopyran-7-yl)propenyl]benzoesäure,
Aethyl p-[2-(3,4-dihydro-4,4-dimethyl-2H-1-benzothiopyran-7-yl)propenyl]benzoat,
p-[2-(3,4-Dihydro-4,4-dimethyl-2H-1-benzothiopyran-7-yl)propenyl]benzoesäure,
Aethyl-p-[2-(3',4'-dihydro-4',4'-dimethyl-2'H-1-benzothiopyran-7'-yl)propenyl]benzoat-1',1'-dioxid,
p-[2-(3',4'-Dihydro-4'4'-dimethyl-2'H-1-benzothiopyran-7'-yl)propenyl]benzoesäure 1',1'-dioxid,
Methyl p-[(E)-2-(2,3,4,5-tetrahydro-5-methyl-1-benzothiepin-8-yl)propenyl]benzoat,
Aethyl p-[2-(1,2,3,4-Tetrahydro-1,4,4-trimethyl-7-chinolinyl)propenyl]benzoat,
p-[2-(1,2,3,4-Tetrahyro-1,4,4-trimethyl-7-chinolinyl)propenyl]benzoesäure,
p-[(E)-2-(2,3,4,5-Tetrahydro-1-benzoxepin-8-yl)propenyl]benzoesäureäthylester,
p-[(E)-2-(2,3,4,5-Tetrahydro-3,3-dimethyl-2H-1-benzothiepin-8-yl)propenyl]benzoesäuremethylester,
p-[(E)-2-(2,3,4,5-Tetrahydro-1-benzothiepin-8-yl)propenyl]benzoesäureäthylester,
p-[(E)-2-(2',3',4',5'-Tetrahydro-1-benzothiepin-8'-yl)propenyl]benzoesäure-1',1'-dioxid,
p-[(E)-2-(2,3,4,5-Tetrahydro-1-benzothiepin-8-yl)propenyl]benzoesäure,
p-(2,3,4,5-Tetrahydro-1-benzothiepin-8-carboxamido)benzoesäureäthylester,

14. Die Verbindungen gemäss Anspruch 11
Aethyl p-[(E)-2-(1,4-benzodioxan-6-yl)propenyl]benzoat,
p-[(E)-2-(1,4-Benzodioxan-6-yl)propenyl]benzoesäure,
Aethyl p-[(E)-2-(1,4-benzodithian-6-yl)propenyl]benzoat,
p-[(E)-2-(1,4-Benzodithian-6-yl)propenyl)benzoesäure,
p-[(E)-2-(2,2-Dimethyl-1,3-benzodioxol-5-yl)propenyl]benzoesäuremethylester,
p-[(E)-2-(3,4-Dihydro-3,3-dimethyl-2H-1,5-benzodioxepin-7-yl)propenyl]benzoesäureäthylester,
p-[(E)-2-(3,4-Dihydro-3-methyl-2H-1,5-benzodithiepin-7-yl)propenyl]benzoesäureäthylester,
p-[(E)-2-(3,4-Dihydro-2H-1,5-benzodithiepin-7-yl)propenyl]benzoesäureäthylester,
p-[(E)-2-(3',4'-Dihydro-3',3'-dimethyl-2'H-1,5-benzodithiepin-7'-yl)propenyl]benzoesäureäthylester-1',1',5',5'-tetroxid,
p-[(E)-2-(3,4-Dihydro-3-methyl-2H-1,5-benzodithiepin-7-yl)propenyl]benzoesäure,
p-[(E)-2-(3,4-Dihydro-3,3-dimethyl-2H-1,5-benzodioxepin-7-yl)propenyl]benzoesäure,
p-[(E)-2-(3,4-Dihydro-3,3-dimethyl-2H-1,5-benzodithiepin-7-yl)propenyl]benzoesäuremethylester,
p-[(E)-2-(2,3-Dihydro-3,3-dimethyl-1,4-benzoxathiin-7-yl)propenyl]benzoesäureäthylester,
p-[(E)-2-(2,3,4,5-Tetrahydro-1,3,3,5-tetramethyl-1H-1,5-benzodiazepin-7-yl)propenyl]benzoesäuremethylester,
4-[2-[p-[(E)-2-(3,4-Dihydro-3,3-dimethyl-2H-1,5-benzodithiepin-7-yl)propenyl]phenoxy]äthyl]morpholin,
4-[2-[p-[(E)-2-(3,4-Dihydro-3,3-dimethyl-2H-1,5-benzodioxepin-7-yl)propenyl]phenoxy]äthyl]morpholin,
p-[(E)-2-(3,4-Dihydro-3,3-dimethyl-2H-1,5-benzodioxepin-7-yl)propenyl]phenyläthylcarbonat,
p-[(E)-2-(3,4-Dihydro-5-methyl-2H-1,5-benzothiazepin-8-yl)propenyl]benzoesäuremethylester,
p-[(E)-2-(3,4-Dihydro-3,5-dimethyl-2H-1,5-benzothiazepin-8-yl)propenyl]benzoesäuremethylester,
N,N-Dimethyl-2-[p-[(E)-2-(3,4-dihydro-3,3-dimethyl-2H-1,5-benzodithiepin-7-yl)propenyl]phenoxy]äthylamin,
p-[(E)-2-(2,3-Dihydro-3,3-dimethyl-1,4-benzoxathiin-7-yl)propenyl]benzoesäure,
p-[(E)-2-(3,4-Dihydro-2H-1,5-benzodithiepin-7-yl)propenyl]benzoesäure,
p-[(E)-2-(3,4-Dihydro-5-methyl-2H-1,5-benzothiazepin-8-yl)propenyl]benzoesäure,
p-[(E)-2-(3,4-Dihydro-3,5-dimethyl-2H-1,5-benzothiazepin-8-yl)propenyl]benzoesäure,
Methyl p-[(E)-2-(2,2-dimethyl-1,3-benzodithiol-5-yl)propenyl]benzoat,
4-[2-[p-[(E)-2-(2,2-Dimethyl-1,3-benzodithiol-5-yl)propenyl]phenoxy]äthyl]morpholin,
Aethyl p-[(E)-2-(2,2-Dimethyl-1,3-benzodithiol-5-yl)propenyl]phenylcarbonat,
p-[(E)-2-(2,2-Dimethyl-1,3-benzodithiol-5-yl)propenyl]benzoesäure,
Methyl-p-[(E)-2-(1,2,3,4 -Tetrahydro-1,4-dimethyl-6-chinoxalinyl)propenyl]benzoat,
p-[(E)-2-(1,2,3,4-Tetrahydro-1,4-dimethyl-6-chinoxalinyl)propenyl]benzoesäure,
Methyl p-[(E)-2-(2-methyl-1,3-benzodithiol-5-yl)propenyl]benzoat,
p-[(E)-2-(2-Methyl-1,3-benzodithiol-5-yl)propenyl]benzoesäure,
Methyl p-[(E)-2-(1,2,3,4-Tetrahydro-1,2,3,4-tetramethyl-chinoxalin-6-yl)propenyl]benzoat,
p-[(E)-2-(2,2-Dimethyl-1,3-benzodioxol-5-yl)propenyl]benzoesäure,
p-(2,2-Dimethyl-1,3-benzodioxol-5-carboxamido)benzoesäure.

15. Die Verbindungen gemäss Anspruch 10,
p-(2,3,4,5-Tetrahydro-5-methyl-1-benzothiepin-8-carboxamido)benzoesäureäthylester,
p-(2,3,4,5-Tetrahydro-1-benzothiepin-8-carboxamido)benzoesäure,
p-(3,4-Dihydro-4,4-dimethyl-2H-1-benzopyran-7-carboxamido)benzoesäureäthylester,
p-(3,4-Dihydro-4,4-dimethyl-2H-1-benzopyran-7-carboxamido)benzoesäure,
Aethyl p-(3,4-dihydro-4,4-dimethyl-2H-1-benzothiopyran-7-carboxamido)benzoat,
p-(3,4-Dihydro-4,4-dimethyl-2H-1-benzothiopyran-7-carboxamido)benzoesäure,
Aethyl p-(3,4-dihydro-4,4-dimethyl-2H-1-benzothiopyran-7-carboxamido)benzoat 1,1-dioxid,
p-(3,4-Dihydro-4,4-dimethyl-2H-1-benzothiopyran-7-carboxamido)benzoesäure-1,1-dioxid,
Aethyl p-(1,2,3,4-tetrahydro-1,4,4-trimethyl-7-chinolin-carboxamido)benzoat,
p-(1,2,3,4-Tetrahydro-1,4,4-trimethyl-7-chinolin-carboxamido)benzoesäure,
Aethyl p-(1-decyl-1,2,3,4-tetrahydro-4,4-dimethyl-7-chinolincarboxamido)benzoat,
p-(1-Decyl-1,2,3,4-tetrahydro-4,4-dimethyl-7-chinolincarboxamido)benzoesäure,
p-(2,3,4,5-Tetrahydro-5-methyl-1-benzothiepin-8-carboxamido)benzoesäure.

16. Die Verbindungen gemäss Anspruch 11
p-(3,4-Dihydro-3,3-dimethyl-2H-1,5-benzodithiepin-7-carboxamido)benzoesäureäthylester,
Aethyl-p-(1,4-benzodioxan-6-carboxamido)benzoat,
p-(1,4-Benzodioxan-6-carboxamido)benzoesäure,
p-(3,4-Dihydro-3,3-dimethyl-2H-1,5-benzodithiepin-7-carboxamido)benzoesäure,
p-(3,4-Dihydro-3,3-dimethyl-2H-1,5-benzodioxepin-7-carboxamido)benzoesäure,
Aethyl p-(2,2-Dimethyl-1,3-benzoxathiol-5-carboxamido)benzoat,
p-(2,2-Dimethyl-1,3-benzoxathiol-5-carboxamido)benzoesäure,
Aethyl p-(2,2-dimethyl-1,3-benzodioxol-5-carboxamido)benzoat,
Aethyl p-(2,2-dimethyl-1,3-benzodioxol-6-carboxamido)benzoat,
2-[p-[(E)-3,4-(Isopropylidendioxy)-β-methylstyryl]phenoxy]-N,N-dimethyläthylamin,
p-(2,2-Dimethyl-1,3-benzodioxol-5-carboxamido)benzoesäure, und
p-(3,4-Dihydro-3,3-dimethyl-2H-1,5-benzodioxepin-7-carboxamido)benzoesäureäthylester,

17. Die Verbindung gemäss Anspruch 7,
p-[(E)-2-(3,4-Dihydro-3,3-dimethyl-2H-1,5-benzodithiepin-7-yl)propenyl]benzoesäure und deren nieder-Alkylester.

18. Die Verbindung gemäss Anspruch 7,
p-[(E)-2-(3,4-Dihydro-3,3-dimethyl-2H-1,5-benzodithiepin-7-yl)propenyl]benzoesäureäthylester.

19. Verfahren zur Herstellung der Verbindungen von Anspruch 7, dadurch gekennzeichnet, dass man
a) eine Verbindung der allgemeinen Formel 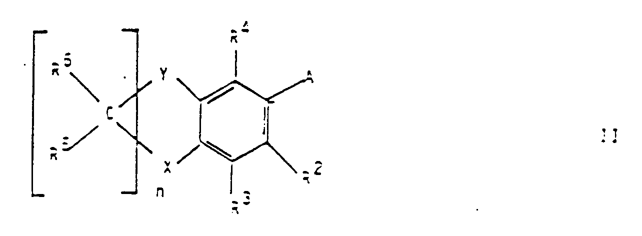 mit einer Verbindung der allgemeinen Formel 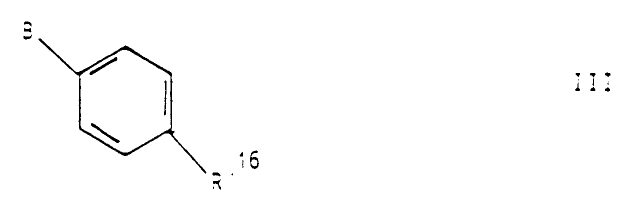 umsetzt, wobei entweder
A einen der Reste -CH(CH₃)P⁺(Q)₃Z⁻ oder -CH(CH₃)-P(O)(OAlk)₂ und B einen Formylrest darstellt; oder
A einen Rest CH₃CO und B einen der Reste -CH₂ P⁺(Q)₃Z⁻, oder -CH₂ -P-(O)(OAlk)₂ darstellt; Q Aryl; Z⁻ das Anion einer organischen oder anorganischen Säure; Alk eine niedere Alkylgruppe; und R¹⁶ einen Rest R¹ mit Ausnahme der Formyl-, Carboxyl-, und Hydroxymethylgruppe darstellt; oder
b) eine Verbindung der allgemeinen Formel 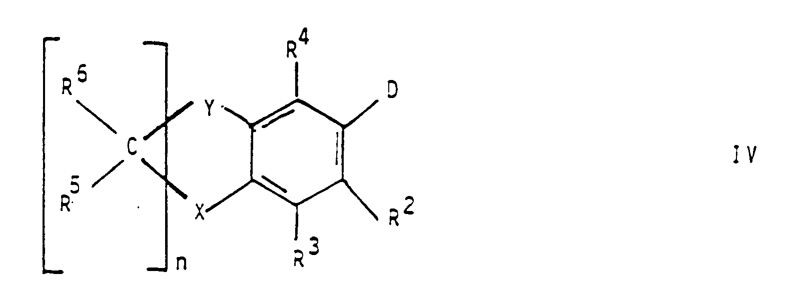 mit einer Verbindung der allgemeinen Formel 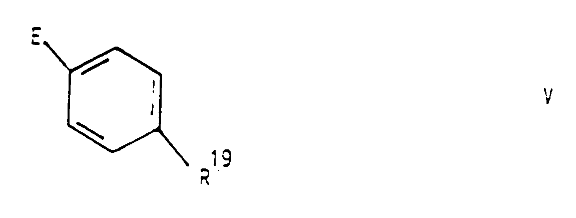 umsetzt,
wobei entweder D eine Carboxylgruppe oder ein reaktionsfähiges Derivat davon und E eine Aminogruppe bedeutet, oder D eine Aminogruppe und E eine Carboxylgruppe oder ein reaktionsfähiges Derivat davon und R¹⁹ einen Rest R¹ mit Ausnahme der Carboxyl-, und Hydroxymethylgruppe bedeutet
c) eine Verbindung der allgemeinen Formel 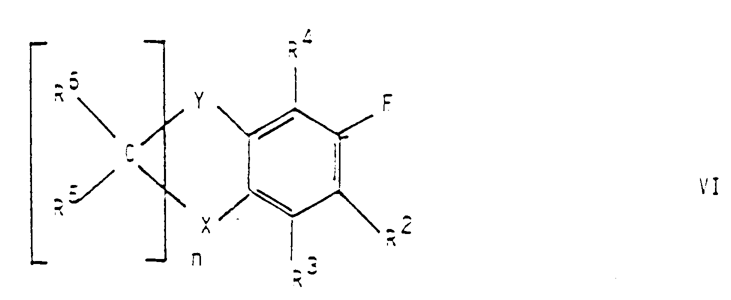 mit einer Verbindung der allgemeinen Formel 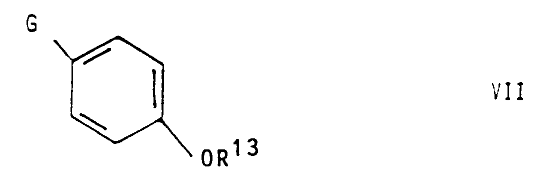 umsetzt und das Reaktionsprodukt dehydratisiert;
wobei entweder F einen Rest -CH(CH₃)MgHal und G einen Formylrest ; oder F einen Formylrest und G einen Rest -CH₂ MgHal; Hal Halogen darstellt; und
wobei "nieder" Gruppen mit 1-6 C-Atomen bezeichnet und die übrigen Symbole in den Formeln II-VII die früher angegebene Bedeutung haben;
worauf man in dem erhaltenen Reaktionsprodukt der Formel I erwünschtenfalls den Rest R¹ funktionell abwandelt und/oder das Schwefelatom in einer Verbindung I, in der X und/oder Y -S- ist, zu einer Sulfoxyl- oder Sulfonylgruppe oxidiert.

20. Pharmazeutische Präparate, enthaltend eine Verbindung der Formel I gemäss Anspruch 1 und pharmazeutisch anwendbare Salze solcher Verbindungen, die eine Carboxylgruppe enthalten.

21. Pharmazeutische Präparate, in Dosiseinheitsform, enthaltend 5 bis 500 mg einer Verbindung der Formel I gemäss Anspruch 1 und pharmazeutisch anwendbare Salze solcher Verbindungen, die eine Carboxylgruppe enthalten.

22. Verwendung einer Verbindung der Formel I gemäss Anspruch 1 und pharmazeutisch anwendbare Salze solcher Verbindungen, die eine Carboxylgruppe enthalten, als Wirkstoffe bei der Herstellung von pharmazeutischen Präparaten zur Behandlung von Neoplasien, Dermatosen und Altershaut.

## Patentansprüche (Patentansprüche für folgende(n) Vertragsstaat(en): ES, GR)

1. Verfahren zur Herstellung von Verbindungen der allgemeinen Formel 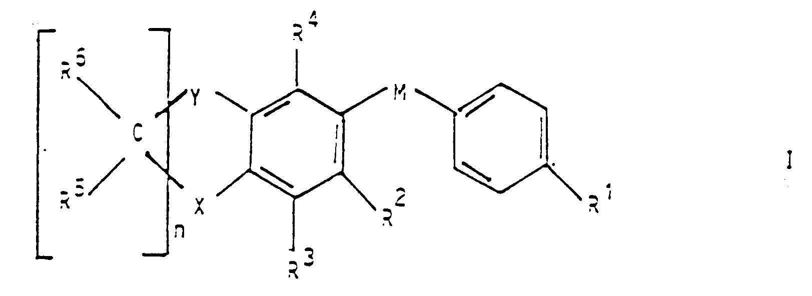 worin R¹ Acyl, oder eine
Gruppe -CHO, -CH₂OR¹⁰, -COR⁷, oder OR¹³; R², R³ und R⁴ Wasserstoff, nieder-Alkyl, nieder-Alkoxy oder Halogen; R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(CH₃)=CH-, -CONH-, oder -NH-CO-; R¹⁴ und R¹⁵ Wasserstoff oder nieder-Alkyl, R¹³, nieder-Alkyl, das durch Amino, mono- oder di-Alkylamino, Morpholino, Thiamorpholino oder Piperazino substituiert ist, oder nieder-Alkoxycarbonyl; und n 1, 2, 3 oder 4 bedeuten; und wobei "nieder" Gruppen mit 1-6 C-Atomen bezeichnet
und Salze von Verbindungen der Formel I in denen R¹ eine Carboxygruppe darstellt, dadurch gekennzeichnet, dass man
a) eine Verbindung der allgemeinen Formel 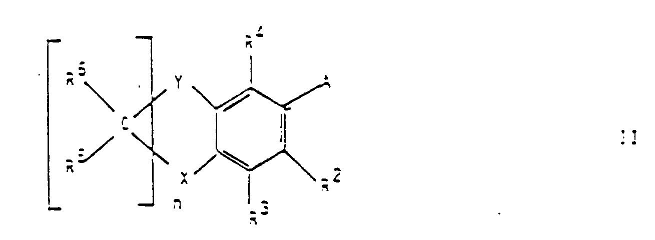 mit einer Verbindung der allgemeinen Formel 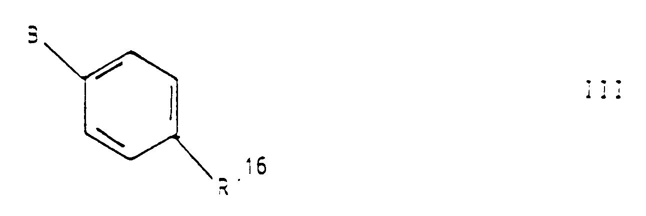 umsetzt, wobei entweder
A einen der Reste -CH(CH₃)P⁺(Q)₃Z⁻ oder -CH(CH₃)-P(O)(OAlk)₂ und B einen Formylrest darstellt; oder
A einen Rest CH₃CO und B einen der Reste -CH₂ P⁺(Q)₃Z⁻, oder -CH₂ -P-(O)(OAlk)₂ darstellt; Q Aryl; Z⁻ das Anion einer organischen oder anorganischen Säure; Alk eine niedere Alkylgruppe; und R¹⁶ einen Rest R¹ mit Ausnahme der Formyl-, Carboxyl-, und Hydroxymethylgruppe darstellt; oder
b) eine Verbindung der allgemeinen Formel 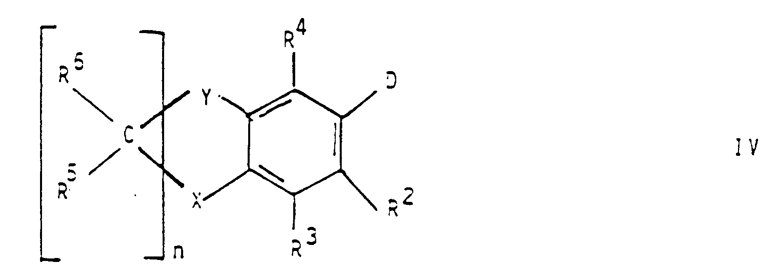 mit einer Verbindung der allgemeinen Formel 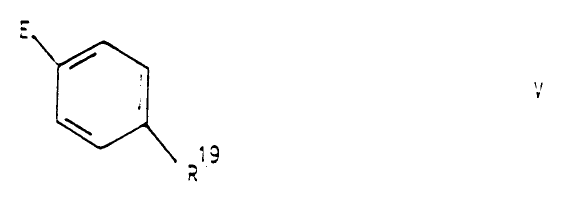 umsetzt,
wobei entweder D eine Carboxylgruppe oder ein reaktionsfähiges Derivat davon und E eine Aminogruppe bedeutet, oder D eine Aminogruppe und E eine Carboxylgruppe oder ein reaktionsfähiges Derivat davon und R¹⁹ einen Rest R¹ mit Ausnahme der Carboxyl-, und Hydroxymethylgruppe bedeutet
c) eine Verbindung der allgemeinen Formel 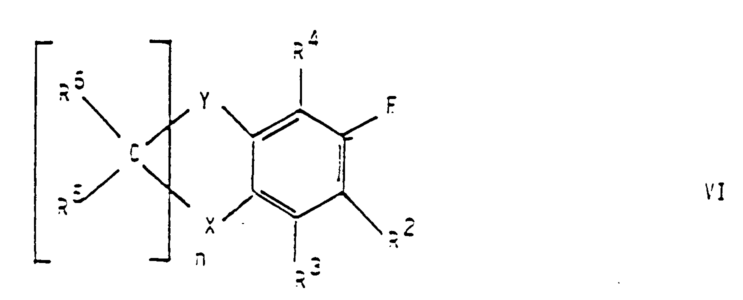 mit einer Verbindung der allgemeinen Formel 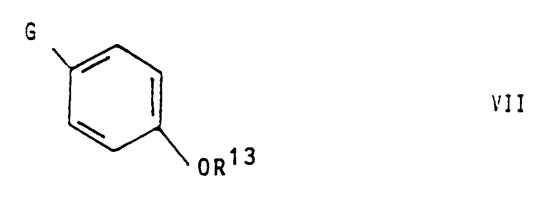 umsetzt und das Reaktionsprodukt dehydratisiert;
wobei entweder F einen Rest -CH(CH₃)MgHal und G einen Formylrest ; oder F einen Formylrest und G einen Rest -CH₂ MgHal; Hal Halogen darstellt;und
wobei "nieder" Gruppen mit 1-6 C-Atomen bezeichnet und die übrigen Symbole in den Formeln II-VII die früher angegebene Bedeutung haben;
worauf man in dem erhaltenen Reaktionsprodukt der Formel I erwünschtenfalls den Rest R¹ funktionell abwandelt und/oder das Schwefelatom in einer Verbindung I, in der X und/oder Y -S- ist, zu einer Sulfoxyl- oder Sulfonylgruppe oxidiert.

2. Verfahren gemäss Anspruch 1, zur Herstellung von Verbindungen der Formel I, wobei R¹³ nieder-Alkyl, das durch Amino, mono- oder di-Alkylamino, substituiert ist, Morpholino, Thiamorpholino oder Piperazino substituiert ist, darstellt und die übrigen Symbole die in Anspruch 1 ange- gebene Bedeutung haben.

3. Verfahren gemäss Anspruch 1 oder 2, zur Herstellung von Verbindungen der Formel I, wobei M -C(CH₃)=CH-darstellt.

4. Verfahren gemäss Anspruch 1 oder 2, zur Herstellung von Verbindungen der Formel I, wobei M -CONH- darstellt.

5. Verfahren gemäss Anspruch 1-4, zur Herstellung von Verbindung der Formel I, wobei X und Y -O-, -S-, >SO, >SO₂ oder >NR¹⁸ sind.

6. Verfahren gemäss den Ansprüchen 1-5, zur Herstellung von Verbindungen der Formel I, wobei X und Y -S- sind.

7. Verfahren gemäss Anspruch 1 zur Herstellung der Verbindungen,
p-[2-(3,4-Dihydro-4,4-dimethyl-2H-1-benzopyran-7-yl)propenyl]benzoesäure-äthylester,
p-[2-(3,4-Dihydro-4,4-dimethyl-2H-1-benzopyran-7-yl)propenyl]benzoesäure
Aethyl p-[2-(3,4-dihydro-4,4-dimethyl-2H-1-benzothiopyran-7-yl)propenyl]benzoat,
p-[2-(3,4-Dihydro-4,4-dimethyl-2H-1-benzothiopyran-7-yl)propenyl]benzoesäure,
Aethyl-p-[2-(3',4'-dihydro-4',4'-dimethyl-2'H-1-benzothiopyran-7'-yl)propenyl]benzoat-1',1'-dioxid,
p-[2-(3',4'-Dihydro-4',4'-dimethyl-2'H-1-benzothiopyran-7'-yl)propenyl]benzoesäure 1',1'-dioxid,
Methyl p-[(E)-2-(2,3,4,5-tetrahydro-5-methyl-1-benzothiepin-8-yl)propenyl]benzoat,
Aethyl p-[2-(1,2,3,4-Tetrahydro-1,4,4-trimethyl-7-chinolinyl)propenyl]benzoat,
p-[2-(1,2,3,4-Tetrahyro-1,4,4-trimethyl-7-chinolinyl)propenyl]benzoesäure,
p-[(E)-2-(2,3,4,5-Tetrahydro-1-benzoxepin-8-yl)propenyl]benzoesäureäthylester,
p-[(E)-2-(2,3,4,5-Tetrahydro-3,3-dimethyl-2H-1-benzothiepin-8-yl)propenyl]benzoesäuremethylester,
p-[(E)-2-(2,3,4,5-Tetrahydro-1-benzothiepin-8-yl)propenyl]benzoesäureäthylester,
p-[(E)-2-(2',3',4',5'-Tetrahydro-1-benzothiepin-8-yl)propenyl]benzoesäure-1',1'-dioxid,
p-[(E)-2-(2,3,4,5-Tetrahydro-1-benzothiepin-8-yl)propenyl]benzoesäure,
p-(2,3,4,5-Tetrahydro-1-benzothiepin-8-carboxamido)benzoesäureäthylester,

8. Verfahren gemäss Anspruch 5 zur Herstellung der Verbindungen,
Aethyl p-[(E)-2-(1,4-benzodioxan-6-yl)propenyl)benzoat,
p-[(E)-2-(1,4-Benzodioxan-6-yl)propenyl]benzoesäure,
Aethyl p-[(E)-2-(1,4-benzodithian-6-yl)propenyl]benzoat,
p-[(E)-2-(1,4-Benzodithian-6-yl)propenyl]benzoesäure,
p-[(E)-2-(2,2-Dimethyl-1,3-benzodioxol-5-yl)propenyl]benzoesäuremethylester,
p-[(E)-2-(3,4-Dihydro-3,3-dimethyl-2H-1,5-benzodioxepin-7-yl)propenyl]benzoesäureäthylester,
p-[(E)-2-(3,4-Dihydro-3-methyl-2H-1,5-benzoditiepin-7-yl)propenyl]benzoesäureäthylester,
p-[(E)-2-(3,4-Dihydro-2H-1,5-benzodithiepin-7-yl)propenyl)benzoesäureäthylester,
p-[(E)-2-(3',4'-Dihydro-3',3'-dimethyl-2'H-1,5-benzodithiepin-7'-yl)propenyl]benzoesäureäthylester-1',1',5',5'-tetroxid,
p-[(E)-2-(3,4-Dihydro-3-methyl-2H-1,5-benzodithiepin-7-yl)propenyl]benzoesäure,
p-[(E)-2-(3,4-Dihydro-3,3-dimethyl-2H-1,5-benzodioxepin-7-yl)propenyl]benzoesäure,
p-[(E)-2-(3,4-Dihydro-3,3-dimethyl-2H-1,5-benzodithiepin-7-yl)propenyl]benzoesäuremethylester,
p-[(E)-2-(2,3-Dihydro-3,3-dimethyl-1,4-benzoxathiin-7-yl)propenyl]benzoesäureäthylester,
p-[(E)-2-(2,3,4,5-Tetrahydro-1,3,3,5-tetramethyl-1H-1,5-benzodiazepin-7-yl)propenyl]benzoesäuremethylester,
4-[2-[p-[(E)-2-(3,4-Dihydro-3,3-dimethyl-2H-1,5-benzodi thiepin-7-yl)propenyl]phenoxy]äthyl]morpholin,
4-[2-[p-[(E)-2-(3,4-Dihydro-3,3-dimethyl-2H-1,5-benzodioxepin-7-yl)propenyl]phenoxy]äthyl]morpholin,
p-[(E)-2-(3,4-Dihydro-3,3-dimethyl-2H-1,5-benzodioxepin-7-yl)propenyl]phenyläthylcarbonat,
p-[(E)-2-(3,4-Dihydro-5-methyl-2H-1,5-benzothiazepin-8-yl)propenyl)benzoesäuremethylester,
p-[(E)-2-(3,4-Dihydro-3,5-dimethyl-2H-1,5-benzothiazepin-8-yl)propenyl]benzoesäuremethylester,
N,N-Dimethyl-2-[p-[(E)-2-(3,4-dihydro-3,3-dimethyl-2H-1,5-benzodithiepin-7-yl)propenyl]phenoxy]äthylamin,
p-[(E)-2-(2,3-Dihydro-3,3-dimethyl-1,4-benzoxathiin-7-yl)propenyl]benzoesäure,
p-[(E)-2-(3,4-Dihydro-2H-1,5-benzodithiepin-7-yl)propenyl]benzoesäure,
p-[(E)-2-(3,4-Dihydro-5-methyl-2H-1,5-benzothiazepin-8-yl)propenyl]benzoesäure,
p-[(E)-2-(3,4-Dihydro-3,5-dimethyl-2H-1,5-benzothiazepin-8-yl)propenyl]benzoesäure,
Methyl p-[(E)-2-(2,2-dimethyl-1,3-benzodithiol-5-yl)propenyl)benzoat,
4-[2-[p-[(E)-2-(2,2-Dimethyl-1,3-benzodithiol-5-yl)propenyl]phenoxy]äthyl]morpholin,
Aethyl p-[(E)-2-(2,2-Dimethyl-1,3-benzodithiol-5-yl)propenyl]phenylcarbonat,
p-[(E)-2-(2,2-Dimethyl-1,3-benzodithiol-5-yl)propenyl]benzoesäure,
Methyl-p-[(E)-2-(1,2,3,4 -Tetrahydro-1,4-dimethyl-6-chinoxalinyl)propenyl]benzoat,
p-[(E)-2-(1,2,3,4-Tetrahydro-1,4-dimethyl-6-chinoxalinyl)propenyl]benzoesäure,
Methyl p-[(E)-2-(2-methyl-1,3-benzodithiol-5-yl)propenyl]benzoat,
p-[(E)-2-(2-Methyl-1,3-benzodithiol-5-yl)propenyl]benzoesäure,
Methyl p-[(E)-2-(1,2,3,4-Tetrahydro-1,2,3,4-tetramethyl-chinoxalin-6-yl)propenyl]benzoat,
p-[(E)-2-(2,2-Dimethyl-1,3-benzodioxol-5-yl)propenyl]benzoesäure,
p-(2,2-Dimethyl-1,3-benzodioxol-5-carboxamido)benzoesäure.

9. Verfahren gemäss Anspruch 4 zur Herstellung der Verbindungen,
p-(2,3,4,5-Tetrahydro-5-methyl-1-benzothiepin-8-carboxamido)benzoesäureäthylester,
p-(2,3,4,5-Tetrahydro-1-benzothiepin-8-carboxomido)benzoesäure,
p-(3,4-Dihydro-4,4-dimethyl-2H-1-benzopyran-7-carboxamido)benzoesäureäthylester,
p-(3,4-Dihydro-4,4-dimethyl-2H-1-benzopyran-7-carboxamido)benzoesäure,
Aethyl p-(3,4-dihydro-4,4-dimethyl-2H-1-benzothiopyran-7-carboxamido)benzoat,
p-(3,4-Dihydro-4,4-dimethyl-2H-1-benzothiopyran-7-carboxamido)benzoesäure,
Aethyl p-(3,4-dihydro-4,4-dimethyl-2H-1-benzothiopyran-7-carboxamido)benzoat 1,1-dioxid,
p-(3,4-Dihydro-4,4-dimethyl-2H-1-benzothiopyran-7-carboxamido)benzoesäure-1,1-dioxid,
Aethyl p-(1,2,3,4-tetrahydro-1,4,4-trimethyl-7-chinolincarboxamido)benzoat,
p-(1,2,3,4-Tetrahydro-1,4,4-trimethyl-7-chinolincarboxamido)benzoesäure,
Aethyl p-(1-decyl-1,2,3,4-tetrahydro-4,4-dimethyl-7-chinolincarboxamido)benzoat,
p-(1-Decyl-1,2,3,4-tetrahydro-4,4-dimethyl-7-chinolincarboxamido)benzoesäure,
p-(2,3,4,5-Tetrahydro-5-methyl-1-benzothiepin-8-carboxamido)benzoesäure.

10. Verfahren gemäss Anspruch 5 zur Herstellung der Verbindungen,
p-(3,4-Dihydro-3,3-dimethyl-2H-1,5-benzodithiepin-7-carboxamido)benzoesäureäthylester,
Aethyl-p-(1,4-benzodioxan-6-carboxamido)benzoat,
p-(1,4-Benzodioxan-6-carboxamido)benzoesäure,
p-(3,4-Dihydro-3,3-dimethyl-2H-1,5-benzodithiepin-7-carboxamido)benzoesäure,
p-(3,4-Dihydro-3,3-dimethyl-2H-1,5-benzodioxepin-7-carboxamido)benzoesäure,
Aethyl p-(2,2-Dimethyl-1,3-benzoxathiol-5-carboxamido)benzoat,
p-(2,2-Dimethyl-1,3-benzoxathiol-5-carboxamido)benzoesäure,
Aethyl p-(2,2-dimethyl-1,3-benzodioxol-5-carboxamido)benzoat,
2-[p-[(E)-3,4-(Isopropylidendioxy)-β-methylstyryl]phenoxyl]-N,N-dimethyläthylamin,
p-(2,2-Dimethyl-1,3-benzodioxol-5-carboxamido)benzoesäure; und
p-(3,4-Dihydro-3,3-dimethyl-2H-1,5-benzodioxepin-7-carboxamido)benzoesäureäthylester.

11. Verfahren gemäss Anspruch 1 zur Herstellung der p-[(E)-2-(3,4-Dihydro-3,3-dimethyl-2H-1,5-benzodithiepin-7-yl)propenyl]benzoesäure und deren nieder-Alkylester.

12. Verfahren gemäss Anspruch 1 zur Herstellung des p-[(E)-2-(3,4-Dihydro-3,3-dimethyl-2H-1,5-benzodithiepin-7-yl)propenyl]benzoesäureäthylesters.

13. Verfahren nach Anspruch 1, dadurch gekennzeichnet, dass man die Umsetzung gemäss den Verfahrensvarianten a), b) und c) in einem organischen Lösungsmittel bei Temperaturen zwischen 0°C und dem Siedepunkt des Reaktionsgemisches bei Atmosphärendruck vornimmt.

14. Verwendung von Verbindung der Formel I,
worin R¹ Wasserstoff, Acyl, nieder-Alkyl oder eine Gruppe -CHO, -CH₂OR¹⁰, -COR⁷, oder OR¹³; R², R³ und R⁴ Wasserstoff, nieder-Alkyl, nieder-Alkoxy oder Halogen; R⁵ und R⁶ Wasserstoff oder nieder-Alkyl; R⁷ Hydroxy, nieder-Alkoxy oder NR⁸R⁹; R⁸ und R⁹ Wasserstoff oder nieder-Alkyl; X >CR¹⁴R¹⁵, -O-, -S-, >SO, >SO₂ oder >NR¹⁸; Y-O-, -S-, >SO, >SO₂ oder >NR¹⁸; R¹⁰ und R¹⁸ Wasserstoff, nieder-Alkyl oder Acyl; M -C(R¹¹)=C(R¹²)-, -CONH-, oder -NH-CO-; R¹¹, R¹², R¹⁴ und R¹⁵ Wasserstoff oder nieder-Alkyl, R¹³ Wasserstoff, nieder-Alkyl, das durch Amino, mono- oder di-Alkylamino, Morpholino, Thiamorpholino oder Piperazino substituiert sein kann, oder nieder-Alkoxycarbonyl; und n 1, 2, 3 oder 4 bedeuten; und wobei "nieder" Gruppen mit 1-6 C-Atomen bezeichnet
und Salze von Verbindungen der Formel I in denen R¹ eine Carboxygruppe darstellt, als Wirkstoffe bei der Herstellung von pharmazeutischen Präparaten zur Behandlung von Neoplasien, Dermatosen und Altershaut.

## Claims (Claims for the following Contracting State(s): AT, BE, CH, DE, FR, GB, IT, LI, LU, NL, SE)

1. Compounds of the general formula 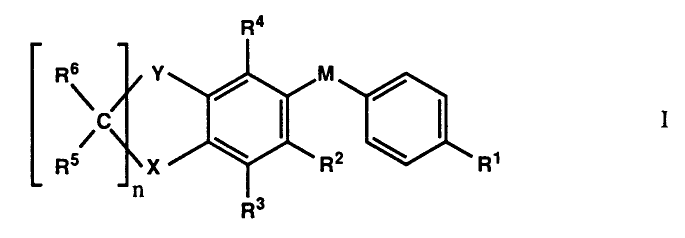 wherein R¹ signifies hydrogen, acyl, lower-alkyl or a group -CHO, -CH₂OR¹⁰, -COR⁷ or OR¹³; R², R³ and R⁴ signify hydrogen, lower-alkyl, lower-alkoxy or halogen; R⁵ and R⁶ signify hydrogen or lower-alkyl; R⁷ signifies hydroxy, lower-alkoxy or NR⁸R⁹; R⁸ and R⁹ signify hydrogen or lower-alkyl; X signifies >CR¹⁴R¹⁵, -O-, -S-, >SO, >SO₂ or >NR¹⁸; Y signifies -O-, -S-, >SO, >SO₂ or >NR¹⁸; R¹⁰ and R¹⁸ signify hydrogen, lower-alkyl or acyl; M signifies -C(R¹¹)=C(R¹²)-, -CONH- or -NH-CO-; R¹¹, R¹², R¹⁴ and R¹⁵ signify hydrogen or lower-alkyl, R¹³ signifies hydrogen, lower-alkyl, which can be substituted by amino, mono- or di-alkylamino, morpholino, thiamorpholino or piperazino, or lower-alkoxycarbonyl; and n signifies 1, 2, 3 or 4; and wherein "lower" denotes groups with 1-6 C atoms,
and salts of compounds of formula I in which R¹ represents a carboxy group for use as medicaments.

2. Compounds in accordance with claim 1 in which R¹³ represents hydrogen or lower-alkyl, which can be substituted by amino, mono- or di-alkylamino, morpholino, thiamorpholino or piperazino, and the remaining symbols have the significance given in claim 1 for use as medicaments.

3. Compounds in accordance with claim 1 or 2 in which M represents -C(R¹¹)=C(R¹²)- for use as medicaments.

4. Compounds in accordance with claim 1 or 2 in which M represents -CONH- for use as medicaments.

5. Compounds in accordance with claim 1-4 in which X and Y are -O-, -S-, >SO, >SO₂ or >NR¹⁸ for use as medicaments.

6. Compounds in accordance with claims 1-5 in which X and Y are -S- for use as medicaments.

7. Compounds of the general formula 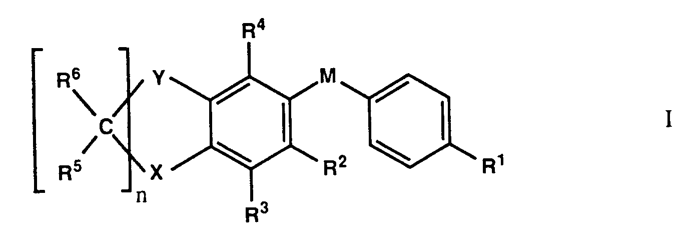 wherein R¹ signifies acyl or a group -CHO, -CH₂OR¹⁰, -COR⁷ or OR¹³; R², R³ and R⁴ signify hydrogen, lower-alkyl, lower-alkoxy or halogen; R⁵ and R⁶ signify hydrogen or lower-alkyl; R⁷ signifies hydroxy, lower-alkoxy or NR⁸R⁹; R⁸ and R⁹ signify hydrogen or lower-alkyl; X signifies >CR¹⁴R¹⁵, -O-, -S-, >SO, >SO₂ or >NR¹⁸; Y signifies -O-, -S-, >SO, >SO₂ or >NR¹⁸; R¹⁰ and R¹⁸ signify hydrogen, lower-alkyl or acyl; M signifies -C(CH₃)=CH-, -CONH- or -NH-CO-; R¹⁴ and R¹⁵ signify hydrogen or lower-alkyl; R¹³ signifies lower-alkyl, which is substituted by amino, mono- or di-alkylamino, morpholino, thiamorpholino or piperazino, or lower-alkoxycarbonyl; and n signifies 1, 2, 3 or 4; and wherein "lower" denotes groups with 1-6 C atoms,
and salts of compounds of formula I in which R¹ represents a carboxy group.

8. Compounds in accordance with claim 7, wherein R¹³ represents lower alkyl, which is substituted by amino, mono- or di-alkylamino, morpholino, thiamorpholino or piperazino, and the remaining symbols have the significance given in claim 1.

9. Compounds in accordance with claim 7 or 8, wherein M represents -C(CH₃)=CH-.

10. Compounds in accordance with claim 7 or 8, wherein M represents -CONH-.

11. Compounds in accordance with claim 7-10, wherein X and Y are -O-, -S-, >SO, >SO₂ or >NR¹⁸ .

12. Compounds in accordance with claim 11, wherein X and Y are -S-.

13. The compounds according to claim 7,
ethyl p-[2-(3,4-dihydro-4,4-dimethyl-2H-1-benzopyran-7-yl)-propenyl]benzoate,
p-[2-(3,4-dihydro-4,4-dimethyl-2H-1-benzopyran-7-yl)propenyl]benzoic acid,
ethyl p-[2-(3,4-dihydro-4,4-dimethyl-2H-1-benzothiopyran-7-yl)propenyl]benzoate,
p-[2-(3,4-dihydro-4,4-dimethyl-2H-1-benzothiopyran-7-yl)propenyl]benzoic acid,
ethyl p-[2-(3',4'-dihydro-4',4'-dimethyl-2'H-1-benzothiopyran-7'-yl)propenyl]benzoate 1',1'-dioxide,
p-[2-(3',4'-dihydro-4',4'-dimethy-2'H-1-benzothiopyran-7'-yl)-propenyl]benzoic acid 1',1'-dioxide,
methyl p-[(E)-2-(2,3,4,5-tetrahydro-5-methyl-1-benzothiepin-8-yl)propenyl]benzoate,
ethyl p-[2-(1,2,3,4-tetrahydro-1,4,4-trimethyl-7-quinolinyl)propenyl]benzoate,
p-[2-(1,2,3,4-tetrahydro-1,4,4-trimethyl-7-quinolinyl)propenyl]benzoic acid,
ethyl p-[(E)-2-(2,3,4,5-tetrahydro-1-benzoxepin-8-yl)propenyl]benzoate,
methyl p-[(E)-2-(2,3,4,5-tetrahydro-3,3-dimethyl-2H-1-benzothiepin-8-yl)propenyl]benzoate,
ethyl p-[(E)-2-(2,3,4,5-tetrahydro-1-benzothiepin-8-yl)propenyl]benzoate,
p-[(E)-2-(2',3',4',5'-tetrahydro-1-benzothiepin-8'-yl)propenyl]benzoic acid 1',1'-dioxide,
p-[(E)-2-(2,3,4,5-tetrahydro-1-benzothiepin-8-yl)propenyl]benzoic acid,
ethyl p-(2,2,4,5-tetrahydro-1-benzothiepin-8-carboxamido)benzoate.

14. The compounds in accordance with claim 11,
ethyl p-[(E)-2-(1,4-benzodioxan-6-yl)propenyl]benzoate,
p-[(E)-2-(1,4-benzodioxan-6-yl)propenyl]benzoic acid,
ethyl p-[(E)-2-(1,4-benzodithian-6-yl)propenyl]benzoate,
p-[(E)-2-(1,4-benzodithian-6-yl)propenyl]benzoic acid,
methyl p-[(E)-2-(2,2-dimethyl-1,3-benzodioxol-5-yl)propenyl]benzoate,
ethyl p-[(E)-2-(3,4-dihydro-3,3-dimethy-2H-1,5-benzodioxepin-7-yl)propenyl]benzoate,
ethyl p-[(E)-2-(3,4-dihydro-3-methyl-2H-1,5-benzodithiepin-7-yl)propenyl]benzoate,
ethyl p-[(E)-2-(3,4-dihydro-2H-1,5-benzodithiepin-7-yl)propenyl]benzoate,
ethyl p-[(E)-2-(3',4'-dihydro-3',3'-dimethyl-2'H-1,5-benzodithiepin-7'-yl)propenyl]benzoate 1',1',5',5'-tetroxide,
p-[(E)-2-(3,4-dihydro-3-methyl-2H-1,5-benzodithiepin-7-yl)propenyl]benzoic acid,
p-[(E)-2-(3,4-dihydro-3,3-dimethyl-2H-1,5-benzodioxepin-7-yl)propenyl]benzoic acid,
methyl p-[(E)-2-(3,4-dihydro-3,3-dimethyl-2H-1,5-benzodithiepin-7-yl)propenyl]benzoate,
ethyl p-[(E)-2-(2,3-dihydro-3,3-dimethyl-1,4-benzoxathiin-7-yl)propenyl]benzoate,
methyl p-[(E)-2-(2,3,4,5-tetrahydro-1,3,3,5-tetramethyl-1H-1,5-benzodiazepin-7-yl)propenyl]benzoate,
4-[2-[p-[(E)-2-(3,4-dihydro-3,3-dimethyl-2H-1,5-benzodithiepin-7-yl)propenyl]phenoxy]ethyl]morpholine,
4-[2-[p-[(E)-2-(3,4-dihydro-3,3-dimethyl-2H-1,5-benzodioxepin-7-yl)propenyl]phenoxy]ethyl]morpholine,
p-[(E)-2-(3,4-dihydro-3,3-dimethyl-2H-1,5-benzodioxepin-7-yl)propenyl]phenyl ethyl carbonate,
methyl p-[(E)-2-(3,4-dihydro-5-methyl-2H-1,5-benzothiazepin-8-yl)propenyl]benzoate,
methyl p-[(E)-2-(3,4-dihydro-3,5-dimethyl-2H-1,5-benzothiazepin-8-yl)propenyl]benzoate,
N,N-dimethyl-2-[p-[(E)-2-(3,4-dihydro-3,3-dimethyl-2H-1,5-benzodithiepin-7-yl)propenyl]phenoxy]ethylamine,
p-[(E)-2-(2,3-dihydro-3,3-dimethyl-1,4-benzoxathiin-7-yl)propenyl]benzoic acid,
p-[(E)-2-(3,4-dihydro-2H-1,5-benzodithiepin-7-yl)propenyl]benzoic acid,
p-[(E)-2-(3,4-dihydro-5-methyl-2H-1,5-benzothiazepin-8-yl)propenyl]benzoic acid,
p-[(E)-2-(3,4-dihydro-3,5-dimethyl-2H-1,5-benzothiazepin-8-yl)propenyl]benzoic acid,
methyl p-[(E)-2-(2,2-dimethyl-1,3-benzodithiol-5-yl)propenyl]benzoate,
4-[2-[p-[(E)-2-(2,2-dimethyl-1,3-benzodithiol-5-yl)propenyl]phenoxy]ethyl]morpholine,
ethyl p-[(E)-2-(2,2-dimethyl-1,3-benzodithiol-5-yl)propenyl]phenyl carbonate,
p-[(E)-2-(2,2-dimethyl-1,3-benzodithiol-5-yl)propenyl]benzoic acid,
methyl p-[(E)-2-(1,2,3,4-tetrahydro-1,4-dimethyl-6-quinoxalinyl)propenyl]benzoate,
p-[(E)-2-(1,2,3,4-tetrahydro-1,4-dimethyl-6-quinoxalinyl)propenyl]benzoic acid,
methyl p-[(E)-2-(2-methyl-1,3-benzodithiol-5-yl)propenyl]benzoate,
p-[(E)-2-(2-methyl-1,3-benzodithiol-5-yl)propenyl]benzoic acid,
methyl p-[(E)-2-(1,2,3,4-tetrahydro-1,2,3,4-tetramethylquinoxalin-6-yl)propenyl]benzoate,
p-[(E)-2-(2,2-dimethyl-1,3-benzodioxol-5-yl)propenyl]benzoic acid,
p-(2,2-dimethyl-1,3-benzodioxol-5-carboxamido)benzoic acid.

15. The compounds in accordance with claim 10,
ethyl p-(2,3,4,5-tetrahydro-5-methyl-1-benzothiepin-8-carboxamido)benzoate,
p-(2,3,4,5-tetrahydro-1-benzothiepine-8-carboxamido)benzoic acid,
ethyl p-(3,4-dihydro-4,4-dimethyl-2H-1-benzopyran-7-carboxamido)benzoate,
p-(3,4-dihydro-4,4-dimethyl-2H-1-benzopyran-7-carboxamido)benzoic acid,
ethyl p-(3,4-dihydro-4,4-dimethyl-2H-1-benzothiopyran-7-carboxamido)benzoate,
p-(3,4-dihydro-4,4-dimethyl-2H-1-benzothiopyran-7-carboxamido)benzoic acid,
ethyl p-(3,4-dihydro-4,4-dimethyl-2H-1-benzothiopyran-7-carboxamido)benzoate 1,1-dioxide,
p-(3,4-dihydro-4,4-dimethyl-2H-1-benzothiopyran-7-carboxamido)benzoic acid 1,1-dioxide,
ethyl p-(1,2,3,4-tetrahydro-1,4,4-trimethyl-7-quinolinecarboxamido)benzoate,
p-(1,2,3,4-tetrahydro-1,4,4-trimethyl-7-quinolinecarboxamido)benzoic acid,
ethyl p-(1-decyl-1,2,3,4-tetrahydro-4,4-dimethyl-7-quinolinecarboxamido)benzoate,
p-(1-decyl-1,2,3,4-tetrahydro-4,4-dimethyl-7-quinolinecarboxamido)benzoic acid,
p-(2,3,4,5-tetrahydro-5-methyl-1-benzothiepine-8-carboxamido)benzoic acid.

16. The compounds in accordance with claim 11,
ethyl p-(3,4-dihydro-3,3-dimethyl-2H-1,5-benzodithiepine-7-carboxamido)benzoate,
ethyl p-(1,4-benzodioxane-6-carboxamido)benzoate,
p-(1,4-benzodioxane-6-carboxamido)benzoic acid,
p-(3,4-dihydro-3,3-dimethyl-2H-1,5-benzodithiepine-7-carboxamido)benzoic acid,
p-(3,4-dihydro-3,3-dimethyl-2H-1,5-benzodioxepine-7-carboxamido)benzoic acid,
ethyl p-(2,2-dimethyl-1,3-benzoxathiol-5-carboxamido)benzoate,
p-(2,2-dimethyl-1,3-benzoxathiol-5-carboxamido)benzoic acid,
ethyl p-(2,2-dimethyl-1,3-benzodioxol-5-carboxamido)benzoate,
ethyl p-(2,2-dimethyl-1,3-benzodioxol-6-carboxamido)benzoate,
2-[p-[(E)-3,4-(isopropylidenedioxy)-β-methylstyryl]phenoxy]-N,N-dimethylethylamine,
p-(2,2-dimethyl-1,3-benzodioxol-5-carboxamido)benzoic acid and
ethyl p-(3,4-dihydro-3,3-dimethyl-2H-1,5-benzodioxepine-7-carboxamido)benzoate.

17. The compound in accordance with claim 7,
p-[(E)-2-(3,4-dihydro-3,3-dimethyl-2H-1,5-benzodithiepin-7-yl)propenyl]benzoic acid and its lower-alkyl esters.

18. The compound in accordance with claim 7,
ethyl p-[(E)-2-(3,4-dihydro-3,3-dimethyl-2H-1,5-benzodithiepin-7-yl)propenyl]benzoate.

19. A process for the manufacture of the compounds of claim 7, characterized by
a) reacting a compound of the general formula 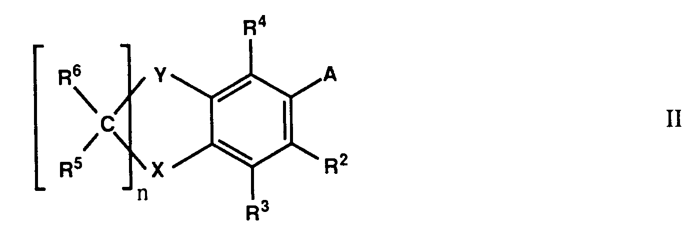 with a compound of the general formula 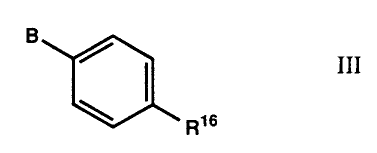 in which either
A represents the residue -CH(CH₃)P⁺(Q)₃Z⁻ or -CH(CH₃)-P(O)(OAlk)₂ and B represents a formyl residue; or
A represents a residue CH₃CO and B represents the residue -CH₂P⁺(Q)₃Z⁻ or -CH₂-P-(O)(OAlk)₂; Q represents aryl; Z⁻ represents the anion of an organic or inorganic acid; Alk represents a lower alkyl group; and R¹⁶ represents a residue R¹ with the exception of the formyl, carboxyl and hydroxymethyl group; or
b) reacting a compound of the general formula 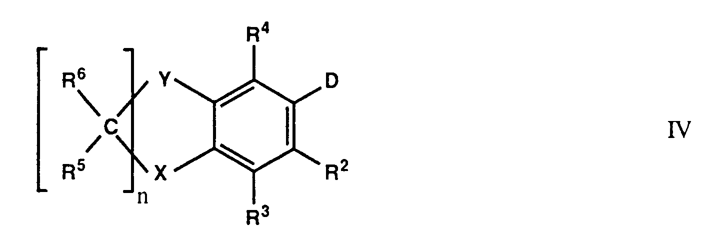 with a compound of the general formula 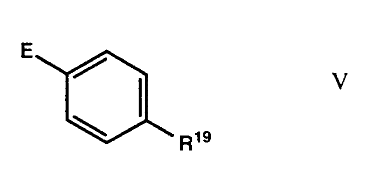 in which either D signifies a carboxyl group or a reactive derivative thereof and E signifies an amino group or D signifies an amino group and E signifies a carboxyl group or a reactive derivative thereof and R¹⁹ signifies a residue R¹ with the exception of the carboxyl and hydroxymethyl group;
or
c) reacting a compound of the general formula 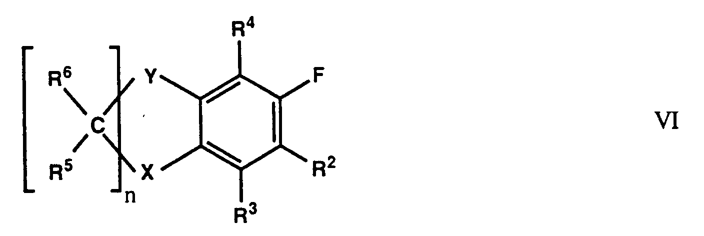 with a compound of the general formula 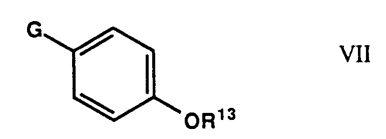 in which either F represents a residue -CH(CH₃)MgHal and G represents a formyl residue; or F represents a formyl residue and G represents a residue -CH₂-MgHal; Hal represents halogen; and wherein "lower" denotes groups with 1-6 C atoms, and the remaining symbols have the significance given earlier in formulae II-VII;
and dehydrating the reaction product;
whereupon, if desired, in the resulting reaction product of formula I the residue R¹ is functionally modified and/or the sulphur atom in a compound I in which X and/or Y is -S- is oxidized to a sulphoxyl or sulphonyl group.

20. Pharmaceutical preparations containing a compound of formula I in accordance with claim 1 and pharmaceutically acceptable salts of such compounds which contain a carboxyl group.

21. Pharmaceutical preparations in unit dosage form containing 5 to 500 mg of a compound of formula I in accordance with claim 1 and pharmaceutically acceptable salts of such compounds which contain a carboxyl group.

22. The use of a compound of formula I in accordance with claim 1 and pharmaceutically acceptable salts of such compounds which contain a carboxyl group as active ingredients in the manufacture of pharmaceutical preparations for the treatment of neoplasms, dermatoses and aged skin.

## Claims (Claims for the following Contracting State(s): ES, GR)

1. A process for the manufacture of compounds of the general formula 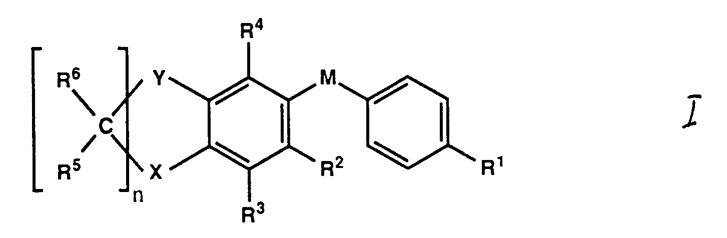 wherein R¹ signifies acyl or a group -CHO, -CH₂OR¹⁰, -COR⁷ or OR¹³; R², R³ and R⁴ signify hydrogen, lower-alkyl, lower-alkoxy or halogen; R⁵ and R⁶ signify hydrogen or lower-alkyl; R⁷ signifies hydroxy, lower-alkoxy or NR⁸R⁹; R⁸ and R⁹ signify hydrogen or lower-alkyl; X signifies >CR¹⁴R¹⁵, -O-, -S-, >SO, >SO₂ or >NR¹⁸; Y signifies -O-, -S-, >SO, >SO₂ or >NR¹⁸; R¹⁰ and R¹⁸ signify hydrogen, lower-alkyl or acyl; M signifies -C(CH₃)=CH-, -CONH- or -NH-CO-; R¹⁴ and R¹⁵ signify hydrogen or lower-alkyl; R¹³ signifies lower-alkyl, which is substituted by amino, mono- or di-alkylamino, morpholino, thiamorpholino or piperazino, or lower-alkoxycarbonyl; and n signifies 1, 2, 3 or 4; and wherein "lower" denotes groups with 1-6 C atoms,
and salts of compounds of formula I in which R¹ represents a carboxy group, characterized by
a) reacting a compound of the general formula 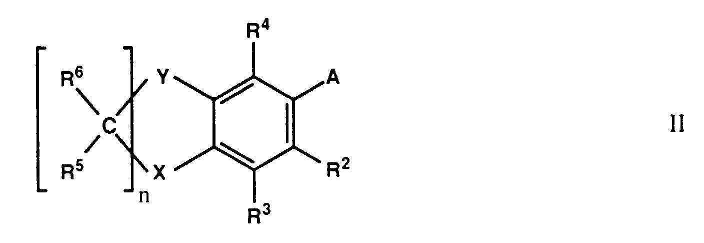 with a compound of the general formula 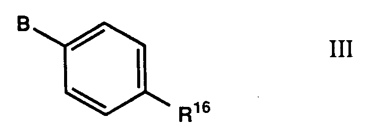 in which either
A represents the residue -CH(CH₃)P⁺(Q)₃Z⁻ or -CH(CH₃)-P(O)(OAlk)₂ and B represents a formyl residue; or
A represents a residue CH₃CO and B represents the residue -CH₂P⁺(Q)₃Z⁻ or -CH₂-P-(O)(OAlk)₂; Q represents aryl; Z⁻ represents the anion of an organic or inorganic acid; Alk represents a lower alkyl group; and R¹⁶ represents a residue R¹ with the exception of the formyl, carboxyl and hydroxymethyl group; or
b) reacting a compound of the general formula 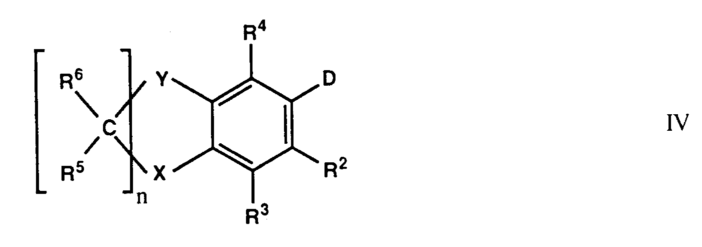 with a compound of the general formula 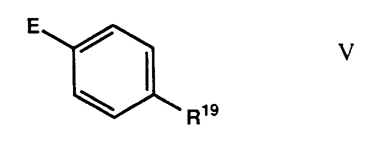 in which either D signifies a carboxyl group or a reactive derivative thereof and E signifies an amino group or D signifies an amino group and E signifies a carboxyl group or a reactive derivative thereof and R¹⁹ signifies a residue R¹ with the exception of the carboxyl and hydroxymethyl group;
or
c) reacting a compound of the general formula 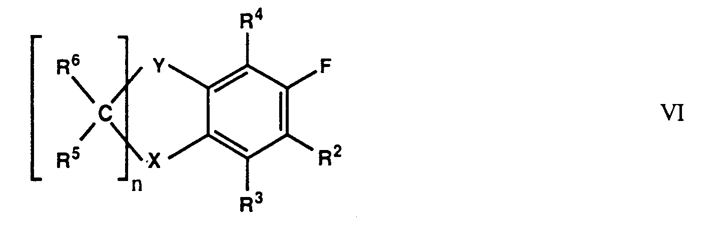 with a compound of the general formula 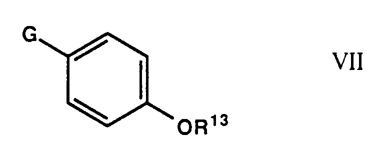 in which either F represents a residue -CH(CH₃)MgHal and G represents a formyl residue; or F represents a formyl residue and G represents a residue -CH₂-MgHal; Hal represents halogen; and wherein "lower" denotes groups with 1-6 C atoms, and the remaining symbols have the significance given earlier in formulae II-VII;
and dehydrating the reaction product;
whereupon, if desired, in the resulting reaction product of formula I the residue R¹ is functionally modified and/or the sulphur atom in a compound I in which X and/or Y is -S- is oxidized to a sulphoxyl or sulphonyl group.

2. A process in accordance with claim 1 for the manufacture of compounds of formula I in which R¹³ represents lower-alkyl, which is substituted by amino, mono- or di-alkylamino, morpholino, thiamorpholino or piperazino, and the remaining symbols have the significance given in claim 1.

3. A process in accordance with claim 1 or 2 for the manufacture of compounds of formula I in which M represents -C(CH₃)=CH-.

4. A process in accordance with claim 1 or 2 for the manufacture of compounds of formula I in which M represents -CONH-.

5. A process in accordance with claim 1-4 for the manufacture of compounds of formula I in which X and Y are -O-, -S-, >SO, >SO₂ or >NR¹⁸.

6. A process in accordance with claims 1-5 for the manufacture of compounds of formula I in which X and Y are -S-.

7. A process in accordance with claim 5 for the manufacture of the compounds
ethyl p-[2-(3,4-dihydro-4,4-dimethyl-2H-1-benzopyran-7-yl)-propenyl]benzoate,
p-[2-(3,4-dihydro-4,4-dimethyl-2H-1-benzopyran-7-yl)propenyl]benzoic acid,
ethyl p-[2-(3,4-dihydro-4,4-dimethyl-2H-1-benzothiopyran-7-yl)propenyl]benzoate,
p-[2-(3,4-dihydro-4,4-dimethyl-2H-1-benzothiopyran-7-yl)propenyl]benzoic acid,
ethyl p-[2-(3',4'-dihydro-4',4'-dimethyl-2'H-1-benzothiopyran-7'-yl)propenyl]benzoate 1',1'-dioxide,
p-[2-(3',4'-dihydro-4',4'-dimethyl-2'H-1-benzothiopyran-7'-yl)-propenyl]benzoic acid 1',1'-dioxide,
methyl p-[(E)-2-(2,3,4,5-tetrahydro-5-methyl-1-benzothiepin-8-yl)propenyl]benzoate,
ethyl p-[2-(1,2,3,4-tetrahydro-1,4,4-trimethyl-7-quinolinyl)propenyl]benzoate,
p-[2-(1,2,3,4-tetrahydro-1,4,4-trimethyl-7-quinolinyl)propenyl]benzoic acid,
ethyl p-[(E)-2-(2,3,4,5-tetrahydro-1-benzoxepin-8-yl)propenyl]benzoate,
methyl p-[(E)-2-(2,3,4,5-tetrahydro-3,3-dimethyl-2H-1-benzothiepin-8-yl)propenyl]benzoate,
ethyl p-[(E)-2-(2,3,4,5-tetrahydro-1-benzothiepin-8-yl)propenyl)benzoate,
p-[(E)-2-(2',3',4',5'-tetrahydro-1-benzothiepin-8'-yl)propenyl]benzoic acid 1',1'-dioxide,
p-[(E)-2-(2,3,4,5-tetrahydro-1-benzothiepin-8-yl)propenyl]benzoic acid,
ethyl p-(2,3,4,5-tetrahydro-1-benzothiepin-8-carboxamido)benzoate.

8. A process in accordance with claim 5 for the manufacture of the compounds
ethyl p-[(E)-2-(1,4-benzodioxan-6-yl)propenyl]benzoate,
p-[(E)-2-(1,4-benzodioxan-6-yl)propenyl]benzoic acid,
ethyl p-[(E)-2-(1,4-benzodithian-6-yl)propenyl]benzoate,
p-[(E)-2-(1,4-benzodithian-6-yl)propenyl)benzoic acid,
methyl p-[(E)-2-(2,2-dimethyl-1,3-benzodioxol-5-yl)propenyl]benzoate,
ethyl p-[(E)-2-(3,4-dihydro-3,3-dimethyl-2H-1,5-benzodioxepin-7-yl)propenyl]benzoate,
ethyl p-[(E)-2-(3,4-dihydro-3-methyl-2H-1,5-benzodithiepin-7-yl)propenyl]benzoate,
ethyl p-[(E)-2-(3,4-dihydro-2H-1,5-benzodithiepin-7-yl)propenyl]benzoate,
ethyl p-[(E)-2-(3',4'-dihydro-3',3'-dimethyl-2'H-1,5-benzodithiepin-7'-yl)propenyl]benzoate 1',1',5',5'-tetroxide,
p-[(E)-2-(3,4-dihydro-3-methyl-2H-1,5-benzodithiepin-7-yl)propenyl]benzoic acid,
p-[(E)-2-(3,4-dihydro-3,3-dimethyl-2H-1,5-benzodioxepin-7-yl)propenyl]benzoic acid,
methyl p-[(E)-2-(3,4-dihydro-3,3-dimethyl-2H-1,5-benzodithiepin-7-yl)propenyl]benzoate,
ethyl p-[(E)-2-(2,3-dihydro-3,3-dimethyl-1,4-benzoxathiin-7-yl)propenyl]benzoate,
methyl p-[(E)-2-(2,3,4,5-tetrahydro-1,3,3,5-tetramethyl-1H-1,5-benzodiazepin-7-yl)propenyl]benzoate,
4-[2-[p-[(E)-2-(3,4-dihydro-3,3-dimethyl-2H-1,5-benzodithiepin-7-yl)propenyl]phenoxy]ethyl]morpholine,
4-[2-[p-[(E)-2-(3,4-dihydro-3,3-dimethyl-2H-1,5-benzodioxepin-7-yl)propenyl]phenoxy]ethyl]morpholine,
p-[(E)-2-(3,4-dihydro-3,3-dimethyl-2H-1,5-benzodioxepin-7-yl)propenyl]phenyl ethyl carbonate,
methyl p-[(E)-2-(3,4-dihydro-5-methyl-2H-1,5-benzothiazepin-8-yl)propenyl]benzoate,
methyl p-[(E)-2-(3,4-dihydro-3,5-dimethyl-2H-1,5-benzothiazepin-8-yl)propenyl]benzoate,
N,N-dimethyl-2-[p-[(E)-2-(3,4-dihydro-3,3-dimethyl-2H-1,5-benzodithiepin-7-yl)propenyl]phenoxy]ethylamine,
p-[(E)-2-(2,3-dihydro-3,3-dimethyl-1,4-benzoxathiin-7-yl)propenyl]benzoic acid,
p-[(E)-2-(3,4-dihydro-2H-1,5-benzodithiepin-7-yl)propenyl]benzoic acid,
p-[(E)-2-(3,4-dihydro-5-methyl-2H-1,5-benzothiazepin-8-yl)propenyl]benzoic acid,
p-[(E)-2-(3,4-dihydro-3,5-dimethyl-2H-1,5-benzothiazepin-8-yl)propenyl]benzoic acid,
methyl p-[(E)-2-(2,2-dimethyl-1,3-benzodithiol-5-yl)propenyl]benzoate,
4-[2-[p-[(E)-2-(2,2-dimethyl-1,3-benzodithiol-5-yl)propenyl]phenoxy]ethyl]morpholine,
ethyl p-[(E)-2-(2,2-dimethyl-1,3-benzodithiol-5-yl)propenyl]phenyl carbonate,
p-[(E)-2-(2,2-dimethyl-1,3-benzodithiol-5-yl)propenyl]benzoic acid,
methyl p-[(E)-2-(1,2,3,4-tetrahydro-1,4-dimethyl-6-quinoxalinyl)propenyl]benzoate,
p-[(E)-2-(1,2,3,4-tetrahydro-1,4-dimethyl-6-quinoxalinyl)propenyl]benzoic acid,
methyl p-[(E)-2-(2-methyl-1,3-benzodithiol-5-yl)propenyl]benzoate,
p-[(E)-2-(2-methyl-1,3-benzodithiol-5-yl)propenyl]benzoic acid,
methyl p-[(E)-2-(1,2,3,4-tetrahydro-1,2,3,4-tetramethylquinoxalin-6-yl)propenyl]benzoate,
p-[(E)-2-(2,2-dimethyl-1,3-benzodioxol-5-yl)propenyl]benzoic acid,
p-(2,2-dimethyl-1,3-benzodioxol-5-carboxamido)benzoic acid.

9. A process in accordance with claim 4 for the manufacture of the compounds
ethyl p-(2,3,4,5-tetrahydro-5-methyl-1-benzothiepin-8-carboxamido)benzoate,
p-(2,3,4,5-tetrahydro-1-benzothiepine-8-carboxamido)benzoic acid,
ethyl p-(3,4-dihydro-4,4-dimethyl-2H-1-benzopyran-7-carboxamido)benzoate,
p-(3,4-dihydro-4,4-dimethyl-2H-1-benzopyran-7-carboxamido)benzoic acid,
ethyl p-(3,4-dihydro-4,4-dimethyl-2H-1-benzothiopyran-7-carboxamido)benzoate,
p-(3,4-dihydro-4,4-dimethyl-2H-1-benzothiopyran-7-carboxamido)benzoic acid,
ethyl p-(3,4-dihydro-4,4-dimethyl-2H-1-benzothiopyran-7-carboxamido)benzoate 1,1-dioxide,
p-(3,4-dihydro-4,4-dimethyl-2H-1-benzothiopyran-7-carboxamido)benzoic acid 1,1-dioxide,
ethyl p-(1,2,3,4-tetrahydro-1,4,4-trimethyl-7-quinolinecarboxamido)benzoate,
p-(1,2,3,4-tetrahydro-1,4,4-trimethyl-7-quinolinecarboxamido)benzoic acid,
ethyl p-(1-decyl-1,2,3,4-tetrahydro-4,4-dimethyl-7-quinolinecarboxamido)benzoate,
p-(1-decyl-1,2,3,4-tetrahydro-4,4-dimethyl-7-quinolinecarboxamido)benzoic acid,
p-(2,3,4,5-tetrahydro-5-methyl-1-benzothiepine-8-carboxamido)benzoic acid,

10. A process in accordance with claim 5 for the manufacture of the compounds
ethyl p-(3,4-dihydro-3,3-dimethyl-2H-1,5-benzodithiepine-7-carboxamido)benzoate,
ethyl p-(1,4-benzodioxane-6-carboxamido)benzoate,
p-(1,4-benzodioxane-6-carboxamido)benzoic acid,
p-(3,4-dihydro-3,3-dimethyl-2H-1,5-benzodithiepine-7-carboxamido)benzoic acid,
p-(3,4-dihydro-3,3-dimethyl-2H-1,5-benzodioxepine-7-carboxamido)benzoic acid,
ethyl p-(2,2-dimethyl-1,3-benzoxathiol-5-carboxamido)benzoate,
p-(2,2-dimethyl-1,3-benzoxathiol-5-carboxamido)benzoic acid,
ethyl p-(2,2-dimethyl-1,3-benzodioxol-5-carboxamido)benzoate,
2-[p-[(E)-3,4-(isopropylidenedioxy)-β-methylstyryl]phenoxy]-N,N-dimethylethylamine,
p-(2,2-dimethyl-1,3-benzodioxol-5-carboxamido)benzoic acid and
ethyl p-(3,4-dihydro-3,3-dimethyl-2H-1,5-benzodioxepine-7-carboxamido)benzoate.

11. A process in accordance with claim 1 for the manufacture of p-[(E)-2-(3,4-dihydro-3,3-dimethyl-2H-1,5-benzodithiepin-7-yl)propenyl]-benzoic acid and its lower-alkyl esters.

12. A process in accordance with claim 1 for the manufacture of ethyl p-[(E)-2-(3,4-dihydro-3,3-dimethyl-2H-1,5-benzodithiepin-7-yl)propenyl]-benzoate.

13. A process according to claim 1, characterized in that the reaction in accordance with process variants a), b) and c) is performed in an organic solvent at temperatures between 0°C and the boiling point of the reaction mixture at atmospheric pressure.

14. Use of compounds of formula I in which R¹ signifies hydrogen, acyl, lower-alkyl or a group -CHO, -CH₂OR¹⁰, -COR⁷ or OR¹³; R², R³ and R⁴ signify hydrogen, lower-alkyl, lower-alkoxy or halogen; R⁵ and R⁶ signify hydrogen or lower-alkyl; R⁷ signifies hydroxy, lower-alkoxy or NR⁸R⁹; R⁸ and R⁹ signify hydrogen or lower-alkyl; X signifies >CR¹⁴R¹⁵, -O-, -S-, >SO, >SO₂ or >NR¹⁸; Y signifies -O-, -S-, >SO, >SO₂ or >NR¹⁸; R¹⁰ and R¹⁸ signify hydrogen, lower-alkyl or acyl; M signifies -C(R¹¹)=C(R¹²)-, -CONH- or -NH-CO-; R¹¹, R¹², R¹⁴ and R¹⁵ signify hydrogen or lower-alkyl; R¹³ signifies hydrogen, lower-alkyl, which can be substituted by amino, mono- or di-alkylamino, morpholino, thiamorpholino or piperazino, or lower-alkoxycarbonyl; and n signifies 1, 2, 3 or 4; and wherein "lower" denotes groups with 1-6 C atoms, and salts of compounds of formula I in which R¹ represents a carboxy group as active ingredients in the manufacture of pharmaceutical preparations for the treatment of neoplasms, dermatoses and aged skin.

## Revendications (Revendications pour l'(les) Etat(s) contractant(s) suivant(s): AT, BE, CH, DE, FR, GB, IT, LI, LU, NL, SE)

1. Composés de formule générale 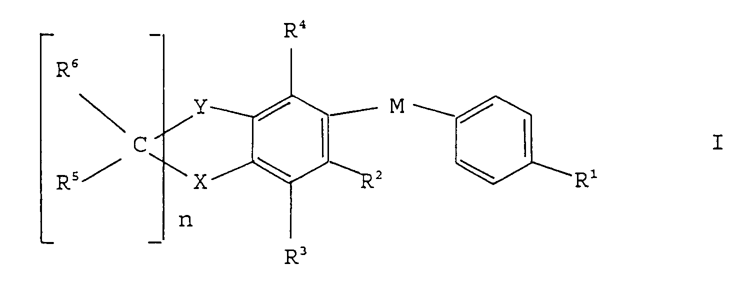 où R¹ représente l'hydrogène, un acyle, un alkyle inférieur ou un groupe -CHO, -CH₂OR¹⁰, -COR⁷ ou OR¹³ ; R², R³ et R⁴ représentent l'hydrogène, un alkyle inférieur, un alcoxy inférieur ou un halogène ; R⁵ et R⁶ représentent l'hydrogène ou un alkyle inférieur ; R⁷ représente l'hydroxy, un alcoxy inférieur ou NR⁸R⁹ ; R⁸ et R⁹ représentent l'hydrogène ou un alkyle inférieur ; X représente >CR¹⁴R¹⁵, -O-, -S-, >SO, >SO₂ ou >NR¹⁸ ; Y représente -O-, -S-, >SO, >SO₂ ou >NR¹⁸ ; R¹⁰ et R¹⁸ représentent l'hydrogène, un alkyle inférieur ou un acyle ; M représente -C(R¹¹)=C(R¹²)-, -CONH- ou -NH-CO- ; R¹¹, R¹², R¹⁴ et R¹⁵ représentent l'hydrogène ou un alkyle inférieur, R¹³ l'hydrogène, un alkyle inférieur pouvant être substitué par un amino, un mono- ou dialkylamino, un morpholino, un thiamorpholino ou un pipérazino ou un alcoxy inférieur carbonyle ; et n est égal à 1, 2, 3 ou 4, le terme "inférieur" désignant des groupes comportant 1 à 6 atomes de C,
et les sels des composés de formule I dans lesquels R¹ représente un groupe carboxylique, destinés à être utilisés comme médicament.

2. Composés selon la revendication 1, où R¹³ représente l'hydrogène ou un alkyle inférieur pouvant être substitué par un amino, un mono- ou dialkylamino un morpholino un thiamorpholino ou un pipérazino et les autres symboles ont la signification donnée dans la revendication 1, destinés à être utilisés comme médicament.

3. Composés selon la revendication 1 ou 2, où M représente C(R¹¹)=C(R¹²)-, destinés à être utilisés comme médicament.

4. Composés selon la revendication 1 ou 2, où M représente -CONH-, destinés à être utilisés comme médicament.

5. Composés selon les revendications 1 à 4, où X et Y sont -O-, -S-, >SO, >SO₂ ou >NR¹⁸, destinés à être utilisés comme médicament.

6. Composés selon les revendications 1 à 5, où X et Y sont -S-, destinés à être utilisés comme médicament.

7. Composé de formule générale 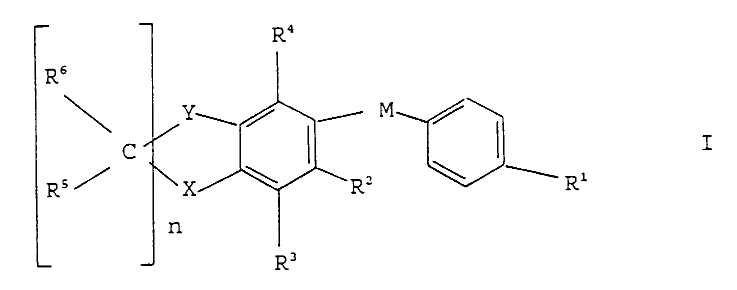 où R¹ représente un acyle ou un groupe -CHO, -CH₂OR¹⁰, -COR⁷ ou OR¹³ ; R², R³ et R⁴ représentent l'hydrogène, un alkyle inférieur, un alcoxy inférieur ou un halogène ; R⁵ et R⁶ représentent l'hydrogène ou un alkyle inférieur ; R⁷ représente l'hydroxy, un alcoxy inférieur ou NR⁸R⁹ ; R⁸ et R⁹ représentent l'hydrogène ou un alkyle inférieur ; X représente >CR¹⁴R¹⁵, -O-, -S-, >SO, >SO₂ ou NR¹⁸ ; Y représente -O-, -S-, >SO, >SO₂ ou >NR¹⁸; R¹⁰ et R¹⁸ représentent l'hydrogène, un alkyle inférieur ou un acyle ; M représente -C(CH₃)=CH-, -CONH-ou -NHCO- ; R¹⁴ et R¹⁵ représentent l'hydrogène ou un alkyle inférieur, R¹³ représente un alkyle inférieur substitué par un amino, un mono- ou dialkylamino, un morpholino, un thiamorpholino ou un pipérazino ou un alcoxy inférieur carboxyle ; et n est égal à 1, 2, 3 ou 4, le terme "inférieur" désignant des groupes comportant 1 à 6 atomes de C,
et les sels des composés de formule I dans lesquels R¹ représente un groupe carboxylique.

8. Composés selon la revendication 7 où R¹³ représente un alkyle inférieur substitué par un amino, un mono- ou dialkylamino, un morpholino, un thiamorpholino ou un pipérazino et les autres symboles ont les significations données dans la revendication 1.

9. Composés selon la revendication 7 ou 8, où M représente -C(CH₃)=CH-.

10. Composés selon la revendication 7 ou 8, où M représente -CONH-.

11. Composés selon les revendications 7 à 10, où X et Y sont -O-, -S-, >SO, >SO₂ ou >NR¹⁸.

12. Composés selon la revendication 11, où X et Y sont -S-.

13. Les composés selon la revendication 7,
l'ester éthylique de l'acide p-[2-(3,4dihydro-4,4-diméthyl-2H-1-benzopyran-7-yl)propényl] benzoïque,
l'acide p-[2-(3,4-dihydro-4,4-diméthyl-2H-1-benzopyran-7-yl)propényl] benzoïque,
le p-[2-(3,4-dihydro-4,4-diméthyl-2H-1-benzothiopyran-7-yl)propényl] benzoate d'éthyle,
l'acide p-[2-(3,4-dihydro-4,4-diméthyl-2H-1-benzothiopyran-7-yl)propényl] benzoïque,
le 1',1'-dioxyde p-[2-(3',4'-dihydro-4'-dihydro-4',4'-diméthyl-2'H-1-benzothiopyran-7'-yl)propényl] benzoate d'éthyle,
le 1',1'-dioxyde acide p-[2-(3',4'-dihydro-4',4'-diméthyl-2'H-1-benzothiopyran-7'-yl)propényl] benzoïque,
le p-[(E)-2-(2,3,4,5-tétrahydro-5-méthyl-1-benzothiépin-8-yl)propényl] benzoate de méthyle,
le p-[2-(1,2,3,4-tétrahydro-1,4,4-triméthyl-7-quinoléinyl)propényl] benzoate d'éthyle,
l'acide p-[2-(1,2,3,4-tétrahydro-1,4,4-triméthyl-7-quinoléinyl)propényl] benzoïque,
l'ester éthylique de l'acide p-[(E)-2-(2,3,4,5-tétrahydro-1-benzoxépin-8-yl)propényl] benzoïque,
l'ester méthylique de l'acide p-[(E)-2-(2,3,4,5-tétrahydro-3,3-diméthyl-2H-1-benzothiépin-8-yl)propényl]benzoïque,
l'ester éthylique de l'acide p-[(E)-2-(2,3,4,5-tétrahydro-1-benzothiépin-8-yl)propényl] benzoïque,
le 1'1'-dioxyde acide p-[(E)-2-(2',3',4',5'-tétrahydro-1-benzothiépin-8'-yl)propényl] benzoïque,
l'acide p-[(E)-2-(2,3,4,5-tétrahydro-1-benzothiépin-8-yl)propényl] benzoïque,
l'ester éthylique de l'acide p-(2,3,4,5-tétrahydro-1-benzothiépine-8-carboxamido)benzoïque.

14. Les composés selon la revendication 11,
le p-[(E)-2-(1,4-benzodioxan-6-yl)propényl] benzoate d'éthyle,
l'acide p-[(E)-2-(1,4-benzodioxan-6-yl)propényl] benzoïque,
le p-[(E)-2-(1,4-benzodithian-6-yl)propényl] benzoate d'éthyle,
l'acide p-[(E)-2-(1,4-benzodithian-6-yl)propényl] benzoïque,
l'ester méthylique de l'acide p-[(E)-2-(2,2-diméthyl-1,3-benzodioxol-5-yl)propényl] benzoïque,
l'ester éthylique de l'acide p-[(E)-2-(3,4-dihydro-3,3-diméthyl-2H-1,5-benzodioxépin-7-yl)propényl] benzoïque,
l'ester éthylique de l'acide p-[(E)-2-(3,4-dihydro-3-méthyl-2H-1,5-benzodithiépin-7-yl)propényl] benzoïque,
l'ester éthylique de l'acide p-[(E)-2-(3,4-dihydro-2H-1,5-benzodithiépin-7-yl)propényl] benzoïque,
le 1',1',5',5'-tétroxyde ester éthylique de l'acide p-[(E)-2-(3',4'-dihydro-3',3'-diméthyl-2'H-1,5-benzodithiépin-7'-yl)propényl]benzoïque,
l'acide p-[(E)-2-(3,4-dihydro-3-méthyl-2H-1,5-benzodithiépin-7-yl)propényl]benzoïque,
l'acide p-[(E)-2-(3,4-dihydro-3,3-diméthyl-2H-1,5-benzodioxépin-7-yl)propényl] benzoïque,
l'ester méthylique de l'acide p-[(E)-2-(3,4-dihydro-3,3-diméthyl-2H-1,5-benzodithiépin-7-yl)propényl] benzoïque,
l'ester éthylique de l'acide p-[(E)-2-(2,3-dihydro-3,3-diméthyl-1,4-benzoxathiin-7-yl)propényl] benzoïque,
l'ester méthylique de l'acide p-[(E)-2-(2,3,4,5-tétrahydro-1,3,3,5-tétraméthyl-1H-1,5-benzodiazépin-7-yl)propényl] benzoïque,
la 4-[2-[p-[(E)-2-(3,4-dihydro-3,3-diméthyl-2H-1,5-benzodithiépin-7-yl)propényl] phénoxy] éthyl] morpholine,
la 4-[2-[p-[(E)-2-(3,4-dihydro-3,3-diméthyl-2H-1,5-benzodioxépin-7-yl)propényl] phénoxy] éthyl] morpholine,
le p-[(E)-2-(3,4-dihydro-3,3-diméthyl-2H-1,5-benzodioxépin-7-yl)propényl] phényléthylcarbonate,
l'ester méthylique de l'acide p-[(E)-2-(3,4-dihydro-5-méthyl-2H-1,5-benzothiazépin-8-yl)propényl] benzoïque,
l'ester méthylique de l'acide p-[(E)-2-(3,4-dihydro-3,5-diméthyl-2H-1,5-benzothiazépin-8-yl)propényl] benzoïque,
la N,N-diméthyl-2-[p-[(E)-2-(3,4-dihydro-3,3-diméthyl-2H-1,5-benzodithiépin-7-yl)propényl] phénoxy] éthylamine,
l'acide p-[(E)-2-(2,3-dihydro-3,3-diméthyl-1,4-benzoxathiin-7-yl)propényl] benzoïque,
l'acide p-[(E)-2-(3,4-dihydro-2H-1,5-benzodithiépin-7-yl)propényl] benzoïque,
l'acide p-[(E)-2-(3,4-dihydro-5-méthyl-2H-1,5-benzothiazépin-8-yl)propényl] benzoïque,
l'acide p-[(E)-2-(3,4-dihydro-3,5-diméthyl-2H-1,5-benzothiazépin-8-yl)propényl] benzoïque,
le p-[(E)-2-(2,2-diméthyl-1,3-benzodithiol-5-yl)propényl] benzoate de méthyle,
la 4-[2-[p-[(E)-2-(2,2-diméthyl-1,3-benzodithiol-5-yl)propényl] phénoxy] éthyl] morpholine,
le p-[(E)-2-(2,2-diméthyl-1,3-benzodithiol-5-yl)propényl] éthylphénylcarbonate,
l'acide p-[(E)-2-(2,2-diméthyl-1,3-benzodithiol-5-yl)propényl] benzoïque,
le p-[(E)-2-(1,2,3,4-tétrahydro-1,4-diméthyl-6-quinoxalinyl)propényl] benzoate de méthyle,
l'acide p-[(E)-2-(1,2,3,4-tétrahydro-1,4-diméthyl-6-quinoxalinyl)propényl] benzoïque,
le p-[(E)-2-(2-méthyl-1,3-benzodithiol-5-yl)propényl] benzoate de méthyle,
l'acide p-[(E)-2-(2-méthyl-1,3-benzodithiol-5-yl)propényl] benzoïque,
le p-[(E)-2-(1,2,3,4-tétrahydro-1,2,3,4-tétraméthylquinoxalin-5-yl)propényl] benzoate de méthyle,
l'acide p-[(E)-2-(2,2-diméthyl-1,3-benzodioxol-5-yl)propényl] benzoïque,
l'acide p-(2,2-diméthyl-1,3-benzodioxol-5-carboxamido)benzoïque.

15. Les composés selon la revendication 10,
l'ester éthylique de l'acide p-(2,3,4,5-tétrahydro-5-méthyl-1-benzothiépine-8-carboxamido)benzoïque,
l' acide p-(2,3,4,5-tétrahydro-1-benzothiépine-8-carboxamido)benzoïque,
l'ester éthylique de l'acide p-(3,4-dihydro-4,4-diméthyl-2H-1-benzopyranne-7-carboxamido)benzoïque,
l' acide p-(3,4-dihydro-4,4-diméthyl-2H-1-benzopyranne-7-carboxamido)benzoïque,
le p-(3,4-dihydro-4,4-diméthyl-2H-1-benzothiopyranne-7-carboxamido)benzoate d'éthyle,
l'acide p-(3,4-dihydro-4,4-diméthyl-2H-1-benzothiopyranne-7-carboxamido)benzoïque,
le 1,1-dioxyde p-(3,4-dihydro-4,4-diméthyl-2H-1-benzothiopyranne-7-carboxamido)benzoate d'éthyle,
le 1,1-dioxyde acide p-(3,4-dihydro-4,4-diméthyl-2H-1-benzothiopyranne-7-carboxamido)benzoïque,
le p-(1,2,3,4-tétrahydro-1,4,4-diméthyl-7-quinoléinecarboxamido)benzoate d'éthyle,
l'acide p-(1,2,3,4-tétrahydro-1,4,4-triméthyl-7-quinoléine-carboxamido)benzoïque,
le p-(1-décyl-1,2,3,4-tétrahydro-4,4-diméthyl-7-quinoléine-carboxamido)benzoate d'éthyle,
l'acide p-(1-décyle-1,2,3,4-tétrahydro-4,4-diméthyl-7-quinoléine-carboxamido)benzoïque,
l'acide p-(2,3,4,5-tétrahydro-5-méthyl-1-benzothiépine-8-carboxamido)benzoïque.

16. Les composés selon la revendication 11,
l'ester éthylique de l'acide p-(3,4-dihydro-3,3-diméthyl-2H-1,5-benzodithiépine-7-carboxamido)benzoïque,
le p-(1,4-benzodioxane-6-carboxamido)benzoate d'éthyle,
l'acide p-(1,4-benzodioxane-6-carboxamido)benzoïque,
l'acide p-(3,4-dihydro-3,3-diméthyl-2H-1,5-benzodithiépine-7-carboxamido)benzoïque,
l'acide p-(3,4-dihydro-3,3-diméthyl-2H-1,5-benzodioxépine-7-carboxamido)benzoïque,
le p-(2,2-diméthyl-1,3-benzoxathiol-5-carboxamido)benzoate d'éthyle,
l'acide p-(2,2-diméthyl-1,3-benzoxathiol-5-carboxamido)benzoïque,
le p-(2,2-diméthyl-1,3-benzodioxol-5-carboxamido)benzoate d'éthyle,
le p-(2,2-diméthyl-1,3-benzodioxol-6-carboxamido)benzoate d'éthyle,
la 2-[p-[(E)-3,4-(isopropylidènedioxy)-β-méthylstyryl] phénoxy]-N,N-diméthyléthylamine,
l'acide p-(2,2-diméthyl-1,3-benzodioxol-5-carboxamido)benzoïque et
l'ester éthylique de l'acide p-(3,4-dihydro-3,3-diméthyl-2H-1,5-benzodioxépine-7-carboxamido)benzoïque.

17. Le composé selon la revendication 7,
l'acide p-[(E)-2-(3,4-dihydro-3,3-diméthyl-2H-1,5-benzodithiépin-7-yl)propényl] benzoïque et ses esters alkyliques inférieurs.

18. Le composé selon la revendication 7,
l'ester éthylique de l'acide p-[(E)-2-(3,4-dihydro-3,3-diméthyl-2H-1,5-benzodithiépin-7-yl)propényl]benzoïque.

19. Procédé pour la préparation des composés de la revendication 7, caractérisé
a) en ce que l'on fait réagir un composé de formule générale 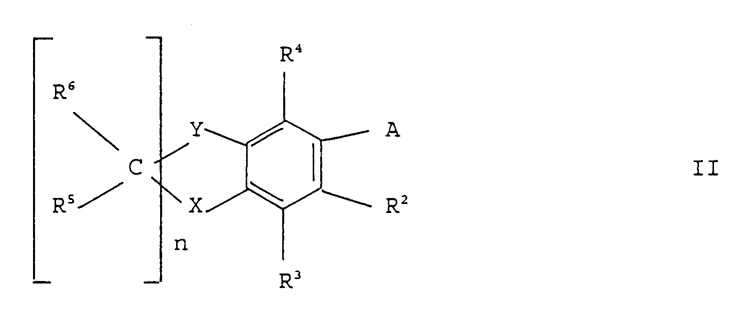 avec un composé de formule générale 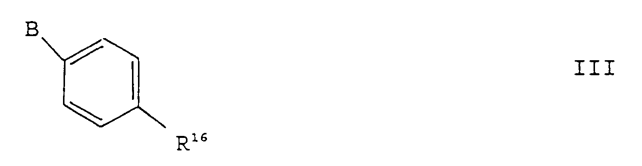 où soit A représente l'un des restes -CH(CH₃)P⁺(Q)₃Z⁻ ou -CH(CH₃)-P(O)(OAlk)₂ et B un reste formyle ; soit A représente un reste CH₃CO et B l'un des restes -CH₂ P+(Q)₃Z⁻ ou -CH₂ -P-(O) (OAlk)₂ ; Q représente un aryle ; Z⁻ l'anion d'un acide organique ou minéral ; Alk un groupe alkyle inférieur ; et R¹⁶ un reste R¹, à l'exception des groupes formyle, carboxyle et hydroxyméthyle ;
ou
b) en ce que l'on fait réagir un composé de formule générale 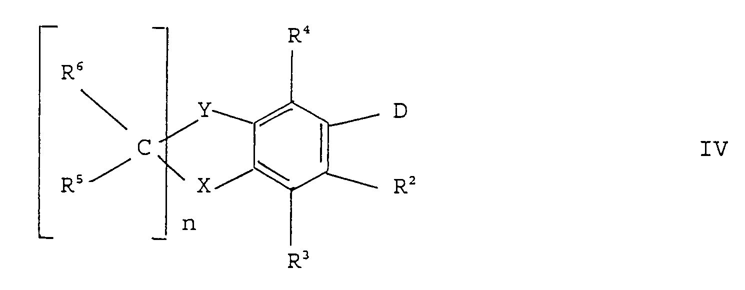 avec un composé de formule générale 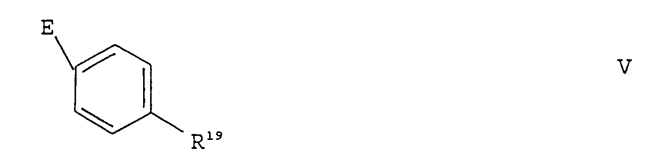 où soit D représente un groupe carboxylique ou l'un de ses dérivés réactifs et E représente un groupe amino, soit D représente un groupe amino et E un groupe carboxylique ou l'un de ses dérivés réactifs et R¹⁹ représente un reste R¹, à l'exception des groupes carboxyliques et hydroxyméthyliques,
c) en ce que l'on fait réagir un composé de formule générale 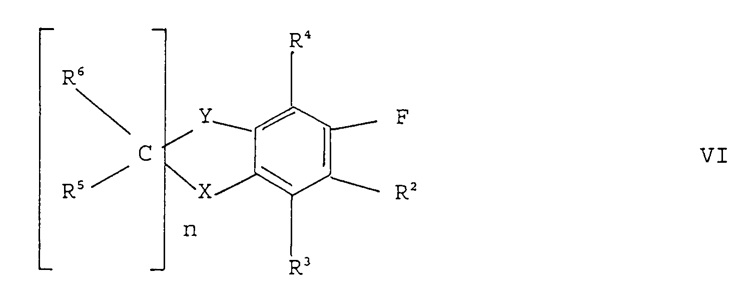 avec un composé de formule générale 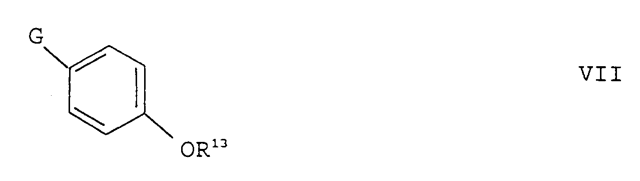 et en ce que l'on déshydrate le produit de la réaction ;
où soit F représente un reste -CH(CH₃)MgHal et G un reste formyle ; soit F représente un reste formyle et G un reste -CH₂MgHal ; Hal désigne un halogène et le terme "inférieur" désigne des groupes comportant 1 à 6 atomes de C et les autres symboles dans les formules II à VII ont la signification précédemment indiquée ;
en ce que, si désiré, l'on modifie fonctionnellement ensuite dans le produit de la réaction de formule I obtenu le reste R¹ et/ou en ce que l'on oxyde l'atome de soufre dans un composé I dans lequel X et/ou Y est -S-, pour former un groupe sulfoxyle ou sulfonyle.

20. Préparations pharmaceutiques contenant un composé de formule I selon la revendication I et les sels pharmaceutiquement utilisables d'un tel composé contenant un groupe carboxylique.

21. Préparations pharmaceutiques sous forme de doses unitaires contenant 5 à 500mg d'un composé de formule I selon la revendication 1 et les sels pharmaceutiquement utilisables d'un tel composé contenant un groupe carboxylique.

22. Utilisation d'un composé de formule I selon la revendication 1 et des sels pharmaceutiquement utilisables d'un tel composé contenant un groupe carboxylique, comme substances actives pour l'obtention de préparations pharmaceutiques destinées au traitement des néoplasies, dermatoses et de la gérodermie.

## Revendications (Revendications pour l'(les) Etat(s) contractant(s) suivant(s): ES, GR)

1. Procédé pour la préparation des composés de formule générale 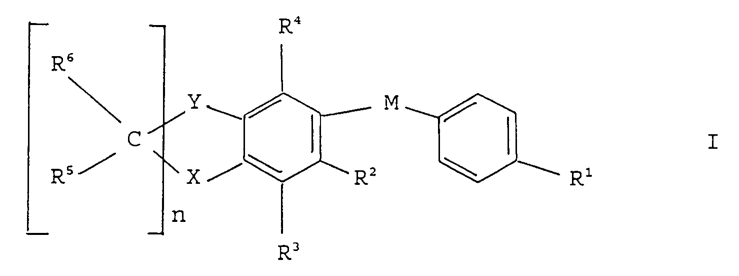 où R¹ représente un acyle ou un groupe -CHO, -CH₂OR¹⁰, - COR⁷ ou OR¹³ ; R², R³ et R⁴ représentent l'hydrogène, un alkyle inférieur, un alcoxy inférieur ou un halogène ; R⁵ et R⁶ représentent l'hydrogène ou un alkyle inférieur ; R⁷ représente l'hydroxy, un alcoxy inférieur ou NR⁸R⁹ ; R⁸ et R⁹ représentent l'hydrogène ou un alkyle inférieur ; X représente >CR¹⁴R¹⁵, -O-, -S-, >SO, >SO₂ ou >NR¹⁸ ; Y représente -O-, -S-, >SO, >SO₂ ou >NR¹⁸ ; R¹⁰ et R¹⁸ représentent l'hydrogène, un alkyle inférieur ou un acyle ; M représente -C(CH₃)=CH-, -CONH- ou -NHCO- ; R¹⁴ et R¹⁵ représentent l'hydrogène ou un alkyle inférieur, R¹³ représente un alkyle inférieur substitué par un amino, un mono- ou dialkylamino, un morpholino, un thiamorpholino ou un pipérazino, ou un alcoxy inférieur carbonyle ; et n est égal à 1, 2, 3 ou 4, le terme "inférieur" désignant des groupes comportant 1 à 6 atomes de C,
et des sels des composés de formule I dans lesquels R¹ représente un groupe carboxylique, caractérisé
a) en ce que l'on fait réagir un composé de formule générale 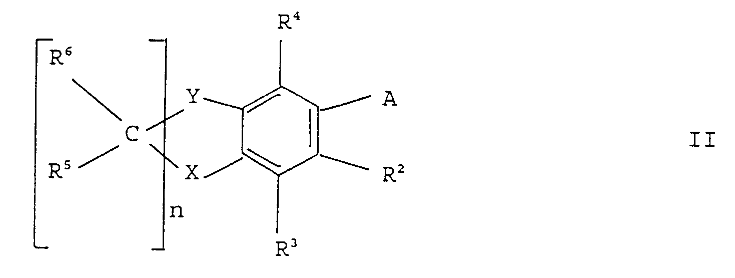 avec un composé de formule générale 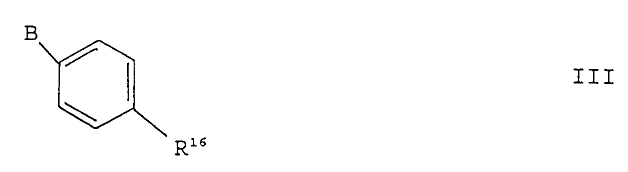 où soit A représente l'un des restes -CH(CH₃)P⁺(Q)₃Z⁻ ou -CH(CH₃)-P(O)(OAlk)₂ et B un reste formyle ; soit A représente un reste CH₃CO et B l'un des restes -CH₂ P⁺(Q)₃Z⁻ ou -CH₂ -P-(OAlk)₂ ; Q représente un aryle ; Z⁻ l'anion d'un acide organique ou minéral ; Alk un groupe alkyle inférieur ; et R¹⁶ un reste R¹, à l'exception des groupes formyle, carboxyle et hydroxyméthyle ; ou
b) en ce que l'on fait réagir un composé de formule générale 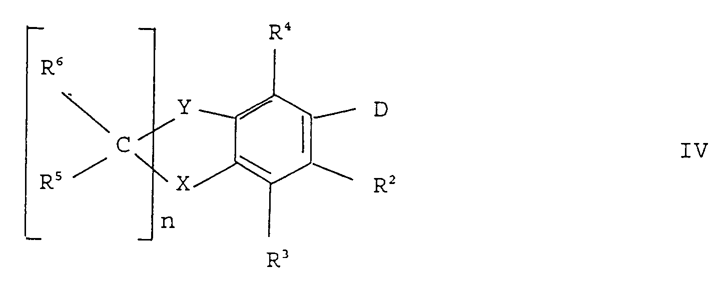 avec un composé de formule générale 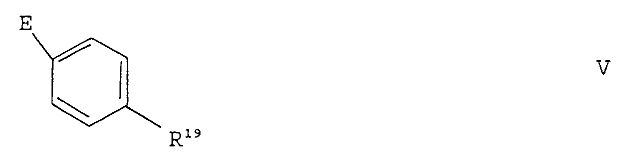 où soit D représente un groupe carboxylique ou l'un de ses dérivés réactifs et E représente un groupe amino, soit D représente un groupe amino et E un groupe carboxylique ou l'un de ses dérivés réactifs et R¹⁹ représente un reste R¹, à l'exception des groupes carboxyliques et hydroxyméthyliques,
c) en ce que l'on fait réagir un composé de formule générale 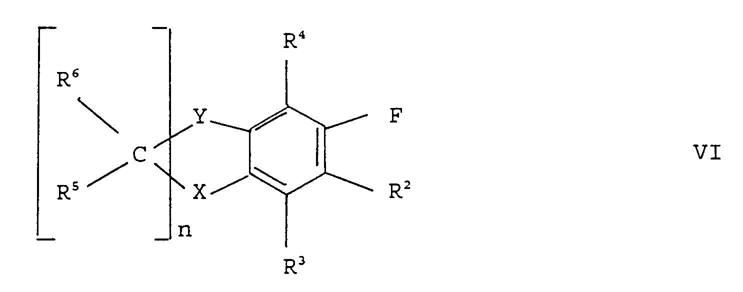 avec un composé de formule générale 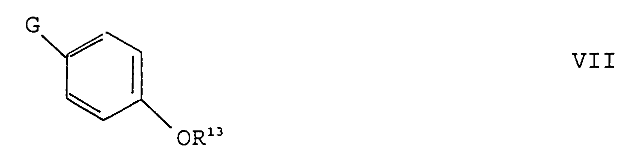 et en ce que l'on déshydrate le produit de la réaction ;
où soit F représente un reste -CH(CH₃)MgHal et G un reste formyle ; soit F représente un reste formyle et G un reste -CH₂MgHal ; Hal désigne un halogène et le terme "inférieur" désigne des groupes comportant 1 à 6 atomes de C et les autres symboles dans les formules II à VII ont la signification précédemment indiquée ;
en ce que, si désiré, l'on modifie fonctionnellement ensuite dans le produit de la réaction de formule I obtenue le reste R¹ et/ou en ce que l'on oxyde l'atome de soufre dans un composé I dans lequel X et/ou Y est -S-, pour former un groupe sulfoxyle ou sulfonyle.

2. Procédé selon la revendication 1 pour la préparation des composés de formule I où R¹³ représente un alkyle inférieur substitué par un amino, un mono- ou dialkylamino, un morpholino, un thiamorpholino ou un pipérazino et les autres symboles ont la signification donnée dans la revendication 1.

3. Procédé selon la revendication 1 ou 2 pour la préparation des composés de formule I où M représente - C(CH₃)=CH-.

4. Procédé selon la revendication 1 ou 2 pour la préparation des composés de formule I où M représente -CONH-.

5. Procédé selon les revendications 1 à 4 pour la préparation des composés de formule I où A et Y sont -O-, -S-, >SO, >SO₂ ou >NR¹⁸.

6. Procédé selon les revendications 1 à 5 pour la préparation des composés de formule I où X et Y sont -S-.

7. Procédé selon la revendication 1 pour la préparation des composés,
l'ester éthylique de l'acide p-[2-(3,4-dihydro-4,4-diméthyl-2H-1-benzopyranne-7-yl)propényl] benzoïque,
l'acide p-[2-(3,4-dihydro-4,4-diméthyl-2H-1-benzopyran-7-yl)propényl] benzoïque,
le p-[2-(3,4-dihydro-4,4-diméthyl-2H-1-benzothiopyran-7-yl)propényl] benzoate d'éthyle,
l'acide p-[2-(3,4-dihydro-4,4-diméthyl-2H-1-benzothiopyran-7-yl)propényl] benzoïque,
le 1',1'-dioxyde p-[2-(3',4'-dihydro-4'-dihydro-4',4'-diméthyl-2'H-1-benzothiopyran-7'-yl)propényl] benzoate d'éthyle,
le 1',1'-dioxyde acide p-[2-(3',4'-dihydro-4',4'-diméthyl-2'H-1-benzothiopyran-7'-yl)propényl] benzoïque,
le p-[(E)-2-(2,3,4,5-tétrahydro-5-méthyl-1-benzothiépin-8-yl)propényl]benzoate de méthyle,
le p-[2-(1,2,3,4-tétrahydro-1,4,4-triméthyl-7-quinoléinyl)propényl]benzoate d'éthyle,
l'acide p-[2-(1,2,3,4-tétrahydro-1,4,4-triméthyl-7-quinoléinyl)propényl] benzoïque,
l'ester éthylique de l'acide p-[(E)-2-(2,3,4,5-tétrahydro-1-benzoxépin-8-yl)propényl] benzoïque,
l'ester méthylique de l'acide p-[(E)-2-(2,3,4,5-tétrahydro-3,3-diméthyl-2H-1-benzothiépin-8-yl)propényl]benzoïque,
l'ester éthylique de l'acide p-[(E)-2-(2,3,4,5-tétrahydro-1-benzothiépin-8-yl)propényl] benzoïque,
le 1'1'-dioxyde acide p-[(E)-2-(2',3',4',5'-tétrahydro1-benzothiépin-8'-yl)propényl] benzoïque,
l'acide p-[(E)-2-(2,3,4,5-tétrahydro-1-benzothiépin-8-yl)propényl] benzoïque,
l'ester éthylique de l'acide p-(2,3,4,5-tétrahydro-1-benzothiépine-8-carboxamido)benzoïque.

8. Procédé selon la revendication 5 pour la préparation des composés,
le p-[(E)-2-(1,4-benzodioxan-6-yl)propényl] benzoate d'éthyle,
l'acide p-[(E)-2-(1,4-benzodioxan-6-yl)propényl] benzoïque,
le p-[(E)-2-(1,4-benzodithian-6-yl)propényl] benzoate d'éthyle,
l'acide p-[(E)-2-(1,4-benzodithian-6-yl)propényl] benzoïque,
l'ester méthylique de l'acide p-[(E)-2-(2,2-diméthyl-1,3-benzodioxol-5-yl)propényl] benzoïque,
l'ester éthylique de l'acide p-[(E)-2-(3,4-dihydro-3,3-diméthyl-2H-1,5-benzodioxépin-7-yl)propényl] benzoïque,
l'ester éthylique de l'acide p-[(E)-2-(3,4-dihydro-3-méthyl-2H-1,5-benzodithiépin-7-yl)propényl] benzoïque,
l'ester éthylique de l'acide p-[(E)-2-(3,4-dihydro-2H-1,5-benzodithiépin-7-yl)propényl] benzoïque,
le 1',1',5',5'-tétroxyde ester éthylique de l'acide p-[(E)-2-(3',4'-dihydro-3',3'-diméthyl-2'H-1,5-benzodithiépin- 7'-yl)propényl] benzoïque,
l'acide p-[(E)-2-(3,4-dihydro-3-méthyl-2H-1,5-benzodithiépin-7-yl)propényl] benzoïque,
l'acide p-[(E)-2-(3,4-dihydro-3,3-diméthyl-2H-1,5-benzodioxépin-7-yl)propényl] benzoïque,
l'ester méthylique de l'acide p-[(E)-2-(3,4-dihydro-3,3-diméthyl-2H-1,5-benzodithiépin-7-yl)propényl] benzoïque,
l'ester éthylique de l'acide p-[(E)-2-(2,3-dihydro-3,3-diméthyl-1,4-benzoxathiin-7-yl)propényl] benzoïque,
l'ester méthylique de l'acide p-[(E)-2-(2,3,4,5-tétrahydro-1,3,3,5-tétraméthyl-1H-1,5-benzodiazépin-7-yl)propényl] benzoïque,
la 4-[2-[p-[(E)-2-(3,4-dihydro-3,3-diméthyl-2H-1,5-benzodithiépin-7-yl)propényl] phénoxy] éthyl] morpholine,
la 4-[2-[p-[(E)-2-(3,4-dihydro-3,3-diméthyl-2H-1,5-benzodioxépin-7-yl)propényl] phénoxy] éthyl] morpholine,
le p-[(E)-2-(3,4-dihydro-3,3-diméthyl-2H-1,5-benzodioxépin-7-yl)propényl] phényléthylcarbonate,
l'ester méthylique de l'acide p-[(E)-2-(3,4-dihydro-5-méthyl-2H-1,5-benzothiazépin-8-yl)propényl] benzoïque,
l'ester méthylique de l'acide p-[(E)-2-(3,4-dihydro-3,5-diméthyl-2H-1,5-benzothiazépin-8-yl)propényl] benzoïque,
la N,N-diméthyl-2-[p-[(E)-2-(3,4-dihydro-3,3-diméthyl-2H-1,5-benzodithiépin-7-yl)propényl] phénoxy] éthylamine,
l'acide p-[(E)-2-(2,3-dihydro-3,3-diméthyl-1,4-benzoxathiin-7-yl)propényl] benzoïque,
l'acide p-[(E)-2-(3,4-dihydro-2H-1,5-benzodithiépin-7-yl)propényl] benzoïque,
l'acide p-[(E)-2-(3,4-dihydro-5-méthyl-2H-1,5-benzothiazépin-8-yl)propényl] benzoïque,
l'acide p-[(E)-2-(3,4-dihydro-3,5-diméthyl-2H-1,5-benzothiazépin-8-yl)propényl] benzoïque,
le p-[(E)-2-(2,2-diméthyl-1,3-benzodithiol-5-yl)propényl] benzoate de méthyle,
la 4-[2-[p-[(E)-2-(2,2-diméthyl-1,3-benzodithiol-5-yl)propényl] phénoxy] éthyl] morpholine,
le p-[(E)-2-(2,2-diméthyl-1,3-benzodithiol-5-yl)propényl] éthylphénylcarbonate,
l'acide p-[(E)-2-(2,2-diméthyl-1,3-benzodithiol-5-yl)propényl] benzoïque,
le p-[(E)-2-(1,2,3,4-tétrahydro-1,4-diméthyl-6-quinoxalinyl)propényl] benzoate de méthyle,
l'acide p-[(E)-2-(1,2,3,4-tétrahydro-1,4-diméthyl-6-quinoxalinyl)propényl] benzoïque,
le p-[(E)-2-(2-méthyl-1,3-benzodithiol-5-yl)propényl] benzoate de méthyle,
l'acide p-[(E)-2-(2-méthyl-1,3-benzodithiol-5-yl)propényl] benzoïque,
le p-[(E)-2-(1,2,3,4-tétrahydro-1,2,3,4-tétraméthylquinoxalin-5-yl)propényl] benzoate de méthyle,
l'acide p-[(E)-2-(2,2-diméthyl-1,3-benzodioxol-5-yl)propényl] benzoïque,
l'acide p-(2,2-diméthyl-1,3-benzodioxol-5-carboxamido) benzoïque.

9. Procédé selon la revendication 4 pour la préparation des composés,
l'ester éthylique de l'acide p-(2,3,4,5-tétrahydro-5-méthyl-1-benzothiépine-8-carboxamido)benzoïque,
l'acide p-(2,3,4,5-tétrahydro-1-benzothiépine-8-carboxamido)benzoïque,
l'ester éthylique de l'acide p-(3,4-dihydro-4,4-diméthyl-2H-1-benzopyranne-7-carboxamido)benzoïque,
l'acide p-(3,4-dihydro-4,4-diméthyl-2H-1-benzopyranne-7-carboxamido)benzoïque,
le p-(3,4-dihydro-4,4-diméthyl-2H-1-benzothiopyranne-7-carboxamido)benzoate d'éthyle,
l'acide p-(3,4-dihydro-4,4-diméthyl-2H-1-benzothiopyranne-7-carboxamido)benzoïque,
le 1,1-dioxyde p-(3,4-dihydro-4,4-diméthyl-2H-1-benzothiopyranne-7-carboxamido)benzoate d'éthyle,
le 1,1-dioxyde acide p-(3,4-dihydro-4,4-diméthyl-2H-1-benzothiopyranne-7-carboxamido)benzoïque,
le p-(1,2,3,4-tétrahydro-1,4,4-diméthyl-7-quinoléinecarboxamido)benzoate d'éthyle,
l'acide p-(1,2,3,4-tétrahydro-1,4,4-triméthyl-7-quinoléine-carboxamido)benzoïque,
le p-(1-décyl-1,2,3,4-tétrahydro-4,4-diméthyl-7-quinoléine-carboxamido)benzoate d'éthyle,
l'acide p-(1-décyle-1,2,3,4-tétrahydro-4,4-diméthyl-7-quinoléine-carboxamido)benzoïque,
l'acide p-(2,3,4,5-tétrahydro-5-méthyl-1-benzothiépine-8-carboxamido)benzoïque.

10. Les composés selon la revendication 11,
l'ester éthylique de l'acide p-(3,4-dihydro-3,3-diméthyl-2H-1,5-benzodithiépine-7-carboxamido)benzoïque,
le p-(1,4-benzodioxane-6-carboxamido)benzoate d'éthyle,
l'acide p-(1,4-benzodioxane-6-carboxamido)benzoïque,
l'acide p-(3,4-dihydro-3,3-diméthyl-2H-1,5-benzodithiépine-7-carboxamido)benzoïque,
l'acide p-(3,4-dihydro-3,3-diméthyl-2H-1,5-benzodioxépine-7-carboxamido)benzoïque,
le p-(2,2-diméthyl-1,3-benzoxathiol-5-carboxamido)benzoate d'éthyle,
l'acide p-(2,2-diméthyl-1,3-benzoxathiol-5-carboxamido)benzoïque,
le p-(2,2-diméthyl-1,3-benzodioxol-5-carboxamido)benzoate d'éthyle,
la 2-[p-[(E)-3,4-(isopropylidènedioxy)-β-méthylstyryl] phénoxy]-N,N-diméthyléthylamine,
l'acide p-(2,2-diméthyl-1,3-benzodioxol-5-carboxamido)benzoïque et
l'ester éthylique de l'acide p-(3,4-dihydro-3,3-dimthyl-2H-1,5-benzodioxépine-7-carboxamido)benzoïque.

11. Procédé selon la revendication 1 pour la préparation de l'acide p-[(E)-2-(3,4-dihydro-3,3-diméthyl-2H-1,5-benzodithiépin-7-yp)propényl] benzoïque et de ses esters alkyliques inférieurs.

12. Procédé selon la revendication 1 pour la préparation de l'ester éthylique de l'acide p-[(E)-2-(3,4-dihydro-3,3-diméthyl-2H-1,5-benzodithiépin-7-yl)propényl]benzoïque.

13. Procédé selon la revendication 1, caractérisé en ce que l'on procède à la réaction selon les variantes a), b) et c) du procédé dans un solvant organique à des températures comprises entre 0°C et le point d'ébullition du mélange réactionnel à la pression atmosphérique.

14. Utilisation d'un composé de formule I
où R¹ représente l'hydrogène, un acyle, un alkyle inférieur ou un groupe -CHO, -CH₂OR¹⁰, -COR⁷ ou OR¹³ ; R², R³ et R⁴ représentent l'hydrogène, un alkyle inférieur, un alcoxy inférieur ou un halogène ; R⁵ et R⁶ représentent l'hydrogène ou un alkyle inférieur ; R⁷ représente l'hydroxy, un alcoxy inférieur ou NR⁸R⁹ ; R⁸ et R⁹ représentent l'hydrogène ou un alkyle inférieur ; X représente >CR¹⁴R¹⁵, -O-, -S-, >SO, >SO₂ ou >NR¹⁸ ; Y représente -O-, -S-, >SO, >SO₂ ou >NR¹⁸ ; R¹⁰ et R¹⁸ représentent l'hydrogène, un alkyle inférieur ou un acyle ; M représente -C(R¹¹)=C(R¹²)-, -CONH- ou -NH-CO- ; R¹¹, R¹², R¹⁴ et R¹⁵ représentent l'hydrogène ou un alkyle inférieur, R¹³ l'hydrogène, un alkyle inférieur pouvant être substitué par un amino, un mono- ou dialkylamino, un morpholino, un thiamorpholino ou un pipérazino, ou un alcoxy inférieur carbonyle ; et n est égal à 1, 2, 3 ou 4, le terme "inférieur" désignant des groupes comportant 1 à 6 atomes de C,
et des sels des composés de formule I dans lesquels R¹ représente un groupe carboxylique, comme substances actives pour l'obtention de préparations pharmaceutiques destinées au traitement des néoplasies, des dermatoses et de la gérodermie.
